# EUROPEAN PATENT APPLICATION

(11) **EP 4 464 773 A1**
(43) Date of publication of application: **20.11.2024**
(21) Application number: 23737198.4
(22) Date of filing: 10.01.2023
(51) Int. Cl.: C12N 1/21, C12N 15/63, C12N 1/36, A61P 35/00, C12R 1/42

(54) **MODIFIED BACTERIUM WITH ANTI-TUMOUR ACTIVITY**

(30) Priority: 10.01.2022 CN 202210021722
(71) Applicant: Shenzhen Synthetica Pioneering Co., Ltd., Shenzhen, Guangdong 518107 (CN)
(72) Inventor: LIU, Chenli, Shenzhen, Guangdong 518055 (CN); WANG, Zuowei, Shenzhen, Guangdong 518055 (CN); GUO, Xuan, Shenzhen, Guangdong 518055 (CN); SHENG, Fangqian, Shenzhen, Guangdong 518055 (CN); ZANG, Zhongsheng, Shenzhen, Guangdong 518055 (CN); LI, Yang, Shenzhen, Guangdong 518055 (CN); CHEN, Qian, Shenzhen, Guangdong 518055 (CN)
(74) Representative: Michalski Hüttermann & Partner Patentanwälte mbB
(86) International application number: PCT/CN2023/071506
(87) International publication number: WO 2023/131336

(57) **Abstract**

The present invention relates to a modified bacterium with anti-tumor activity, which comprises an essential gene expression cassette under the control of a strictly hypoxia inducible promoter and is deficient in at least one gene involved in or regulating the endogenous anti-oxidative stress response pathway or a functional expression product thereof. The present invention also relates to a pharmaceutical composition comprising the modified bacterium and anti-tumor use thereof.

## Description

### Technical Field

The present invention relates to a modified bacterium with anti-tumor activity, a pharmaceutical composition comprising the modified bacterium and the anti-tumor use thereof.

### Background

Cancer has become one of the most important diseases threatening human life and health, but existing treatments (radiotherapy, chemotherapy, surgery and targeted drugs) all have shortcomings and there is an urgent need to develop new treatment approaches. Traditional bacterial tumor therapies, represented by Coley's toxin, have a history of 150 years, but are known for their unstable efficacy and safety concerns. The development of genetic engineering technology has made great progress in reducing the toxicity of bacterial strains, and a series of clinical trials have demonstrated that the attenuated engineered bacteria for human tumor therapy are safe but with limited efficacy, i.e., are unable to combine safety and therapeutic efficacy, thus failing to satisfy the needs of clinical tumor therapy. Further optimization for tumor therapy is imperative.

### Summary of the Invention

According to the present invention, a strictly anaerobic bacterium is constructed by means of synthetic biological genetic circuits, which shows therapeutic efficacy against tumors after being administered to an animal body or a human body, and can be cleared by normal tissues and organs in a short period of time, thereby reducing the toxic and side effects on the animal body or the human body due to the long-term retention of the bacterium *in vivo,* and thus providing a more reliable anti-tumor efficacy and safety.

In one embodiment, the present invention provides a modified bacterium, wherein the bacterium comprises a hypoxia regulated essential gene expression cassette comprising an essential gene of the bacterium under the control of a strictly hypoxia inducible promoter, and the bacterium is deficient in at least one gene required for survival in macrophages or a functional expression product thereof, as compared to an unmodified starting strain.

In one embodiment, the present invention provides a modified bacterium, wherein the bacterium comprises a hypoxia regulated essential gene expression cassette comprising an essential gene of the bacterium under the control of a strictly hypoxia inducible promoter, and the bacterium is deficient in at least one gene involved in or regulating the endogenous anti-oxidative stress response pathway or a functional expression product thereof, as compared to an unmodified starting strain.

In one embodiment, the present invention provides a modified bacterium, wherein the bacterium comprises a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

In one embodiment, the present invention provides a modified bacterium, wherein the bacterium comprises a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof, as compared to an unmodified starting strain.

In one embodiment, the present invention provides a modified bacterium of *Salmonella typhimurium,* wherein the bacterium comprise a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

In one embodiment, the present invention provides a modified bacterium of *Salmonella typhimurium,* wherein the bacterium comprise a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof, as compared to an unmodified starting strain.

In one embodiment, the present invention provides a pharmaceutical composition comprising the bacterium of any one of the preceding embodiments. In one embodiment, the pharmaceutical composition is used for treating a malignant tumor.

In one embodiment, the present invention provides a method for treating a malignant tumor comprising administering to a subject with the malignant tumor the pharmaceutical composition of any one of the preceding embodiments.

In one embodiment, the present invention provides use of the bacterium of any one of the preceding embodiments in the treatment of a malignant tumor.

### Brief Description of Drawings

FIG. 1 shows a schematic diagram of the construction scheme of DB-ZW1 strain.
FIG.2 shows the growth characteristics of DB-ZW1 and DB-ZW2 strains in various cells. Among them,
FIG.2A shows the transcription level of *dapE* genes in anaerobic and aerobic conditions for DB-ZW1 and DB-ZW2 strains; FIG.2B shows the growth of DB-ZW1 and DB-ZW2 strains under aerobic conditions;
and FIG.2C shows the doubling time of DB-ZW1 strain under aerobic conditions with the addition of DAP as well as under anaerobic conditions.
FIG.3 shows the growth of DB-ZW1 and DB-ZW3 strains in Raw264.7 mouse macrophages or MB49 mouse bladder cancer cells.
FIG.4 shows distributions of strains DB-ZW1, 2, 3, 4, 5 and wild-type SL7207 in normal tissues and tumors; FIG.4A shows the contents of strains DB-ZW1, 2, 3, 4, 5 and wild-type SL7207 in normal tissues;
FIG.4B shows the contents of strains DB-ZW1, 2, 3, 4, 5 and wild-type SL7207 in tumors.
FIG.5 shows distributions of strains DB-ZW1, DB-ZW1 (*ΔmsbB*)*,* DB-ZW1 (*Δlpp*) and DB-ZW1 (*ΔfliC*) in normal tissues and tumors.
FIG.6 shows anti-tumor effects of strains DB-ZW1, DB-ZW1 (*ΔmsbB*)*,* DB-ZW1 (*Δlpp*) and DB-ZW1 (*ΔfliC*) in mice. Among them, FIG.6A shows the effect of strains DB-ZW1, DB-ZW1 (*ΔmsbB*)*,* DB-ZW1 (*Δlpp*)*,* DB-ZW1 (*ΔfliC*) and wild-type SL7207 on the tumor volume of MB49 bladder tumor-bearing mice;
FIG.6B shows the effect of strains DB-ZW1, DB-ZW1 (*ΔmsbB*)*,* DB-ZW1 (*Δlpp*)*,* DB-ZW1 (*ΔfliC*) and wild-type SL7207 on the body weight of MB49 bladder tumor-bearing mice; FIG.6C shows the effect of strains DB-ZW1, DB-ZW1 (*ΔmsbB*)*,* DB-ZW1 (*Δlpp*) and DB-ZW1 (Δ*fliC*) and wild-type SL7207 on the survival of MB49 bladder tumor-bearing mice; FIG.6D shows various response types of DB-ZW1 to the treatment of MB49 mouse bladder cancer.
FIG.7 shows changes in the distribution of DB-ZW1 in tumors and various organs over time. Among them,
FIG.7A shows the changes of the density of DB-ZW1 in tumors and various organs over time; FIG.7B shows the changes of the density of DB-ZW1 in various normal organs over time.
FIG.8 shows the anti-tumor effects of DB-ZW1 on bladder cancer, melanoma and *in situ* colon cancer. Among them, FIGs. 8A-C illustrate the effects of DB-ZW1 on the tumor volume of subcutaneous bladder cancer, *in situ* melanoma, and subcutaneous *in situ* colon cancer, respectively; and FIG.8D illustrates the effect of DB-ZW1 on the tumor number of *in situ* colon cancer.
FIG.9 shows the contents of DB-ZW1 variant DB-ZW1-Δ*fliC* in tumors and normal organs.
FIG.10 shows activation of memory CD8⁺T cells by DB-ZW1. Among them, FIG.10A illustrates the effect of DB-ZW1 on the content of memory CD8⁺T cells in the draining lymph nodes of mice; FIG.10B illustrates the effect of DB-ZW1 on the content of memory CD8⁺T cells in the draining lymph nodes of mice inoculated with B16-OVA melanoma cells.
FIG.11 shows the tumor memory of the anti-tumor effect of DB-ZW1. Among them, FIG.11A illustrates that melanoma mice cured by DB-ZW1 do not develop tumors after another subcutaneous inoculation of 1 ×10⁶ B16 melanoma cells; FIG.11B illustrates that melanoma mice cured by DB-ZW1 have lower lung weight compared to those with the first induction of melanoma; and FIG.11C illustrates that melanoma mice cured by DB-ZW1 also developed tumors after another subcutaneously inoculation of 1×10⁶ MB49 bladder cancer cells, just like the mice first subcutaneously injected with MB49 bladder cancer cells.
FIG. 12 shows activation of IFNγ⁺CD8⁺T cells by DB-ZW1. Among them, FIG.12A illustrates the effect on the amount of IFNγ⁺CD8⁺T cells in the tumor microenvironment by treatment of mice subcutaneously inoculated with MB49 bladder cancer cells with DB-ZW1 and variants *DB-ZW1-ΔmsbB* and *DB-ZW1-Δlppon*; and FIG.12B illustrates the killing effects on MB49 and B16 cells by CD8⁺T cells isolated from the tumors of tumor-bearing mice treated with DB-ZW1.
FIG. 13 shows the effect of DB-ZW1 treatment on the proportion of TRM cells in CD8⁺T cells as well as the proportion of IFNγ⁺ cells in TRM cells in tumor-draining lymph nodes and tumors in mice with bladder cancer.
FIG.14 shows activation of CD8⁺T cells *in vitro* by an anti-CD3 antibody, DB-ZW1, DB-ZW1-*ΔmsbB,* and DB-ZW1*-Δlpp.*
FIG.15 shows the effect of DB-ZW1, DB-ZW1*-ΔmsB* and DB-ZW1-*Δlpp*on on the amount of IL-10 in serum of subcutaneous tumor-bearing mice with bladder cancer.
FIG. 16 shows the role of cytokine IL-10 in the treatment of tumors with DB-ZW1. Among them, FIG.16A shows the inhibitory effect of anti-IL-10 antibody on IFNγ⁺CD8⁺T cells induced by DB-ZW1 at the tumor site; and FIG.16A illustrates the inhibitory effect of anti-IL-10 antibody on tumor volume reduction induced by DB-ZW1.
FIG.17 shows IL-10 production in primary macrophages induced by DB-ZW1.
FIG. 18 shows the effect of DB-ZW1 on the distribution of neutrophils, T cells and macrophages in tumors.
FIG.19: FIGs. 19A and 19B illustrate the effects of anti-IL-10 antibody and anti-CSF1R antibody on the ring structure of neutrophil in DB-ZW1 treated tumors, respectively.
FIG.20 shows the effects of various DB-ZW1 administration manners on the anti-tumor efficacy. Among them, FIG.20A illustrates the effect of intratumoral injection or intravenous injection of DB-ZW1 on the distribution of bacteria in tumors and normal tissues; and FIG.20B illustrates the effect of intratumoral injection or intravenous injection of DB-ZW1 on tumor volume.
FIG.21 shows the effect of inactivated DB-ZW1 on the anti-tumor efficacy.
FIG.22 shows the effect of various administration doses of DB-ZW1 on the tumor volume in subcutaneous MB49 bladder tumor-bearing mice.

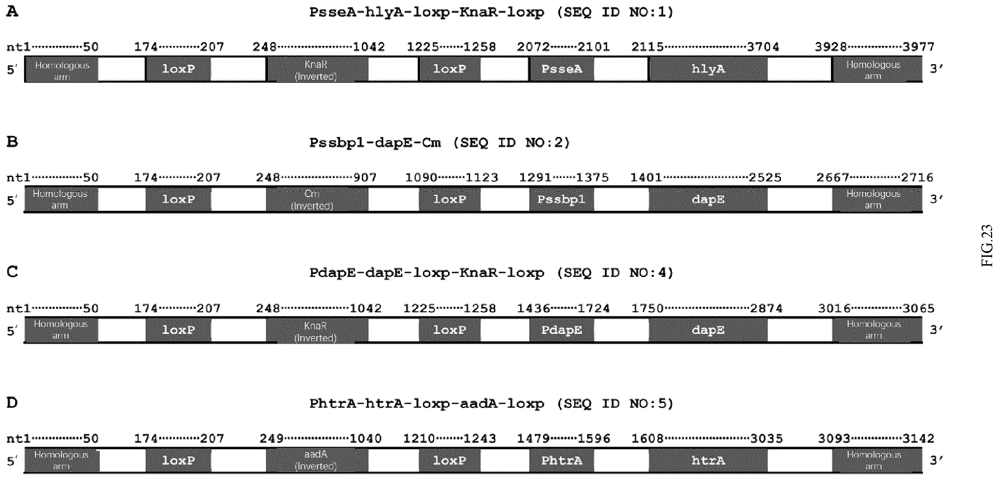

FIG.23 shows a schematic diagram of the desired fragments PsseA-hlyA-loxp-KnaR-loxp (SEQ ID NO:1) (A), Pssbp1-dapE-Cm (SEQ ID NO:2) (B), PdapE-dapE-loxp-KnaR-loxp (SEQ ID NO:4) (C) and PhtrA-htrA-loxp-aadA-loxp (SEQ ID NO:5) (D) used in Examples, with "numeral a ·········· numeral b" representing the initiation and stop nucleotide (nt) numbering of the primary element in the fragment in the sequence.

### Detailed Description of the Invention

### DEFINITIONS

In the present invention, the scientific and technical terms used herein have the meaning as commonly understood by a person skilled in the art unless otherwise specified. Also, the protein and nucleic acid chemistry, molecular biology, cell and tissue culture, microbiology, immunology related terms, and laboratory procedures used herein are terms and routine steps that are widely usedin the corresponding field. At the same time, for a better understanding of the present invention, the definitions and explanations of the related terms are provided below.

"Hypoxia regulated essential gene expression cassette" herein means a stretch of DNA capable of promoting the expression of an essential gene under hypoxic conditions, which contains an essential gene under the control of a hypoxia inducible promoter and, if necessary, may further contain other regulatory elements required for the expression of the essential gene.

As used herein, "essential gene" refers to a gene that is decisive for the growth and/or survival of a bacterium, in the absence of which or its functional expression product the bacterium fails to survive, divide and/or grow normally. Typical examples of deletion of an essential gene or a functional expression product thereof are nutritionally deficient strains that fail to survive, divide and/or grow normally under *in vitro* culture conditions or *in vivo* environment in the absence of specific exogenous supplements. Essential genes usually appear as a single copy on the bacterial chromosome.

As used herein, "strictly hypoxia inducible promoter" refers to a promoter that is capable of initiating the transcription of a specific gene under anaerobic conditions but is virtually impossible to activate and produce transcripts under aerobic conditions.

A "strictly hypoxia inducible promoter" used herein can be identified by the following experiments:
An expression strain containing an essential gene (e.g. *dapA*/*dapE*) under the control of a promoter to be tested is constructed, and streaked on a Luria-Bertani (abbreviated as LB) plate in an anaerobic incubator and incubated anaerobically at 37 °C for 24 h. Under anaerobic conditions, single clones are picked and resuspended into 20 µL of LB liquid medium. 20 µL of monoclonal resuspension solution is added equally to the following four liquid tubes A, B, C and D, with 5 µL added to each tube:
A. 2 mL of LB liquid medium,
B. 2 mL of LB liquid medium,
C. 2mL LB liquid medium + DAP,
D. 2 mL of LB liquid medium + DAP.

The tubes A and C are incubated statically in an anaerobic incubator at 37 °C for 24 h, and the tubes B and D are incubated in an aerobic shaker at 37 °C for 24 h. The promoter is considered to be a strictly hypoxia inducible promoter if bacteria can grow in tubes A, C and D but not in tube B (OD₆₀₀ is less than 0.05). Exemplary strictly hypoxia inducible promoters that can be used in the present invention are shown in Table 1.

**Table 1**

| Promoter Name | Sequences | SEQ ID NO: |
|---|---|---|
| pepTp | GCAGGGGTAAAAGTGACCTGACGCAATATTTGTCT TTTCTTGCTTCTTAATAATGTTGTCACAAAAAGTG AGGGTGACTACATGG | 30 |
| fnrSp | CGCCGCAAAGTTTGAGCGAAGTCAATAAACTCTCT ACCCATTCAGGGCAATATCTCTCTTGCAGGTGAAT | 31 |
| | GCAACGTCAAGCGAT | |
| ysgAp | TCAGAAGAAGCAAAAAGACACTTTACCGAAGGGTT TAACATTTTTTCGTGATACTCATCACCATGACGCA AATGCGTTGCATAAA | 32 |
| ssbp1 | AACCGAGGTCACAACATAGTAAAAGCGCTATTGGT AATGGTACAATCGCGCGTTTACACTTATTCAGAAC GATTTTTTTCAGGAG | 33 |
| Hip1 | GGATAAAAGTGACCTGACGCAATATTTGTCTTTTC TTGCTTAATAATGTTGTCA | 34 |
| BBa_I14018 | TGTAAGTTTATACATAGGCGAGTACTCTGTTATGG | 35 |
| BBa_R1074 | TTAAATTTCCTCTCGTCAGGCCGGAATAACTCCCT ATAATGCGCCACCACACTGATAGTGCTAGTGTAGA TCAC | 36 |
| Ptet-Fnr | AAAATTGATCTGAATCAATATTTTGACAAAAATTG ATCTGAATCAATATTTACTGAGC | 37 |
| Ptet-arcA | GTTAATAAAATGTTATTGACAGTTAATAAAATGTT ATACTGAGC | 38 |
| sseA | TTAGCACGTTAATTATCTATCGTGTATATG | 39 |

As used herein, "gene involved in or regulating the endogenous anti-oxidative stress response pathway" refers to a gene involved in the resistance of a bacterium (e.g., an intracellular bacterium, e.g., *Salmonella*) to the killing effects of reactive oxygen species (ROS) *in vivo* or *in vitro.*

As used herein, "gene required for survival in macrophages" refers to a gene involved in maintaining or enhancing the viability of intracellular bacteria in macrophages. The function of the products of these genes is associated to resistance to the killing effect of macrophages on microorganisms, and thus deletion of these genes or their functional expression products can reduce the viability of bacteria (e.g., intracellular bacteria, such as *Salmonella*) in macrophages.

As used herein, the "functional expression product" includes RNA, proteins, and the like.

As used herein, "facultative anaerobic bacteria" refers to bacteria that can survive under both aerobic and anaerobic conditions.

As used herein, the "pH regulated expression cassette" refers to a group of gene expression elements in which the expression of the corresponding gene(s) is regulated by environmental pH.

As used herein, "a promoter that is active under acid pH condition" refers to a promoter that can be activated under acid pH condition and regulate the expression of the corresponding gene(s). In some embodiments, the promoter that is active under acid pH condition of the present invention may be activated or still have promoter activity at a pH less than 7.0, 6.9, 6.8, 6.7, 6.6, 6.5, 6.4, 6.3, 6.2, 6.1, 6.0, 5.9, 5.8, 5.7, 5.6, or 5.5. In some embodiments, the promoter that is active under acid pH condition also has promoter activity at neutral pH (e.g., pH 7.0 to 7.4).

"Polypeptide", "peptide", and "protein" are used interchangeably in the present invention to refer to a polymer of amino acid residues. The terms apply to an amino acid polymer in which one or more amino acid residues is artificial chemical analogue of corresponding naturally occurring amino acid (s), as well as to a naturally occurring amino acid polymer. The terms "polypeptide", "peptide", "amino acid sequence", and "protein" may also include modified forms including, but not limited to, glycosylation, lipid ligation, sulfation, ycarboxylation of glutamic acid residues, and ADP-ribosylation.

As used herein, "polynucleotide" refers to a macromolecule formed by linking multiple nucleotides via phosphodiester linkages, wherein the nucleotide comprises ribonucleotide and deoxyribonucleotide. The sequence of the polynucleotide of the present invention can be subjected to codon optimization for different host cells (such as *Escherichia coli*), thereby improving the expression of the polypeptide. Methods for codon optimization are known in the art.

"Sequence identity" between two polypeptides or two polynucleotide sequences refers to percentage of identical amino acids or nucleotides between the sequences. Methods for assessing the level of sequence identity between polypeptide or polynucleotide sequences are known in the art. Sequence identity can be assessed using various known sequence analysis software. For example, sequence identity can be assessed by an on-line alignment tool of EMBL-EBI (https://www.ebi.ac.uk/Tools/psa/). Sequence identity between two sequences can be assessed using default parameters through Needleman-Wunsch algorithm.

As used herein, the term "live bacterium" refers to a strain that is viable, and characterized by active nutrient metabolism, and is capable of performing its own biological functions. Live bacteria may include bacterial biomass produced in the metabolic process of the strain.

The term "Gram-negative bacteria" used herein refers to bacteria that do not retain the initial basic dye stain (e.g., crystal violet) that is part of the procedure known as the Gram stain. In an exemplary Gram stain, cells are first fixed to a slide by heat and stained with a basic dye (e.g., crystal violet), which is taken up by both Gram-negative and Gram-positive bacteria. The slides are then treated with a mordant (e.g., Gram's iodine), which binds to basic dye (e.g. crystal violet) and traps it in the cell. The cells are then washed with acetone or alcohol, and then counterstained with a second dye of different color (e.g., safranin). Gram-positive organisms retain the initial violet stain, while Gram-negative organisms are decolorized by the wash solvent organic and hence show the counterstain. Exemplary Gram-negative bacteria include, but are not limited to, *Escherichia spp., Shigella spp., Salmonella spp., Campylobacter spp., Neisseria spp., Haemophilus spp., Aeromonas spp., Francisella spp., Yersinia spp., Klebsiella spp., Bordetella spp., Legionella spp., Corynebacteria spp., Citrobacter spp., Chlamydia spp., Brucella spp., Pseudomonas spp., Helicobacter spp.* and *Vibrio spp.*

As used herein, the term "malignant solid tumor" refers to an abnormal mass of tissues, usually not comprising cysts or liquid areas. Solid tumors may be benign (not cancerous) or malignant (cancerous). Different types of malignant solid tumors are named for the type of cells that form them. Examples of malignant solid tumors are sarcomas, carcinomas, and lymphomas. Leukemia (blood cancer) does not usually form a malignant solid tumor (as defined by the NIH National Cancer Institute). Malignant solid tumors include, but are not limited to, clusters of abnormal cells that may originate from different tissue types, for example, the liver, colon, colorectum, skin, breast, pancreas, cervix, corpus uteri, bladder, gallbladder, kidney, larynx, lip, oral cavity, esophagus, ovary, prostate, stomach, testis, thyroid, or lungs, and thus malignant solid tumors include malignant solid liver tumors, colon tumors, colorectal tumors , skin tumors, breast tumors, pancreatic tumors, cervical tumors, uterine corpus tumors, bladder tumors, gallbladder tumors, renal tumors, laryngeal tumors, lip tumors, oral tumors, esophageal tumors, ovarian tumors, prostate tumors, stomach tumors, testicular tumors, thyroid tumors, or lung tumors.

For purposes of treatment herein, the term "subject" is preferably a subject in need of treatment for a target pathological condition, such as a tumor. For the purpose of prevention, the subject is preferably a subject at risk of or predisposed to developing the target pathological condition. The term "subject" includes living organisms such as prokaryotes and eukaryotes. Examples of subjects include mammals, such as humans, dogs, cows, horses, pigs, sheep, goats, cats, mice, rabbits, rats, and genetically modified non-human animals. In some preferred embodiments, the subject is a human.

As used herein, "treatment" is an approach for obtaining beneficial or desired clinical results. For purposes of this invention, beneficial or desired clinical results include, but are not limited to, one or more of the following: reducing the proliferation of (or destroying) neoplastic or cancerous cells, inhibiting metastasis of neoplastic cells, shrinking or decreasing the size of a tumor, remission of malignant tumor, decreasing symptoms resulting from malignant tumor, increasing the quality of life of those suffering from malignant tumor, decreasing the dose of other medications required to treat malignant tumor, delaying the progression of malignant tumor, curing a malignant tumor, and/or prolong survival of patients having malignant tumor. As used herein, an "effective amount" or "effective dosage" of bacterium, medicament, or pharmaceutical composition is an amount sufficient to affect any one or more beneficial or desired results. For prophylactic use, beneficial or desired results include eliminating or reducing the risk, lessening the severity, or delaying the outset of the disease, including biochemical, histological and/or behavioral symptoms of the disease, its complications and intermediate pathological phenotypes presenting during development of the disease. For therapeutic use, beneficial or desired results include such as reducing one or more symptoms of a disease such as tumor, decreasing the dose of other medications required to treat the disease, enhancing the effect of another medication, and/or delaying the progression of the cancer in patients. For example, an "effective amount" preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

As used herein, "pharmaceutically acceptable carrier" includes any material which, when combined with an active ingredient, allows the ingredient to retain biological activity and is non-reactive with the subject's immune system, including but not limited to a disintegrating agent, an adhesive, a filler, a buffer, a tension agent, a stabilizer, an antioxidant, a surfactant or a lubricant. Preferably, the carrier is suitable for intravenous, intramuscular, subcutaneous, parenteral, spinal or epidermal administration (e.g., by injection or infusion). Depending on the route of administration, the bacterium of the present invention may be coated in a material to protect the compound from the action of acids and other natural conditions that may inactivate the bacterium. Pharmaceutically acceptable carriers include normal saline, PBS buffer, sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated.

### Modified bacteria

The present invention provides a modified bacterium, wherein the bacterium comprises a hypoxia regulated essential gene expression cassette, as compared to an unmodified starting strain. In one embodiment, the essential gene expression cassette comprises an essential gene of the bacterium under the control of a strictly hypoxia inducible promoter. In one embodiment, the bacterium is deficient in at least one gene involved in or regulating the endogenous anti-oxidative stress response pathway or a functional expression product thereof. In one embodiment, the bacterium is deficient in at least one gene required for survival in macrophages or a functional expression product thereof. In one embodiment, the bacterium expresses a wild-type lipopolysaccharide (LPS).

In one embodiment, the strictly hypoxia inducible promoter is selected from the group consisting of pepTp, fnrSp, ysgAp, ssbp1, Hip1, BBa_I14018, BBa_R1074, Ptet-arcA, and Ptet-Fnr. Preferably, the strictly hypoxia inducible promoter is ssbp1 promoter.

In one embodiment, the expression product of the essential gene is responsible for synthesis of 2,6-diaminopimelic acid (DAP) in the bacterium. In one embodiment, when cultured under aerobic conditions, the bacterial growth rely on the additional DAP or an analog thereof added to the medium. In one embodiment, the essential gene is selected from *dapA, dapB, dapd, dapE, αrgD, dapF,* and any combination thereof. Preferably, the essential gene is selected from *dapA* and *dapE.* Preferably, the *dapE* comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 27. Preferably, the nucleotide sequence of *dapE* includes SEQ ID NO: 27. Preferably, the nucleotide sequence of *dapE* consists of SEQ ID NO: 27.

In one embodiment, the gene involved in or regulating the endogenous anti-oxidative stress response pathway is an HtrA serine protease family gene. In one embodiment, the functional expression product of the gene involved in or regulating the endogenous anti-oxidative stress response pathway is an HtrA serine protease family related protein. In one embodiment, the bacterium is deficient in the activity of HtrA serine protease. In one embodiment, the gene involved in or regulating the endogenous anti-oxidative stress response pathway is *htrA.* In one embodiment, the bacterium is deficient in *htrA.* Preferably, the *htrA* comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 28. Preferably, the nucleotide sequence of *htrA* comprises SEQ ID NO: 28. Preferably, the nucleotide sequence of *htrA* consists of SEQ ID NO: 28.

In one embodiment, the essential gene is a gene naturally occurring in the bacterial chromosome. In one embodiment, the natural promoter of the essential gene is functionally replaced by the strictly hypoxia inducible promoter. As a result, the expression of the essential gene in the bacterium is completely under the control of the strictly hypoxia inducible promoter.

In one embodiment, the essential gene expression cassette is exogenous. In one embodiment, the essential gene naturally occurring in the bacterial chromosome is deleted or functionally inactivated. As a result, the expression of the essential gene in the bacterium is completely under the control of the strictly hypoxia inducible promoter. In one embodiment, the exogenous essential gene expression cassette is integrated into the bacterial chromosome. In one embodiment, the exogenous essential gene expression cassette is outside the bacterial chromosome. In one embodiment, the exogenous essential gene expression cassette is present in a plasmid carried by the bacterium.

In one embodiment, the modified bacterium further comprise a pH regulated expression cassette. In one embodiment, the modified bacterium comprises a hypoxia regulated essential gene expression cassette and a pH regulated expression cassette, as compared to an unmodified starting strain. In one embodiment, the pH regulated expression cassette comprises a gene encoding a bacteria-derived hemolysin protein under the control of a promoter that is active under acid pH condition. According to some embodiments of the present invention, the promoter that is active under acid pH condition is active in a pH range of about pH 4.0 to 7.0. In some embodiments, the promoter that is active under acid pH condition is also active at pH 6.0±1. In some embodiments, the promoter that is active under acid pH condition is active at pH values below 7.0, 6.9, 6.8, 6.7, 6.6, 6.5, 6.4, 6.3, 6.2, 6.1, 6.0, 5.9, 5.8, 5.7, 5.6, or 5.5, or between any two of these values. In one embodiment, the promoter that is active under acid pH condition is selected from the group consisting of sseA, ssrA, ssaB, ssaG, ssaM, and ssaR. In one embodiment, the promoter that is active under acid pH condition is sseA. In one embodiment, the bacteria-derived hemolysin protein is a gram-negative bacterial hemolysin protein. In one embodiment, the gene encoding the gram-negative bacterial hemolysin protein is *hlyA* or *hlyE.* In one embodiment, the *hlyA* or *hlyE* is derived from *Listeria monocytogenes, Vibrio cholerae,* or *Escherichia coli.* Preferably, the *hlyA* comprises a nucleotide sequence having a sequence identity of ≥81%, preferably ≥82%, more preferably ≥83%, ≥84%, ≥85%, ≥86%, ≥87%, ≥88%, ≥89%, ≥90%, ≥91%, ≥92%, ≥93%, ≥94%, ≥95%, ≥96%, ≥97%, ≥98%, or most preferably ≥99% to SEQ ID NO: 29. Preferably, the nucleotide sequence of *hlyA* includes SEQ ID NO: 29. Preferably, the nucleotide sequence of *htrA* consists of SEQ ID NO: 29.

In one embodiment, the unmodified starting strain is a facultative anaerobic bacterium. In one embodiment, the bacterium is of *Enterobacteriaceae.* Preferably, the bacterium is a bacteirum from *Escherichia sp., Salmonella sp., Shigella sp., Klebsiella sp., Yersinia sp., Citrobacter sp., Enterobacter sp., Serratia sp., Proteus sp., Morganella sp., Providencia sp., Hajnia sp.,* and *Pantoea sp.* Preferably, the bacterium is selected from the group consisting of *Escherichia coli* (*E.coli*), *Enterobacter blattae* (*E.blattae*), *Enterobacter fergusonii* (*Efergusonii*), *Enterobacter hermannii* (*E.hermannii*), *Enterobacter vulneris* (*Evulneris*)*, Salmonella enterica* (*S.enterica*)*, Salmonella bongori* (*S.bongori*)*, Salmonella typhi* (*S.typhi*), *Salmonella choleraesuis* (*S.choleraesuis*), *Salmonella typhimurium* (*S.typhimurium*), *Shigella dysenteriae* (*S.dysenteriae*), *Shigella flexneri* (*S.flexneri*), *Shigella boydii* (*S.boydii*), *Shigella sonnei* (*S.sonnei*), *Klebsiella pneumoniae* (*K.peumoniae*), *Klebsiella oxytoca* (*K.oxytoca*), *Yersinia pestis* (*Y.pestis*), *Yersinia enterocolitica* (*Y.enterocolitica*)*, Yersinia pseudotuberculosis* (*Y.pseudotuberculosis*), *Yersinia aldouae* (*Y.aldouae*), *Yersinia bercovieri* (*Y.bercovieri*), *Yersinia frederiksenii* (*Y.frederiksenii*), *Yersinia intermedia* (*Y.intermedia*), *Yersinia kristersenii* (*Y.kristersenii*), *Yersinia mollaretti* (*Y.mollaretti*), *Yersinia rohdei* (*Y.rohdei*), *Yersinia ruckeri* (*Y.ruckeri*), *Citrobacter freundii* (*C.freundii*), *Citrobacter koseri* (*C.kaseri*), *Citrobacter braakii* (*C.braakii*), *Enterobacter aerogenes* (*E.aerogenes*), *Enterobacter cloacae* (*E.cloacae*), *Enterobacter gergoviae* (*E.gergoviac*), *Enterobacter sakazakii* (*E.sakazakii*), *Enterobacter taylorae* (*E.tavlorae*), *Enterobacter amnigenus* (*E.aminigenus*), *Enterobacter intermedius* (*E.intermedius*), *Enterobacter asburiae* (*E.asburiac*), *Enterobacter cancerogenus* (*E.cancerogenus*), *Enterobacter dissolvens* (*E.dissolvens*), *Enterobacter nimipressurαlis* (*E.nimipressuralis*), *Serratia marcescens* (*S.marcescens*), *Serratia entomophila* (*S.entomophila*), *Serratia ficaria* (*S.ficaria*), *Serratia fonticola* (*S.fonticola*), *Serratia grimesii* (*S.grimesii*), *Serratia liquefaciens* (*S.liquefaciens*), *Serratia odorifera* (*S.odorifera*), *Serratia plymuthica* (*S.plymuthica*)*, Serratia proteamaculans* (*S.proteamaculans*), *Serratia rubidaea* (*S.rubidaea*), *Serratia ureilytica* (*S.ureilytica*), *Proteus mirabilis* (*P.mirabilis*), *Proteus vulgaris* (*P.vulgaris*), *Proteus myxofaciens* (*P.myxofaciens*), *Proteus penneri* (*P.penneri*), *Proteus hauseri* (*P.hauseri*)), *Morganella morganii* (*M.morganii*), *Providencia alcalifaciens* (*P.alcalifaciens*), *Providencia rustigianii* (*P.rustigianii*), *Providencia stuartii* (*P.stuartii*), *Providencia rettgeri* (*P.rettgeri*), *Providencia heimbachae* (*P.heimbochae*), *Hafnia alvei* (*H.alvei*), and *Pantoea agglomerans* (*P.agglomerans*). Preferably, the bacterium is of *Salmonella typhimurium.* Preferably, the starting strain is *Salmonella typhimurium* SL7207.

In one embodiment, when administrated to a subject with a tumor, the bacterium can survive and proliferate in the tumor tissue but is rapidly cleared in normal tissue. In one embodiment, when administrated to a subject with a malignant tumor, the bacterium can induce anti-tumor specific immune response. In one embodiment, when administrated to a subject with a malignant tumor, the bacterium can induce anti-tumor immune memory.

In one embodiment, the bacterium does not express a wild-type flagellin. In one embodiment, the modified bacterium is deficient in the *fliC* gene.

In one embodiment, the survival level of the bacterium in macrophages ranges from about 50% to about 30% of the survival level of the unmodified starting strain. In one embodiment, the survival level of the bacterium in macrophages ranges from about 30% to about 10% of the survival level of the unmodified starting strain. Preferably, the survival level of the bacterium in macrophages ranges from about 10% to about 1% of the survival level of the unmodified starting strain.

### Pharmaceutical composition

The present invention provides a pharmaceutical composition comprising an effective amount of the modified bacterium of the present invention. In one embodiment, the modified bacterium is a live bacterium.

In one embodiment, the pharmaceutical composition is used for treating a malignant tumor. In one embodiment, the pharmaceutical composition is used for inducing an anti-tumor specific immune response in a subject with a malignant tumor. In one embodiment, the pharmaceutical composition is used for inducing anti-tumor immune memory in a subject with a malignant tumor. In one embodiment, the pharmaceutical composition is used for preventing or treating metastasis or recurrence of a malignant tumor. In one embodiment, the pharmaceutical composition is used for treating a malignant tumor that is resistant to, or has failed to, a prior anti-tumor therapy.

In one embodiment, the malignant tumor is a malignant tumor from the nervous system, respiratory system, digestive system, urinary system, reproductive system, hematopoietic system, lymphatic system, endocrine system, or skin mucosa. In some embodiments, the malignant tumor is a sarcoma or cancer. In one embodiment, the malignant tumor is a solid tumor. In one embodiment, the malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

In one embodiment, the pharmaceutical composition further comprises a pharmaceutically acceptable carrier.

### Treatment Methods and Uses

The present invention provides a method for treating a malignant tumor, comprising administering an effective amount of the modified bacterium of the present invention or the pharmaceutical composition of the present invention to a subject with the malignant tumor.

The present invention provides a method for inducing an anti-tumor specific immune response in a subject with a malignant tumor, comprising administering an effective amount of the modified bacterium of the present invention or the pharmaceutical composition of the present invention to a subject with the malignant tumor.

The present invention provides a method for inducing anti-tumor immune memory in a subject with a malignant tumor, comprising administering an effective amount of the modified bacterium of the present invention or the pharmaceutical composition of the present invention to a subject with the malignant tumor. The present invention provides a method for preventing or treating metastasis or recurrence of a malignant tumor, comprising administering an effective amount of the modified bacterium of the present invention or the pharmaceutical composition of the present invention to a subject with the malignant tumor.

The present invention provides a method for preventing or treating metastasis or recurrence of a malignant tumor, comprising administering an effective amount of the modified bacterium of the present invention or the pharmaceutical composition of the present invention to a subject with the malignant tumor or at a high risk of metastasis or recurrence of the malignant tumor.

The present invention provides a method for treating a malignant tumor that is resistant to, or has failed to, a prior anti-tumor therapy, comprising administering an effective amount of the modified bacterium of the present invention or the pharmaceutical composition of the present invention to a subject with the malignant tumor that is resistant to, or has failed to, a prior anti-tumor therapy.

The present invention provides use of the modified bacterium of the present invention in the manufacture of a medicament in the treatment of a malignant tumor.

The present invention provides use of the modified bacterium of the present invention in the manufacture of a medicament for inducing an anti-tumor specific immune response in a subject with a malignant tumor. The present invention provides use of the modified bacterium of the present invention in the manufacture of a medicament for inducing anti-tumor immune memory in a subject with a malignant tumor.

The present invention provides use of the modified bacterium of the present invention in the manufacture of a medicament for preventing or treating metastasis or recurrence of a malignant tumor.

The present invention provides use of the modified bacterium of the present invention in the manufacture of a medicament for treating a malignant tumor that is resistant to, or has failed to, a prior anti-tumor therapy.

In one embodiment, the modified bacterium is a live bacterium.

In one embodiment, the malignant tumor is a malignant tumor from the nervous system, respiratory system, digestive system, urinary system, reproductive system, hematopoietic system, lymphatic system, endocrine system, or skin mucosa. In some embodiments, the malignant tumor is a sarcoma or cancer. In one embodiment, the malignant tumor is a solid tumor. In one embodiment, the malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

In one embodiment, the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes. In one embodiment, the bacterium, medicament, or pharmaceutical composition of the present invention may be formulated in a form that is administered by intravenous, intratumoral, intramuscular, subcutaneous, intraperitoneal, transcerebral, gastrointestinal, body surface, oral mucosal, transnasal, transrectal, or transvaginal routes.

In one embodiment, the dosage form of the medicament or pharmaceutical composition of the present invention may, for example, be in the form of a solution, an emulsion, a freeze-dried preparation or suspension; for oral administration, the dosage form may be in the form of tablets or capsules; for intranasal dosage, the dosage form may be in the form of powder, drop or aerosol; for body surface administration, the dosage form may be in the form of an aqueous solution, a suspension, an ointment, a cream or a gel; and for rectal or vaginal administration, the dosage form may be a suppository, an enema, or delivered as part of an endoscopy or colonoscopy procedure. In one embodiment, the medicament or pharmaceutical composition is formulated in the form of a solution, an emulsion, a freeze-dried preparation or suspension; for oral administration, it may be formulated in the form of a tablet or capsule; for intranasal dosage form, it may be formulated in the form of powder, nasal drops, or aerosol; for body surface administration, it may be formulated in the form of an aqueous solution, a suspension, an ointment, a cream, or a gel; and for rectal or vaginal administration, it can be formulated as a suppository, an enema or delivered as part of an endoscopy or colonoscopy procedure. For methods known in the art for preparing dosage forms see, for example, "Remington's Pharmaceutical Sciences" (20th ed.), edited by A. Gennaro, 2000, Mack Publishing Company, Inc. Easton, PA.

In some embodiments, the bacterium, medicament, or pharmaceutical composition of the present invention may be administered at various intervals, including but not limited to: 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 7 days, 8 days, 9 days, or 10 days, including the range between any two of the listed values, for example: 1 minute to 10 minutes, 1 minute to 30 minutes, 1 minute to 1 hour, 1 minute to 2 hours, 1 minute to 4 hours, 1 minute to 12 hours, 1 minute to 18 hours, 1 minute to 1 day, 10 minutes to 30 minutes, 10 minutes to 1 hour, 10 minutes to 2 hours, 10 minutes to 4 hours, 10 minutes to 12 hours, 10 minutes to 18 hours, 10 minutes to 1 day, 30 minutes to 1 hour, 30 minutes to 2 hours, 30 minutes to 4 hours, 30 minutes to 12 hours, 30 minutes to 18 hours, 30 minutes to 1 day, 30 minutes to 2 days, 1 hour to 2 hours, 1 hour to 4 hours, 1 hour to 12 hours, 1 hour to 18 hours, 1 hour to 1 day, 4 hours to 12 hours, 4 hours to 18 hours, 4 hours to 1 day, 1 hour to 2 days, 1 hour to 3 days, 1 hour to 4 days, 1 hour to 5 days, 1 hour to 7 days, 1 hour to 10 days, 2 hours to 3 days, 2 hours to 4 days, 2 hours to 5 days, 2 hours to 7 days, 2 hours to 10 days, or 5 days to 10 days. In some embodiments, the bacterium, medicament, or pharmaceutical composition is administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. In some embodiments, the bacterium, medicament, or pharmaceutical composition of the present invention may be formulated to be administered at various intervals, including but not limited to: 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 7 days, 8 days, 9 days, or 10 days, including the range between any two of the listed values, for example: 1 minute to 10 minutes, 1 minute to 30 minutes, 1 minute to 1 hour, 1 minute to 2 hours, 1 minute to 4 hours, 1 minute to 12 hours, 1 minute to 18 hours, 1 minute to 1 day, 10 minutes to 30 minutes, 10 minutes to 1 hour, 10 minutes to 2 hours, 10 minutes to 4 hours, 10 minutes to 12 hours, 10 minutes to 18 hours, 10 minutes to 1 day, 30 minutes to 1 hour, 30 minutes to 2 hours, 30 minutes to 4 hours, 30 minutes to 12 hours, 30 minutes to 18 hours, 30 minutes to 1 day, 30 minutes to 2 days, 1 hour to 2 hours, 1 hour to 4 hours, 1 hour to 12 hours, 1 hour to 18 hours, 1 hour to 1 day, 4 hours to 12 hours, 4 hours to 18 hours, 4 hours to 1 day, 1 hour to 2 days, 1 hour to 3 days, 1 hour to 4 days, 1 hour to 5 days, 1 hour to 7 days, 1 hour to 10 days, 2 hours to 3 days, 2 hours to 4 days, 2 hours to 5 days, 2 hours to 7 days, 2 hours to 10 days, or 5 days to 10 days. In some embodiments, the bacterium, medicament, or pharmaceutical composition is formulated to be administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. The dosage regimen may depend on the pharmacokinetic decay pattern expected to be achieved by the practitioner. Progress in therapy can be monitored by routine techniques and assays. The dosing regimen can change over time.

In some embodiments, the effective amount of bacteria in the medicament or pharmaceutical composition comprises at least about 10⁴ colony-forming unit (cfu), for example, at least about 10⁴, 10⁵, 10⁶, 10⁷, 10⁸, 10⁹, 10¹⁰ ,10¹¹ ,10¹² or 10¹³ cfu, including the range between any two of the listed values, such as 10⁴-10⁸ cfu, 10⁴-10⁹ cfu, 10⁴-10¹⁰cfu, 10⁴-10¹¹ cfu, 10⁴-10¹² cfu, 10⁴-10¹² cfu, 10⁵-10⁸ cfu, 10⁵-10⁹ cfu, 10⁵-10¹⁰ cfu, 10⁵-10¹¹ cfu, 10⁵-10¹² cfu, 10⁵-10¹² cfu, 10⁶-10⁸ cfu, 10⁶-10⁹ cfu, 10⁶-10¹⁰ cfu, 10⁶-10¹¹ cfu, 10⁶-10¹² cfu, 10⁶- 10¹² cfu, 10⁷-10⁸ cfu, 10⁷-10⁹ cfu, 10⁷-10¹⁰ cfu, 10⁷-10¹¹ cfu, 10⁷-10¹² cfu, 10⁷-10¹² cfu, 10⁸-10⁹ cfu, 10⁸-10¹⁰ cfu, 10⁸-10¹¹ cfu, 10⁸-10¹² cfu, or 10⁸-10¹² cfu.

In some embodiments, the volume of an effective dose of the bacterium, medicament, or pharmaceutical composition of the present invention is less than or equal to about 3 mL, about 2.5 mL, about 2 mL, about 1.5 mL, about 1 mL, about 0.75 mL, about 0.5 mL, about 0.25 mL, or about 0.1 mL. In some embodiments, the volume of an effective dose of the bacterium, medicament, or pharmaceutical composition is about 20 mL, about 19 mL, about 18 mL, about 17 mL, about 16 mL, about 15 mL, about 14 mL, about 13 mL, about 12 mL, about 11 mL, about 10 mL, about 9 mL, about 8 mL, about 7 mL, about 6 mL, about 5 mL, about 4 mL, about 3 mL, about 2 mL, or about 1 mL. Alternatively, the volume of an effective dose of the bacterium, medicament, or pharmaceutical composition is about 20.5 mL, about 19.5 mL, about 18.5 mL, about 17.5 mL, about 16.5 mL, about 15.5 mL, about 14.5 mL, about 13.5 mL, about 12.5 mL, about 11.5 mL, about 10.5 mL, about 9.5 mL, about 8.5 mL, about 7.5 mL, about 6.5 mL, about 5.5 mL, about 4.5 mL, about 3.5 mL, about 2.5 mL, about 1.5 mL, or about 0.5 mL. In some embodiments, the volume of an effective dose of the bacterial, medicament, or pharmaceutical composition is about 200 mL, about 190 mL, about 180 mL, about 170 mL, about 160 mL, about 150 mL, about 140 mL, about 130 mL, about 120 mL, about 110 mL, about 100 mL, about 90 mL, about 80 mL, about 70 mL, about 60 mL, about 50 mL, about 40 mL, or about 30 mL, as well as the range between any two of the listed values, for example, 100 mL-110 mL, 100 mL-120 mL, 90 mL-120 mL, 90 mL-130 mL. Alternatively, the volume of an effective dose of the bacterium, medicament, or pharmaceutical composition is about 205 mL, about 195 mL, about 185 mL, about 175 mL, about 165 mL, about 155 mL, about 145 mL, about 135 mL, about 125 mL, about 115 mL, about 105 mL, about 95 mL, about 85 mL, about 75 mL, about 65 mL, about 55 mL, about 45 mL, about 35 mL, or about 25 mL, as well as the range between any two of the listed values, for example, 105 mL-115 mL, 105 mL-125 mL, 95 mL-125 mL, 95 mL-135 mL. Alternatively, the volume of an effective dose of the bacterium, medicament or pharmaceutical composition is about 900 microlitres, about 800 microlitres, about 700 microlitres, about 600 microlitres, about 500 microlitres, about 400 microlitres, about 300 microlitres, about 200 microlitres, or about 100 microlitres, optionally about 950 microlitres, about 850 microlitres, about 750 microlitres, about 650 microlitres, about 550 microlitres, about 450 microlitres, about 350 microlitres, about 250 microlitres, about 150 microlitres, or about 50 microlitres. In some embodiments, the volume of an effective dose of the bacterium, medicament, or pharmaceutical composition is less than or equal to about 2.0 mL.

The bacterium concentration of the present invention, for example, in a medicament or pharmaceutical composition, may be at least about 10³, 10⁴, 10⁵, 10⁶, 10⁷, 10⁸, 10⁹, 10¹⁰, 10¹¹, 10¹² or 10¹³ cfu/mL, including the range between any two of the listed values, for example, 10³-10⁸ cfu/mL, 10³-10⁹ cfu/mL, 10³-10¹⁰ cfu/mL, 10³-10¹¹ cfu/mL, 10³-10¹²c fu/mL, 10³-10¹³ cfu/mL, 10⁴-10⁸ cfu/mL, 10⁴-10⁹ cfu/mL, 10⁴-10¹⁰ cfu/mL, 10⁴-10¹¹ cfu/mL, 10⁴-10¹² cfu/mL, 10⁴-10¹³ cfu/mL, 10⁵-10⁸ cfu/mL, 10⁵-10⁹ cfu/mL, 10⁵-10¹⁰ cfu/mL, 10⁵-10¹¹ cfu/mL, 10⁵-10¹² cfu/mL, 10⁵-10¹³ cfu/mL, 10⁶-10⁸ cfu/mL, 10⁶-10⁹ cfu/mL, 10⁶-10¹⁰ cfu/mL, 10⁶-10¹¹ cfu/mL, 10⁶-10¹² cfu/mL, 10⁶-10¹³ cfu/mL, 10⁷-10⁸ cfu/mL, 10⁷-10⁹ cfu/mL, 10⁷-10¹⁰ cfu/mL, 10⁷-10¹¹ cfu/mL, 10⁷-10¹² cfu/mL, 10⁷-10¹³ cfu/mL, 10⁸-10⁹ cfu/mL, 10⁸-10¹⁰ cfu/mL, 10⁸-10¹¹ cfu/mL, 10⁸-10¹² cfu/mL, or 10⁸-10¹³ cfu/mL. The bacterium concentration of the present invention, for example, in a medicament or pharmaceutical composition, may be at least about 10³, 10⁴, 10⁵, 10⁶, 10⁷, 10⁸, 10⁹, 10¹⁰, 10¹¹, 10¹², or 10¹³cfu/g, including the range between any two of the listed values, for example, 10³-10⁸ cfu/g, 10³-10⁹ cfu/g, 10³-10¹⁰ cfu/g, 10³-10¹¹ cfu/g, 10³-10¹² cfu/g, 10³-10¹³ cfu/g, 10⁴-10⁸ cfu/g, 10⁴-10⁹ cfu/g, 10⁴-10¹⁰ cfu/g, 10⁴-10¹¹ cfu/g, 10⁴-10¹² cfu/g, 10⁴-10¹³ cfu/g, 10⁵-10⁸ cfu/g, 10⁵-10⁹ cfu/g, 10⁵-10¹⁰ cfu/g, 10⁵-10¹¹ cfu/g, 10⁵-10¹² cfu/g, 10⁵-10¹³ cfu/g, 10⁶-10⁸ cfu/g, 10⁶-10⁹ cfu/g, 10⁶-10¹⁰ cfu/g, 10⁶-10¹¹ cfu/g, 10⁶-10¹²cfu/g, 10⁶-10¹³ cfu/g, 10⁷-10⁸ cfu/g, 10⁷-10⁹ cfu/g, 10⁷-10¹⁰ cfu/g, 10⁷-10¹¹ cfu/g, 10⁷-10¹² cfu/g, 10⁷-10¹³cfu/g, 10⁸-10⁹ cfu/g, 10⁸-10¹⁰ cfu/g, 10⁸-10¹¹ cfu/g, 10⁸-10¹² cfu/g, or 10⁸-10¹³ cfu/g.

Dosage ranges set forth herein are exemplary only and do not limit the dosage ranges that may be selected by medical practitioners. The amount of active compound (e.g., the bacterium of the invention) in the pharmaceutical composition of the present invention may vary according to factors such as the disease state, age, sex, and weight of the individual, including the presence or absence of the malignant tumor, the type and severity of the malignant tumor to be treated, the activity or viability of the bacterium, medicament or pharmaceutical composition, the route of administration, the duration of treatment, the medicament, if any, used in combination with the bacterium, medicament or pharmaceutical composition, the dietary and general health status of the subject, and similar factors well known in the art. Dosage regimens may be adjusted to provide the optimum therapeutic response. For example, a single dose may be administered, several divided doses may be administered over time or the dose may be proportionally reduced or increased as indicated by the exigencies of the therapeutic situation.

The bacterium, medicament or pharmaceutical composition of the present invention may be manufactured by methods well known in the art such as microbial growth in fermenters, followed by concentration and washing by centrifugation, filtration or dialysis, conventional granulating, mixing, dissolving, encapsulating, lyophilizing, or emulsifying processes, among others. The bacterium, medicament or pharmaceutical composition of the present invention may be produced in various forms, including granules, precipitates, or particulates, powders, including freeze dried, rotary dried or spray dried powders, amorphous powders, injections, emulsions, elixirs, suspensions or solutions. Formulations may optionally contain stabilizers, pH modifiers, surfactants, bioavailability modifiers and combinations of these.

In one embodiment, the bacterium, medicament or pharmaceutical composition of the present invention may be administered alone or in combination with other compounds or compositions in the presence of a carrier. In one embodiment, the bacterium, medicament or pharmaceutical composition may be administered in combination with other malignancy therapies, including, but not limited to, radiotherapy, chemotherapy, and surgery. The bacterium, medicament or pharmaceutical composition may be used as an adjuvant in therapies in such cases. Therefore, another aspect of the embodiment relates to a bacterium, medicament, or pharmaceutical composition for use as an adjuvant in a malignancy therapy selected from radiotherapy and chemotherapy.

### Exemplary embodiments

### Embodiment A

Embodiment A1: a modified bacterium, wherein the bacterium comprise a hypoxia regulated essential gene expression cassette comprising an essential gene of the bacterium under the control of a strictly hypoxia inducible promoter, and the bacterium is deficient in at least one gene involved in or regulating the endogenous anti-oxidative stress response pathway or a functional expression product thereof, as compared to an unmodified starting strain.

Embodiment A2: the modified bacterium of embodiment A1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment A3: the modified bacterium of embodiment A1, wherein the strictly hypoxia inducible promoter is ssbp1 promoter.

Embodiment A4: the modified bacterium of any one of the preceding embodiments A, wherein the expression product of the essential gene is responsible for synthesis of 2,6-diaminopimelic acid (DAP) in the bacterium, and when cultured under aerobic conditions, the bacterial growth is dependent on the additional DAP or an analog thereof added to the medium.

Embodiment A5: the modified bacterium of embodiment A4, wherein the essential gene is selected from *dapA, dapB, dapd, dapE, argD, dapF,* and any combination thereof.

Embodiment A6: the modified bacterium of embodiment A4, wherein the essential gene is selected from *dapA* and *dapE.*

Embodiment A7: the modified bacterium of any one of the preceding embodiments A, wherein the gene involved in or regulating the endogenous anti-oxidative stress response pathway is an HtrA serine protease family gene.

Embodiment A8: the modified bacterium of any one of the preceding embodiments A, wherein the functional expression product of the gene involved in or regulating the endogenous anti-oxidative stress response pathway is an HtrA serine protease family related protein.

Embodiment A9: the modified bacterium of any one of the preceding embodiments A, wherein the bacterium is deficient in the activity of HtrA serine protease.

Embodiment A10: the modified bacterium of any one of the preceding embodiments A, wherein the gene involved in or regulating the endogenous anti-oxidative stress response pathway is *htrA.*

Embodiment A11: the modified bacterium of any one of the preceding embodiments A, wherein the bacterium is deficient in *htrA.*

Embodiment A12: the modified bacterium of any one of embodiments A1-A11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment A13: the modified bacterium of any one of embodiments A1-A11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment A14: the modified bacterium of embodiments A13, wherein the exogenous essential gene expression cassette is integrated into the bacterial chromosome.

Embodiment A15: the modified bacterium of embodiments A13, wherein the exogenous essential gene expression cassette is outside the bacterial chromosome.

Embodiment A16: the modified bacterium of embodiments A15, wherein the exogenous essential gene expression cassette is present in the plasmid carried by the bacterium.

Embodiment A17: the modified bacterium of any one of the preceding embodiments A, which further comprises a pH regulated expression cassette, wherein the pH regulated expression cassette comprises a gene encoding a bacteria-derived hemolysin protein under the control of a promoter that is active under acid pH condition.

Embodiment A18: the modified bacterium of embodiments A17, wherein the bacteria-derived hemolysin protein is a gram-negative bacterial hemolysin protein.

Embodiment A19: the modified bacterium of embodiments A18, wherein the gene encoding the gram-negative bacterial hemolysin protein is *hlyA* or *hlyE.*

Embodiment A20: the modified bacterium of embodiments A19, wherein the *hlyA* or *hlyE* is derived from *Listeria monocytogenes, Vibrio cholerae,* or *Escherichia coli.*

Embodiment A21: the modified bacterium of any one of embodiments A17-A20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment A22: the modified bacterium of any one of embodiments A17-A21, wherein the promoter that is active under acid pH condition is selected from the group consisting of sseA, ssrA, ssaB, ssaG, ssaM, and ssaR.

Embodiment A23: the modified bacterium of any one of embodiments A17-A21, wherein the promoter that is active under acid pH condition is sseA.

Embodiment A24: the modified bacterium of any one of the preceding embodiments A, wherein the unmodified starting strain is a facultative anaerobic bacterium.

Embodiment A25: the modified bacterium of any one of the preceding embodiments A, wherein the bacterium is of *Enterobacteriaceae.*

Embodiment A26: the modified bacterium of any one of the preceding embodiments A, wherein the bacterium is a bacteirum of *Escherichia sp., Salmonella* sp., *Shigella sp., Klebsiella sp., Yersinia sp., Citrobacter sp., Enterobacter* sp., *Serratia sp., Proteus sp., Morganella sp., Providencia sp., Hafnia sp.,* and *Pantoea sp.*

Embodiment A27: the modified bacterium of any one of the preceding embodiments A, wherein the bacterium is selected from the group consisting of *Escherichia coli* (*E.coli*)*, Enterobacter blattae* (*E.blattae*), *Enterobacter fergusonii* (*E.fergusonii*), *Enterobacter hermannii* (*E.hermannii*), *Enterobacter vulneris* (*E.vulneris*)*, Salmonella enterica* (*S.enterica*)*, Salmonella bongori* (*S.bongori*), *Salmonella typhi* (*S.typhi*), *Salmonella choleraesuis* (*S.choleraesuis*), *Salmonella typhimurium* (*S.typhimurium*), *Shigella dysenteriae* (*S.dysenteriae*), *Shigella flexneri* (*S.flexneri*), *Shigella boydii* (*S.boydii*), *Shigella sonnei* (*S.sonnei*), *Klebsiella pneumoniae* (*K.peumoniae*), *Klebsiella oxytoca* (*K.oxytoca*), *Yersinia pestis (*Y*.pestis*), *Yersinia enterocolitica* (*Y.enterocolitica*), *Yersinia pseudotuberculosis* (*Y.pseudotuberculosis*), *Yersinia aldouae* (*Y.aldouae*), *Yersinia bercovieri* (*Y.bercovieri*), *Yersinia frederiksenii* (*Y.frederiksenii*), *Yersinia intermedia* (*Y.intermedia*), *Yersinia kristersenii* (*Y.kristersenii*), *Yersinia mollaretti* (*Y.mollaretti*), *Yersinia rohdei* (*Y.rohdei*), *Yersinia ruckeri* (*Y.ruckeri*), *Citrobacter freundii* (*C.freundii*), *Citrobacter koseri* (*C.kaseri*), *Citrobacter braakii* (*C.braakii*), *Enterobacter aerogenes* (*E.aerogenes*), *Enterobacter cloacae* (*E.cloacae*), *Enterobacter gergoviae* (*E.gergoviac*), *Enterobacter sakazakii* (*E.sakazakii*), *Enterobacter taylorae* (*E.tavlorae*), *Enterobacter amnigenus* (*E.aminigenus*), *Enterobacter intermedius* (*E.intermedius*), *Enterobacter asburiae* (*E.asburiac*), *Enterobacter cancerogenus* (*E.cancerogenus*), *Enterobacter dissolvens* (*E.dissolvens*), *Enterobacter nimipressuralis* (*E.nimipressuralis*), *Serratia marcescens* (*S.marcescens*), *Serratia entomophila* (*S.entomophila*), *Serratia ficaria* (*S.ficaria*), *Serratia fonticola* (*S.fonticola*), *Serratia grimesii* (*S.grimesii*), *Serratia liquefaciens* (*S.liquefaciens*), *Serratia odorifera* (*S.odorifera*), *Serratia plymuthica* (*S.plymuthica*), *Serratia proteamaculans* (*S.proteamaculans*), *Serratia rubidaea* (*S.rubidaea*), *Serratia ureilytica* (*S.ureilytica*), *Proteus mirabilis* (*P.mirabilis*), *Proteus vulgaris* (*P.vulgaris*), *Proteus myxofaciens* (*P.myxofaciens*), *Proteus penneri* (*P.penneri*), *Proteus hauseri* (*P.hauseri*))*, Morganella morganii* (*M.morganii*), *Providencia alcalifaciens* (*P.alcalifaciens*), *Providencia rustigianii* (*P.rustigianii*), *Providencia stuartii* (*P.stuartii*), *Providencia rettgeri* (*P.rettgeri*)*, Providencia heimbachae* (*P.heimbochae*), *Hafnia alvei* (*H.alvei*), and *Pantoea agglomerans* (*P.agglomerans*).

Embodiment A28: the modified bacterium of any one of the preceding embodiments A, wherein the bacterium is of *Salmonella typhimurium.*

Embodiment A29: the modified bacterium of embodiment A28, wherein the starting strain is *Salmonella typhimurium* SL7207.

Embodiment A30: the modified bacterium of any one of the preceding embodiments A, wherein the bacterium expresses a wild-type lipopolysaccharide (LPS).

Embodiment A31: the modified bacterium of any one of the preceding embodiments A, wherein, when administrated to a subject with a tumor, the bacterium is capable of surviving and proliferating in tumor tissue and is rapidly cleared in normal tissue.

Embodiment A32: the modified bacterium of any one of the preceding embodiments A, wherein, when administrated to a subject with a malignant tumor, the bacterium is capable of inhibiting the growth of the malignant tumor.

Embodiment A33: the modified bacterium of any one of the preceding embodiments A, wherein, when administrated to a subject with a malignant tumor, the bacterium is capable of inducing an anti-tumor specific immune response.

Embodiment A34: the modified bacterium of any one of the preceding embodiments A, wherein, when administrated to a subject with a malignant tumor, the bacterium is capable of inducing anti-tumor immune memory.

Embodiment A35: the modified bacterium of any one of the preceding embodiments A, which does not express a wild-type flagellin.

Embodiment A36: the modified bacterium of any one of the preceding embodiments A, which is deficient in the *fliC* gene.

Embodiment A37: the modified bacterium of any one of the preceding embodiments A, wherein the survival level of the bacterium in macrophages ranges from about 50% to about 30% of the survival level of the unmodified starting strain.

Embodiment A38: the modified bacterium of any one of embodiments A1-A36, wherein the survival level of the bacterium in macrophages ranges from about 30% to about 10% of the survival level of the unmodified starting strain.

Embodiment A39: the modified bacterium of any one of embodiments A1-A36, wherein the survival level of the bacterium in macrophages ranges from about 10% to about 1% of the survival level of the unmodified starting strain.

### Embodiment B

Embodiment B1: a modified bacterium, wherein the bacterium comprise a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

Embodiment B2: the modified bacterium of embodiment B1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment B3: the modified bacterium of embodiment B1, wherein the strictly hypoxia inducible promoter is ssbp1 promoter.

Embodiment B4: the modified bacterium of any one of the preceding embodiments B, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment B5: the modified bacterium of embodiment B4, wherein the essential gene is selected from *dapA, dapB, dapd, dapE, argD, dapF,* and any combination thereof.

Embodiment B6: the modified bacterium of embodiment B4, wherein the essential gene is selected from *dapA* and *dapE.*

Embodiment B7: the modified bacterium of any one of the preceding embodiments B, wherein the gene involved in or regulating the endogenous anti-oxidative stress response pathway is an HtrA serine protease family gene.

Embodiment B8: the modified bacterium of any one of the preceding embodiments A, wherein the functional expression product of the gene involved in or regulating the endogenous anti-oxidative stress response pathway is an HtrA serine protease family related protein.

Embodiment B9: the modified bacterium of any one of the preceding embodiments B, wherein the bacterium is deficient in the activity of HtrA serine protease.

Embodiment B10: the modified bacterium of any one of the preceding embodiments B, wherein the gene involved in or regulating the endogenous anti-oxidative stress response pathway is *htrA.*

Embodiment B11: the modified bacterium of any one of the preceding embodiments B, wherein the bacterium is deficient in *htrA.*

Embodiment B12: the modified bacterium of any one of embodiments B1-B11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment B13: the modified bacterium of any one of embodiments B1-B11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment B14: the modified bacterium of embodiments B13, wherein the exogenous essential gene expression cassette is integrated into the bacterial chromosome.

Embodiment B15: the modified bacterium of embodiments B13, wherein the exogenous essential gene expression cassette is outside the bacterial chromosome.

Embodiment B16: the modified bacterium of embodiments B15, wherein the exogenous essential gene expression cassette is present in the plasmid carried by the bacterium.

Embodiment B17: the modified bacterium of embodiments any one of the preceding embodiments B, wherein the bacteria-derived hemolysin protein is a gram-negative bacterial hemolysin protein.

Embodiment B18: the modified bacterium of embodiments B17, wherein the gene encoding the gram-negative bacterial hemolysin protein is *hlyA* or *hlyE.*

Embodiment B19: the modified bacterium of embodiments B18, wherein the hlyA or *hlyE* is derived from *Listeria monocytogenes, Vibrio cholerae,* or *Escherichia coli.*

Embodiment B20: the modified bacterium of any one of the preceding embodiments B, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment B21: the modified bacterium of any one of the preceding embodiments B, wherein the promoter that is active under acid pH condition is selected from the group consisting of sseA, ssrA, ssaB, ssaG, ssaM, and ssaR.

Embodiment B22: the modified bacterium of any one of the preceding embodiments B, wherein the promoter that is active under acid pH condition is sseA.

Embodiment B23: the modified bacterium of any one of the preceding embodiments B, wherein the unmodified starting strain is a facultative anaerobic bacterium.

Embodiment B24: the modified bacterium of any one of the preceding embodiments B, wherein the bacterium is of *Enterobacteriaceae.*

Embodiment B25: the modified bacterium of any one of embodiments B, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment B26: the modified bacterium of any one of embodiments B, wherein the bacterium is selected from the group consisting of *Escherichia coli* (*E.coli*), *Enterobacter blattae* (*E.blattae*), *Enterobacter fergusonii* (*E.fergusonii*), *Enterobacter hermannii* (*E.hermannii*), *Enterobacter vulneris* (*E.vulneris*), *Salmonella enterica* (*S.enterica*), *Salmonella bongori* (*S.bongori*), *Salmonella typhi* (*S.typhi*), *Salmonella choleraesuis* (*S.choleraesuis*), *Salmonella typhimurium* (*S.typhimurium*), *Shigella dysenteriae* (*S.dysenteriae*), *Shigella flexneri* (*S.flexneri*), *Shigella boydii* (*S.boydii*), *Shigella sonnei* (*S.sonnei*), *Klebsiella pneumoniae* (*K.peumoniae*), *Klebsiella oxytoca* (*K.oxytoca*), *Yersinia pestis* (*Y.pestis*), *Yersinia enterocolitica* (*Y.enterocolitica*), *Yersinia pseudotuberculosis* (*Y.pseudoluherculosis*), *Yersinia aldouae* (*Y.aldouae*), *Yersinia bercovieri* (*Y.bercovieri*), *Yersinia frederiksenii* (*Y.frederiksenii*), *Yersinia intermedia* (*Y.intermedia*), *Yersinia kristersenii* (*Y.kristersenii*), *Yersinia mollaretti* (*Y.mollaretti*), *Yersinia rohdei* (*Y.rohdei*), *Yersinia ruckeri* (*Y.ruckeri*), *Citrobacter freundii* (*C.freundii*), *Citrobacter koseri* (*C.kaseri*), *Citrobacter braakii* (*C.braakii*), *Enterobacter aerogenes* (*E.aerogenes*), *Enterobacter cloacae* (*E.cloacae*)*, Enterobacter gergoviae* (*E.gergoviac*), *Enterobacter sakazakii* (*E.sakazakii*)*, Enterobacter taylorae* (*E.tavlorae*)*, Enterobacter amnigenus* (*E.aminigenus), Enterobacter intermedius* (*E.intermedius*), *Enterobacter asburiae* (*E.asburiac*), *Enterobacter cancerogenus* (*E.cancerogenus*), *Enterobacter dissolvens* (*E.dissolvens*), *Enterobacter nimipressurαlis* (*E.nimipressuralis*), *Serratia marcescens* (*S.marcescens*), *Serratia entomophila* (*S.entomophila*), *Serratia ficaria* (*S.ficaria*), *Serratia fonticola* (*S.fonticola*), *Serratia grimesii* (*S.grimesii*), *Serratia liquefaciens* (*S.liquefaciens*), *Serratia odorifera* (*S.odorifera*), *Serratia plymuthica* (*S.plymuthica*), *Serratia proteamaculans* (*S.proteamaculans*), *Serratia rubidaea* (*S.rubidaea*), *Serratia ureilytica* (*S.ureilytica*), *Proteus mirabilis* (*P.mirabilis*), *Proteus vulgaris* (*P.vulgaris*), *Proteus myxofaciens* (*P.myxofaciens*), *Proteus penneri* (*P.penneri*), *Proteus hauseri* (*P.hauseri*))*, Morganella morganii* (*M.morganii*), *Providencia alcalifaciens* (*P.alcalifaciens*), *Providencia rustigianii* (*P.rustigianii*), *Providencia stuartii* (*P.stuartii*), *Providencia rettgeri* (*P.rettgeri*), *Providencia heimbachae* (*P.heimbochae*). *Hafnia alvei* (*H.alvei*), and *Pantoea agglomerans* (*P.agglomerans*)..

Embodiment B27: the modified bacterium of any one of the preceding embodiments B, wherein the bacterium is of *Salmonella typhimurium.*

Embodiment B28: the modified bacterium of embodiment B27, wherein the starting strain is *Salmonella typhimurium* SL7207.

Embodiment B29: the modified bacterium of any one of the preceding embodiments B, wherein the bacterium expresses a wild-type lipopolysaccharide (LPS).

Embodiment B30: the modified bacterium of any one of the preceding embodiments B, wherein, when administrated to a subject with a tumor, the bacterium is capable of surviving and proliferating in tumor tissue and is rapidly cleared in normal tissue.

Embodiment B31: the modified bacterium of any one of the preceding embodiments B, wherein, when administrated to a subject with a malignant tumor, the bacterium is capable of inhibiting the growth of the malignant tumor.

Embodiment B32: the modified bacterium of any one of the preceding embodiments B, wherein, when administrated to a subject with a malignant tumor, the bacterium is capable of inducing an anti-tumor specific immune response.

Embodiment B33: the modified bacterium of any one of the preceding embodiments B, wherein, when administrated to a subject with a malignant tumor, the bacterium is capable of inducing anti-tumor immune memory.

Embodiment B34: the modified bacterium of any one of the preceding embodiments A, which does not express a wild-type flagellin.

Embodiment B35: the modified bacterium of any one of the preceding embodiments B, which is deficient in the *fliC* gene.

Embodiment B36: the modified bacterium of any one of the preceding embodiments B, wherein the survival level of the bacterium in macrophages ranges from about 50% to about 30% of the survival level of the unmodified starting strain.

Embodiment B37: the modified bacterium of any one of embodiments B1-B35, wherein the survival level of the bacterium in macrophages ranges from about 30% to about 10% of the survival level of the unmodified starting strain.

Embodiment B38: the modified bacterium of any one of embodiments B1-B35, wherein the survival level of the bacterium in macrophages ranges from about 10% to about 1% of the survival level of the unmodified starting strain.

### Embodiment C

Embodiment C1: a modified bacterium of *Salmonella typhimurium,* wherein the bacterium comprise a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

Embodiment C2: the modified bacterium of embodiment C1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment C3: the modified bacterium of embodiment C1, wherein the strictly hypoxia inducible promoter is ssbp1 promoter.

Embodiment C4: the modified bacterium of any one of the preceding embodiments C, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment C5: the modified bacterium of embodiment C4, wherein the essential gene is selected from *dapA, dapB, dapd, dapE, argD, dapF,* and any combination thereof.

Embodiment C6: the modified bacterium of embodiment C4, wherein the essential gene is selected from *dapA* and *dapE.*

Embodiment C7: the modified bacterium of any one of the preceding embodiments C, wherein the gene involved in or regulating the endogenous anti-oxidative stress response pathway is an HtrA serine protease family gene.

Embodiment C8: the modified bacterium of any one of the preceding embodiments C, wherein the functional expression product of the gene involved in or regulating the endogenous anti-oxidative stress response pathway is an HtrA serine protease family related protein.

Embodiment C9: the modified bacterium of any one of the preceding embodiments C, wherein the bacterium is deficient in the activity of HtrA serine protease.

Embodiment C10: the modified bacterium of any one of the preceding embodiments C, wherein the gene involved in or regulating the endogenous anti-oxidative stress response pathway is *htrA.*

Embodiment C11: the modified bacterium of any one of the preceding embodiments C, wherein the bacterium is deficient in *htrA.*

Embodiment C12: the modified bacterium of any one of embodiments C1-C11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment C13: the modified bacterium of any one of embodiments C1-C11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment C14: the modified bacterium of embodiment C13, wherein the exogenous essential gene expression cassette is integrated into the bacterial chromosome.

Embodiment C15: the modified bacterium of embodiment C13, wherein the exogenous essential gene expression cassette is outside the bacterial chromosome.

Embodiment C16: the modified bacterium of embodiment C15, wherein the exogenous essential gene expression cassette is present in the plasmid carried by the bacterium.

Embodiment C17: the modified bacterium of embodiments any one of the preceding embodiments C, wherein the bacteria-derived hemolysin protein is a gram-negative bacterial hemolysin protein.

Embodiment C18: the modified bacterium of embodiment C17, wherein the gene encoding the gram-negative bacterial hemolysin protein is *hlyA* or *hlyE.*

Embodiment C19: the modified bacterium of embodiment C18, wherein the hlyA or *hlyE* is derived from *Listeria monocytogenes, Vibrio cholerae,* or *Escherichia coli.*

Embodiment C20: the modified bacterium of any one of the preceding embodiments C, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment C21: the modified bacterium of any one of the preceding embodiments C, wherein the promoter that is active under acid pH condition is selected from the group consisting of sseA, ssrA, ssaB, ssaG, ssaM, and ssaR.

Embodiment C22: the modified bacterium of any one of the preceding embodiments C, wherein the promoter that is active under acid pH condition is sseA.

Embodiment C23: the modified bacterium of any one of the preceding embodiments C, wherein the unmodified starting strain is a facultative anaerobic bacterium.

Embodiment C24: the modified bacterium of any one of the preceding embodiments C, wherein the starting strain is *Salmonella typhimurium* SL7207.

Embodiment C25: the modified bacterium of any one of embodiments C1-C24, wherein the bacterium expresses a wild-type lipopolysaccharide (LPS).

Embodiment C26: the modified bacterium of any one of the preceding embodiments C, wherein, when administrated to a subject with a tumor, the bacterium is capable of surviving and proliferating in tumor tissue but is rapidly cleared in normal tissue.

Embodiment C27: the modified bacterium of any one of the preceding embodiments C, wherein, when administrated to a subject with a malignant tumor, the bacterium is capable of inhibiting the growth of the malignant tumor.

Embodiment C28: the modified bacterium of any one of the preceding embodiments C, wherein, when administrated to a subject with a malignant tumor, the bacterium is capable of inducing an anti-tumor specific immune response.

Embodiment C29: the modified bacterium of any one of the preceding embodiments C, wherein, when administrated to a subject with a malignant tumor, the bacterium is capable of inducing anti-tumor immune memory.

Embodiment C30: the modified bacterium of any one of the preceding embodiments C, which does not express a wild-type flagellin.

Embodiment C31: the modified bacterium of any one of the preceding embodiments C, which is deficient in the *fliC* gene.

Embodiment C32: the modified bacterium of any one of the preceding embodiments C, wherein the survival level of the bacterium in macrophages ranges from about 50% to about 30% of the survival level of the unmodified starting strain.

Embodiment C33: the modified bacterium of any one of embodiments C1-C31, wherein the survival level of the bacterium in macrophages ranges from about 30% to about 10% of the survival level of the unmodified starting strain.

Embodiment C34: the modified bacterium of any one of embodiments C1-C31, wherein the survival level of the bacterium in macrophages ranges from about 10% to about 1% of the survival level of the unmodified starting strain.

### Embodiment D

Embodiment D1: a modified bacterium, wherein the bacterium comprise a hypoxia regulated essential gene expression cassette comprising an essential gene of the bacterium under the control of a strictly hypoxia inducible promoter, and the bacterium is deficient in at least one gene required for survival in macrophages or a functional expression product thereof, as compared to an unmodified starting strain.

Embodiment D2: the modified bacterium of embodiment D1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment D3: the modified bacterium of embodiment D1, wherein the strictly hypoxia inducible promoter is ssbp1 promoter.

Embodiment D4: the modified bacterium of any one of the preceding embodiments D, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment D5: the modified bacterium of embodiment D4, wherein the essential gene is selected from *dapA, dapB, dapd, dapE, argD, dapF,* and any combination thereof.

Embodiment D6: the modified bacterium of embodiment D4, wherein the essential gene is selected from *dapA* and *dapE.*

Embodiment D7: the modified bacterium of any one of the preceding embodiments D, wherein the gene required for survival in macrophages is *STM3120, STM3119, slyA, sifA, SPI-2, phoP,* or *htrA* gene.

Embodiment D8: the modified bacterium of any one of the preceding embodiments D, wherein the functional expression product of the gene required for survival in macrophages is an HtrA serine protease family related protein.

Embodiment D9: the modified bacterium of any one of the preceding embodiments D, wherein the bacterium is deficient in the activity of HtrA serine protease.

Embodiment D10: the modified bacterium of any one of the preceding embodiments D, wherein the gene required for survival in macrophages is *htrA.*

Embodiment D11: the modified bacterium of any one of the preceding embodiments D, wherein the bacterium is deficient in *htrA.*

Embodiment D12: the modified bacterium of any one of embodiments D1-D11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment D13: the modified bacterium of any one of embodiments D1-D11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment D14: the modified bacterium of embodiment D13, wherein the exogenous essential gene expression cassette is integrated into the bacterial chromosome.

Embodiment D15: the modified bacterium of embodiment D13, wherein the exogenous essential gene expression cassette is outside the bacterial chromosome.

Embodiment D16: the modified bacterium of embodiments D15, wherein the exogenous essential gene expression cassette is present in the plasmid carried by the bacterium.

Embodiment D17: the modified bacterium of any one of the preceding embodiments D, which further comprises a pH regulated expression cassette, wherein the pH regulated expression cassette comprises a gene encoding a bacteria-derived hemolysin protein under the control of a promoter that is active under acid pH condition.

Embodiment D18: the modified bacterium of embodiments D17, wherein the bacteria-derived hemolysin protein is a gram-negative bacterial hemolysin protein.

Embodiment D19: the modified bacterium of embodiments D18, wherein the gene encoding the gram-negative bacterial hemolysin protein is *hlyA* or *hlyE.*

Embodiment D20: the modified bacterium of embodiments D19, wherein the hlyA or *hlyE* is derived from *Listeria monocytogenes, Vibrio cholerae,* or *Escherichia coli.*

Embodiment D21: the modified bacterium of any one of embodiments D17-D20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment D22: the modified bacterium of any one of embodiments D17-D21, wherein the promoter that is active under acid pH condition is selected from the group consisting of sseA, ssrA, ssaB, ssaG, ssaM, and ssaR.

Embodiment D23: the modified bacterium of any one of embodiments D17-D21, wherein the promoter that is active under acid pH condition is sseA.

Embodiment D24: the modified bacterium of any one of the preceding embodiments D, wherein the unmodified starting strain is a facultative anaerobic bacterium.

Embodiment D25: the modified bacterium of any one of the preceding embodiments D, wherein the bacterium is of *Enterobacteriaceae.*

Embodiment D26: the modified bacterium of any one of the preceding embodiments D, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganellα sp., Providencia sp., Hafnia sp.,* and *Pantoea sp.*

Embodiment D27: the modified bacterium of any one of the preceding embodiments D, wherein the bacterium is selected from the group consisting of *Escherichia coli* (*E.coli*), *Enterobacter blattae* (*E.blattae*), *Enterobacter fergusonii* (*E.fergusonii*), *Enterobacter hermannii* (*E.hermannii*), *Enterobacter vulneris* (*E.vulneris*), *Salmonella enterica* (*S.enterica*)*, Salmonella bongori* (*S.bongori*), *Salmonella typhi* (*S.typhi*), *Salmonella choleraesuis* (*S.choleraesuis*), *Salmonella typhimurium* (*S.typhimurium*), *Shigella dysenteriae* (*S.dysenteriae*)*, Shigella flexneri* (*S.flexneri*)*, Shigella boydii* (*S.boydii*)*, Shigella sonnei* (*S.sonnei*), *Klebsiella pneumoniae* (*K.peumoniae*), *Klebsiella oxytoca* (*K.oxytoca*), *Yersinia pestis* (*Y.pestis*), *Yersinia enterocolitica* (*Y.enterocolitica*)*, Yersinia pseudotuberculosis* (*Y.pseudotuberculosis*), *Yersinia aldouae* (*Y.aldouae*), *Yersinia bercovieri* (*Y.bercovieri*), *Yersinia frederiksenii* (*Y.frederiksenii*), *Yersinia intermedia* (*Y.intermedia*), *Yersinia kristersenii* (*Y.kristersenii*), *Yersinia mollaretti* (*Y.mollaretti*), *Yersinia rohdei* (*Y.rohdei*), *Yersinia ruckeri* (*Y.ruckeri*), *Citrobacter freundii* (*C.freundii*), *Citrobacter koseri* (*C.kaseri*), *Citrobacter braakii* (*C.braakii*), *Enterobacter aerogenes* (*E.aerogenes*), *Enterobacter cloacae* (*E.cloacae*), *Enterobacter gergoviae* (*E.gergoviac*), *Enterobacter sakazakii* (*E.sakazakii*)*, Enterobacter taylorae* (*E.tavlorae*)*, Enterobacter amnigenus* (*E.aminigenus*), *Enterobacter intermedius* (*E.intermedius*), *Enterobacter asburiae* (*E.asburiac*), *Enterobacter cancerogenus* (*E.cancerogenus*), *Enterobacter dissolvens* (*E.dissolvens*), *Enterobacter nimipressuralis* (*E.nimipressuralis*), *Serratia marcescens* (*S.marcescens*), *Serratia entomophila* (*S.entomophila*), *Serratia ficaria* (*S.ficaria*), *Serratia fonticola* (*S.fonticola*), *Serratia grimesii* (*S.grimesii*), *Serratia liquefaciens* (*S.liquefaciens*)*, Serratia odorifera* (*S.odorifera*)*, Serratia plymuthica* (*S.plymuthica*), *Serratia proteamaculans* (*S.proteamaculans*), *Serratia rubidaea* (*S.rubidaea*), *Serratia ureilytica* (*S.ureilytica*), *Proteus mirabilis* (*P.mirabilis*), *Proteus vulgaris* (*P.vulgaris*), *Proteus myxofaciens* (*P.myxofaciens*), *Proteus penneri* (*P.penneri*), *Proteus hauseri* (*P.hauseri*))*, Morganella morganii* (*M.morganii*), *Providencia alcalifaciens* (*P.alcalifaciens*), *Providencia rustigianii* (*P.rustigianii*), *Providencia stuartii* (*P.stuartii*), *Providencia rettgeri* (*P.rettgeri*), *Providencia heimbachae* (*P.heimbochae*), *Hafnia alvei* (*H.alvei*), and *Pantoea agglomerans* (*P.agglomerans*).

Embodiment D28: the modified bacterium of any one of the preceding embodiments D, wherein the bacterium is of *Salmonella typhimurium.*

Embodiment D29: the modified bacterium of embodiment D28, wherein the starting strain is *Salmonella typhimurium* SL7207.

Embodiment D30: the modified bacterium of any one of the preceding embodiments D, wherein the bacterium expresses a wild-type lipopolysaccharide (LPS).

Embodiment D31: the modified bacterium of any one of the preceding embodiments D, wherein, when administrated to a subject with a tumor, the bacterium is capable of surviving and proliferating in tumor tissue and is rapidly cleared in normal tissue.

Embodiment D32: the modified bacterium of any one of the preceding embodiments D, wherein, when administrated to a subject with a malignant tumor, the bacterium is capable of inhibiting the growth of the malignant tumor.

Embodiment D33: the modified bacterium of any one of the preceding embodiments D, wherein, when administrated to a subject with a malignant tumor, the bacterium is capable of inducing an anti-tumor specific immune response.

Embodiment D34: the modified bacterium of any one of the preceding embodiments D, wherein, when administrated to a subject with a malignant tumor, the bacterium is capable of inducing anti-tumor immune memory.

Embodiment D35: the modified bacterium of any one of the preceding embodiments D, which does not express a wild-type flagellin.

Embodiment D36: the modified bacterium of any one of the preceding embodiments D, which is deficient in the *fliC* gene.

Embodiment D37: the modified bacterium of any one of the preceding embodiments D, wherein the survival level of the bacterium in macrophages ranges from about 50% to about 30% of the survival level of the unmodified starting strain.

Embodiment D38: the modified bacterium of any one of embodiments D1-D36, wherein the survival level of the bacterium in macrophages ranges from about 30% to about 10% of the survival level of the unmodified starting strain.

Embodiment D39: the modified bacterium of any one of embodiments D1-D36, wherein the survival level of the bacterium in macrophages ranges from about 10% to about 1% of the survival level of the unmodified starting strain.

### Embodiment E

Embodiment E1: a modified bacterium, wherein the bacterium comprise a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof, as compared to an unmodified starting strain.

Embodiment E2: the modified bacterium of embodiment E1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment E3: the modified bacterium of embodiment E1, wherein the strictly hypoxia inducible promoter is ssbp1 promoter.

Embodiment E4: the modified bacterium of any one of the preceding embodiments E, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment E5: the modified bacterium of embodiment E4, wherein the essential gene is selected from *dapA, dapB, dapd, dapE, argD, dapF,* and any combination thereof.

Embodiment E6: the modified bacterium of embodiment E4, wherein the essential gene is selected from *dapA* and *dapE.*

Embodiment E7: the modified bacterium of any one of the preceding embodiments E, wherein the gene required for survival in macrophages is *STM3120, STM3119, slyA, sifA, SPI-2, phoP,* or *htrA* gene.

Embodiment E8: the modified bacterium of any one of the preceding embodiments E, wherein the functional expression product of the gene required for survival in macrophages is an HtrA serine protease family related protein.

Embodiment E9: the modified bacterium of any one of the preceding embodiments E, wherein the bacterium is deficient in the activity of HtrA serine protease.

Embodiment E10: the modified bacterium of any one of the preceding embodiments E, wherein the gene required for survival in macrophages is *htrA.*

Embodiment E11: the modified bacterium of any one of the preceding embodiments E, wherein the bacterium is deficient in *htrA.*

Embodiment E12: the modified bacterium of any one of embodiments E1-E11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment E13: the modified bacterium of any one of embodiments E1-E11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment E14: the modified bacterium of embodiment E13, wherein the exogenous essential gene expression cassette is integrated into the bacterial chromosome.

Embodiment E15: the modified bacterium of embodiment E13, wherein the exogenous essential gene expression cassette is outside the bacterial chromosome.

Embodiment E16: the modified bacterium of embodiments E15, wherein the exogenous essential gene expression cassette is present in the plasmid carried by the bacterium.

Embodiment E17: the modified bacterium of any one of the preceding embodiments E, wherein the bacteria-derived hemolysin protein is a gram-negative bacterial hemolysin protein.

Embodiment E18: the modified bacterium of embodiment E17, wherein the gene encoding the gram-negative bacterial hemolysin protein is *hlyA* or *hlyE.*

Embodiment E19: the modified bacterium of embodiment E18, wherein the hlyA or *hlyE* is derived from *Listeria monocytogenes, Vibrio cholerae,* or *Escherichia coli.*

Embodiment E20: the modified bacterium of any one of preceding embodiments E, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment E21: the modified bacterium of any one of embodiments E, wherein the promoter that is active under acid pH condition is selected from the group consisting of sseA, ssrA, ssaB, ssaG, ssaM, and ssaR.

Embodiment E22: the modified bacterium of any one of embodiments E, wherein the promoter that is active under acid pH condition is sseA.

Embodiment E23: the modified bacterium of any one of embodiments E, wherein the unmodified starting strain is a facultative anaerobic bacterium.

Embodiment E24: the modified bacterium of any one of embodiments E, wherein the bacterium is of *Enterobacteriaceae.*

Embodiment E25: the modified bacterium of any one of embodiments E, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment E26: the modified bacterium of any one of the preceding embodiments E, wherein the bacterium is selected from the group consisting of *Escherichia coli* (*E.coli*), *Enterobacter blattae* (*E.blattae), Enterobacter fergusonii* (*E.fergusonii*), *Enterobacter hermannii* (*E.hermannii*), *Enterobacter vulneris* (*E.vulneris*)*, Salmonella enterica* (*S.enterica*), *Salmonella bongori* (*S.bongori*), *Salmonella typhi* (*S.typhi*), *Salmonella choleraesuis* (*S.choleraesuis*), *Salmonella typhimurium* (*S.typhimurium*), *Shigella dysenteriae* (*S.dysenteriae*), *Shigella flexneri* (*S.flexneri*), *Shigella boydii* (*S.boydii*), *Shigella sonnei* (*S.sonnei*), *Klebsiella pneumoniae* (*K.peumoniae*), *Klebsiella oxytoca* (*K.oxytoca*), *Yersinia pestis* (*Y.pestis*), *Yersinia enterocolitica* (*Y.enterocolitica*), *Yersinia pseudotuberculosis* (*Y.pseudotuberculosis*), *Yersinia aldouae* (*Y.aldouae*), *Yersinia bercovieri* (*Y.bercovieri*), *Yersinia frederiksenii* (*Y.frederiksenii*), *Yersinia intermedia* (*Y.intermedia*), *Yersinia kristersenii* (*Y.kristersenii*), *Yersinia mollaretti* (*Y.mollaretti*), *Yersinia rohdei* (*Y.rohdei*), *Yersinia ruckeri* (*Y.ruckeri*), *Citrobacter freundii* (*C.freundii*), *Citrobacter koseri* (*C.kaseri*), *Citrobacter braakii* (*C.braakii*), *Enterobacter aerogenes* (*E.aerogenes*), *Enterobacter cloacae* (*E.cloacae*), *Enterobacter gergoviae* (*E.gergoviac*), *Enterobacter sakazakii* (*E.sakazakii*), *Enterobacter taylorae* (*E.tavlorae*), *Enterobacter amnigenus* (*E.aminigenus*), *Enterobacter intermedius* (*E.intermedius*), *Enterobacter asburiae* (*E.asburiac*), *Enterobacter cancerogenus* (*E.cancerogenus*), *Enterobacter dissolvens* (*E.dissolvens*), *Enterobacter nimipressurαlis* (*E.nimipressuralis*), *Serratia marcescens* (*S.marcescens*), *Serratia entomophila* (*S.entomophila*), *Serratia ficaria* (*S.ficaria*), *Serratia fonticola* (*S.fonticola*), *Serratia grimesii* (*S.grimesii*), *Serratia liquefaciens* (*S.liquefaciens*), *Serratia odorifera* (*S.odorifera*), *Serratia plymuthica* (*S.plymuthica*), *Serratia proteamaculans* (*S.proteamaculans*), *Serratia rubidaea* (*Srubidaea*), *Serratia ureilytica* (*S.ureilytica*), *Proteus mirabilis* (*P.mirabilis*), *Proteus vulgaris* (*P.vulgaris*), *Proteus myxofaciens* (*P.myxofaciens*), *Proteus penneri* (*P.penneri*), *Proteus hauseri* (*P.hauseri*))*, Morganella morganii* (*M.morganii*), *Providencia alcalifaciens* (*P.alcalifaciens*), *Providencia rustigianii* (*P.rustigianii*), *Providencia stuartii* (*P.stuartii*), *Providencia rettgeri* (*P.rettgeri*), *Providencia heimbachae* (*P.heimbochae*)*, Hafnia alvei* (*H.alvei*), and *Pantoea agglomerans* (*P.agglomerans*).

Embodiment E27: the modified bacterium of any one of the preceding embodiments E, wherein the bacterium is of *Salmonella typhimurium.*

Embodiment E28: the modified bacterium of embodiment E27, wherein the starting strain is *Salmonella typhimurium* SL7207.

Embodiment E29: the modified bacterium of any one of the preceding embodiments E, wherein, when administrated to a subject with a tumor, the bacterium is capable of surviving and proliferating in tumor tissue and is rapidly cleared in normal tissue.

Embodiment E30: the modified bacterium of any one of the preceding embodiments E, wherein, when administrated to a subject with a malignant tumor, the bacterium is capable of inhibiting the growth of the malignant tumor.

Embodiment E31: the modified bacterium of any one of the preceding embodiments E, wherein, when administrated to a subject with a malignant tumor, the bacterium is capable of inducing an anti-tumor specific immune response.

Embodiment E32: the modified bacterium of any one of the preceding embodiments E, wherein, when administrated to a subject with a malignant tumor, the bacterium is capable of inducing anti-tumor immune memory.

Embodiment E33: the modified bacterium of any one of the preceding embodiments E, which does not express a wild-type flagellin.

Embodiment E34: the modified bacterium of any one of the preceding embodiments E, which is deficient in the *fliC* gene.

Embodiment E35: the modified bacterium of any one of the preceding embodiments E, wherein the survival level of the bacterium in macrophages ranges from about 50% to about 30% of the survival level of the unmodified starting strain.

Embodiment E36: the modified bacterium of any one of embodiments E1-E34, wherein the survival level of the bacterium in macrophages ranges from about 30% to about 10% of the survival level of the unmodified starting strain.

Embodiment E37: the modified bacterium of any one of embodiments E1-E34, wherein the survival level of the bacterium in macrophages ranges from about 10% to about 1% of the survival level of the unmodified starting strain.

### Embodiment F

Embodiment F1: a modified bacterium of *Salmonella typhimurium,* wherein the bacterium comprise a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of an promoter that is active under acid pH condition, and the bacterium expresses a wild-type lipopolysaccharide (LPS) and is deficient in at least one gene required for survival in macrophages or a functional expression product, as compared to an unmodified starting strain.

Embodiment F2: the modified bacterium of embodiment F1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment F3: the modified bacterium of embodiment F1, wherein the strictly hypoxia inducible promoter is ssbp1 promoter.

Embodiment F4: the modified bacterium of any one of the preceding embodiments F, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment F5: the modified bacterium of embodiment F4, wherein the essential gene is selected from *dapA, dapB, dapd, dapE, argD, dapF,* and any combination thereof.

Embodiment F6: the modified bacterium of embodiment F4, wherein the essential gene is selected from *dapA* and *dapE.*

Embodiment F7: the modified bacterium of any one of the preceding embodiments F, wherein the gene required for survival in macrophages is *STM3120, STM3119, slyA, sifA, SPI-2, phoP,* or *htrA* gene.

Embodiment F8: the modified bacterium of any one of the preceding embodiments F, wherein the gene required for survival in macrophages is the gene involved in or regulating the endogenous anti-oxidative stress response pathway.

Embodiment F9: the modified bacterium of embodiment F8, wherein the gene involved in or regulating the endogenous anti-oxidative stress response pathway is an HtrA serine protease family gene.

Embodiment F10: the modified bacterium of embodiment F9, wherein the functional expression product of the gene involved in or regulating the endogenous anti-oxidative stress response pathway is an HtrA serine protease family related protein.

Embodiment F11: the modified bacterium of embodiment F9, wherein the bacterium is deficient in the activity of HtrA serine protease.

Embodiment F12: the modified bacterium of embodiment F9, wherein the gene involved in or regulating the endogenous anti-oxidative stress response pathway is *htrA.*

Embodiment F13: the modified bacterium of embodiment F9, wherein the bacterium is deficient in *htrA.*

Embodiment F14: the modified bacterium of any one of embodiments F1-F13, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment F15: the modified bacterium of any one of embodiments F1-F13, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment F16: the modified bacterium of embodiment F15, wherein the exogenous essential gene expression cassette is integrated into the bacterial chromosome.

Embodiment F17: the modified bacterium of embodiment F15, wherein the exogenous essential gene expression cassette is outside the bacterial chromosome.

Embodiment F18: the modified bacterium of embodiments F17, wherein the exogenous essential gene expression cassette is present in the plasmid carried by the bacterium.

Embodiment F19: the modified bacterium of any one of the preceding embodiments F, wherein the bacteria-derived hemolysin protein is a gram-negative bacterial hemolysin protein.

Embodiment F20: the modified bacterium of embodiment F19, wherein the gene encoding the gram-negative bacterial hemolysin protein is *hlyA* or *hlyE.*

Embodiment F21: the modified bacterium of embodiment F20, wherein the hlyA or *hlyE* is derived from *Listeria monocytogenes, Vibrio cholerae,* or *Escherichia coli.*

Embodiment F22: the modified bacterium of any one of the preceding embodiments F, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment F23: the modified bacterium of any one of the preceding embodiments F, wherein the promoter that is active under acid pH condition is selected from the group consisting of sseA, ssrA, ssaB, ssaG, ssaM, and ssaR.

Embodiment F24: the modified bacterium of any one of the preceding embodiments F, wherein the promoter that is active under acid pH condition is sseA.

Embodiment F25: the modified bacterium of any one of the preceding embodiments F, wherein the starting strain is *Salmonella typhimurium* SL7207.

Embodiment F26: the modified bacterium of any one of the preceding embodiments F, wherein, when administrated to a subject with a tumor, the bacterium is capable of surviving and proliferating in tumor tissue and is rapidly cleared in normal tissue.

Embodiment F27: the modified bacterium of any one of the preceding embodiments F, wherein, when administrated to a subject with a malignant tumor, the bacterium is capable of inhibiting the growth of the malignant tumor.

Embodiment F28: the modified bacterium of any one of the preceding embodiments F, wherein, when administrated to a subject with a malignant tumor, the bacterium is capable of inducing an anti-tumor specific immune response.

Embodiment F29: the modified bacterium of any one of the preceding embodiments F, wherein, when administrated to a subject with a malignant tumor, the bacterium is capable of inducing anti-tumor immune memory.

Embodiment F30: the modified bacterium of any one of the preceding embodiments F, which does not express a wild-type flagellin.

Embodiment F31: the modified bacterium of any one of the preceding embodiments F, which is deficient in *the fliC* gene.

Embodiment F32: the modified bacterium of any one of the preceding embodiments F, wherein the survival level of the bacterium in macrophages ranges from about 50% to about 30% of the survival level of the unmodified starting strain.

Embodiment F33: the modified bacterium of any one of embodiments F1-F31, wherein the survival level of the bacterium in macrophages ranges from about 30% to about 10% of the survival level of the unmodified starting strain.

Embodiment F34: the modified bacterium of any one of embodiments F1-F31, wherein the survival level of the bacterium in macrophages ranges from about 10% to about 1% of the survival level of the unmodified starting strain.

### Embodiment K

Embodiment K1: a pharmaceutical composition comprising an effective amount of the modified bacterium of any one of the preceding embodiments A, embodiments B, embodiments C, embodiments D, embodiment E, or embodiment F.

Embodiment K2: the pharmaceutical composition of embodiment K1, which is used for treating a malignant tumor.

Embodiment K3: the pharmaceutical composition of embodiment K1, which is used for inducing an anti-tumor specific immune response in a subject with a malignant tumor.

Embodiment K4: the pharmaceutical composition of embodiment K1, which is used for inducing anti-tumor immune memory in a subject with a malignant tumor.

Embodiment K5: the pharmaceutical composition of embodiment K1, which is used for preventing or treating metastasis or recurrence of a malignant tumor.

Embodiment K6: the pharmaceutical composition of embodiment K1, which is used for treating a malignant tumor that is resistant to, or has failed to, a prior anti-tumor therapy.

Embodiment K7: the pharmaceutical composition of embodiments K1-K6, wherein the modified bacterium is a live bacterium.

### Embodiment L

Embodiment L1: a method for treating a malignant tumor, comprising administering to a subject suffering the malignant tumor an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L2: a method for inducing an anti-tumor specific immune response in a subject with a malignant tumor, comprising administering to the subject an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L3: a method for inducing anti-tumor immune memory in a subject with a malignant tumor, comprising administering to the subject an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L4: a method for preventing or treating metastasis or recurrence of a malignant tumor, comprising administering to a subject with the malignant tumor an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L5: a method for preventing or treating metastasis or recurrence of a malignant tumor, comprising administering to a subject with or at a high risk of metastasis or recurrence of the malignant tumor an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L6: a method for treating a malignant tumor that is resistant to, or has failed to, a prior anti-tumor therapy, comprising administering to a subject with the malignant tumor that is resistant to, or has failed to, the prior anti-tumor therapy, an effective amount of the modified bacterium of any one of the preceding embodiments A, embodiments B, embodiments C, embodiments D, embodiments E, or embodiments F, or the pharmaceutical composition of any one of the preceding embodiments G.

Embodiment L7: the method of embodiments L1-L6, wherein the modified bacterium is a live bacterium.

### Embodiment M

Embodiment M1: use of the modified bacterium of any one of the preceding embodiments A, embodiments B, embodiments C, embodiments D, embodiments E, or embodiments F in the manufacture of a medicament for treating a malignant tumor.

Embodiment M2: use of the modified bacterium of any one of the preceding embodiments A, embodiments B, embodiments C, embodiments D, embodiments E, or embodiments F in the manufacture of a medicament for inducing an anti-tumor specific immune response in a subject with a malignant tumor.

Embodiment M3: use of the modified bacterium of any one of the preceding embodiments A, embodiments B, embodiments C, embodiments D, embodiments E, or embodiments F in the manufacture of a medicament for inducing anti-tumor immune memory in a subject with a malignant tumor.

Embodiment M4: use of the modified bacterium of any one of the preceding embodiments A, embodiments B, embodiments C, embodiments D, embodiments E, or embodiments F in the manufacture of a medicament for preventing or treating metastasis or recurrence of a malignant tumor.

Embodiment M5: use of the modified bacterium of any one of the preceding embodiments A, embodiments B, embodiments C, embodiments D, embodiments E, or embodiments F in the manufacture of a medicament for treating a malignant tumor that is resistant to, or has failed to, a prior anti-tumor therapy. Embodiment M7: the use of embodiments M1-M5, wherein the modified bacterium is a live bacterium.

### Embodiment N

Embodiment N1: the pharmaceutical composition of any one of embodiments K, the method of any one of embodiments L, or the use of any one of embodiments M, wherein the malignant tumor is a malignant tumor from the nervous system, respiratory system, digestive system, urinary system, reproductive system, hematopoietic system, lymphatic system, endocrine system, or skin mucosa.

Embodiment N2: the pharmaceutical composition of any one of embodiments K, the method of any one of embodiments L, or the use of any one of embodiments M, wherein the malignant tumor is a sarcoma or cancer.

Embodiment N3: the pharmaceutical composition of any one of embodiments K, the method of any one of embodiments L, or the use of any one of embodiments M, wherein the malignant tumor is a solid tumor.

Embodiment N4: the pharmaceutical composition of any one of embodiments K, the method of any one of embodiments L, or the use of any one of embodiments M, wherein malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

Embodiment N5: the pharmaceutical composition of any one of embodiments K, the method of any one of embodiments L, or the use of any one of embodiments M, wherein the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes.

### Examples

The following embodiments are used as examples to illustrate certain specific features and/or embodiments of the invention. These embodiments should not be interpreted as limiting the invention to the specific features or embodiments described.

### Example 1: Construction of different variant strains of Salmonella typhimurium SL7207 strain

1.1 Desired fragments were obtained by PCR amplification using *PsseA-hlyA*-loxp-KnaR-loxp plasmid (SEQ ID NO: 9) and *Pssbp1-dapE-*Cm plasmid (SEQ ID NO: 10) as templates. For *PsseA-hlyA-loxp-KnaR-loxp,* the following primer sequences were used: a forward primer TTCACAGAAAAGTGTTGCCCCCTTCCATGGCGGAAGGGGGACAAAGGTGAATTTGTCCTACTC AGGAGAGAGCGTTCA (SEQ ID NO:11) and a reverse primer TCTGACGTACACAGCAATTTTGCGT TACCTGTTAATCGAGATTGAAACACCGAAGAAAGGCCCACCCG (SEQ ID NO:12); for *Pssbp1-dapE-Cm,* the following primer sequences were used: a forward primer TTGGTTTCAGTGAATCCCGTTATCAGCAGTTTTTTGATGAGGTGTAGTCTATTTGTCCTACTCAG GAGAGCG (SEQ ID NO:13) and a reverse primer CAATATTTTGCCAGCCAGTCCATGCTTATTTCCT CTTACCGGAACGCTCACGAAGAAAGGCCCACCCG (SEQ ID NO:14), and the annealing temperature is 55°C. Desired fragment 1 was *PsseA-hlyA-*loxp-KnaR-*loxp* (SEQ ID NO: 1) and desired fragment 2 was*Pssbp1-dapE-Cm* (SEQ ID NO: 2).

1.2 The pSim6 plasmid (BioVector NTCC Inc.: 3574840) containing the λ phage Red recombinase was introduced into a facultative anaerobic Salmonella strain SL7207 (NCBI: ASM1320710v1) to prepare a pSim6 plasmid-containing SL7207 strain electrocompetent cells SL7207 (pSim6).

1.3 Desired fragment 1 was introduced into SL7207 (pSim6) competent cells by electroporation (E=18KV/cm). The *dapE* gene (which regulates synthesis of diaminopimelic acid essential for cell wall synthesis) in the SL7207 genome was replaced with desired fragment 1 using λ-red homologous recombination. Specifically, the pSim6 plasmid-containing target strain was cultured at 30°C; the recombinase expression was conducted at 42°C for 15 min; the homologous recombination process was completed by adding homologous arms containing 50 bp away from each upstream and downstream of the target gene locus to the PCR primers under the effect of a homologous recombinase to obtain the strain SL7207 (*ΔdapE*::*PsseA-hlyA-KnaR*)*.*

1.4 The Cre plasmid (Gene Bridges:A112) containing the P1 phage Cre recombinase was introduced into the strain SL7207 (*ΔdapE::PsseA-hlyA-KnaR*) for recombination to remove kanamycin resistance, thereby obtaining the strain SL7207 (*ΔdapE::PsseA-hlyA*)*.*

1.5 The pSim6 plasmid was introduced into the strain SL7207 (*ΔdapE::PsseA-hlyA*) to prepare SL7207 (*ΔdapE::PsseA-hlyA*) (pSim6) electrocompetent cells.

1.6 Desired fragment 2 was introduced into SL7207 (Δ*dapE::PsseA-hlyA*) (pSim6) electrocompetent cells by electroporation (E=18KV/cm). The *htrA* gene (encoding a serine protease) in the SL7207 genome (*ΔdapE::PsseA-hlyA*) was replaced with desired fragment 2 using λ-red homologous recombination to obtain the strain DB-ZW1, which was an SL7207 strain (*ΔdapE::PsseA-hlyA*; Δ*htrA::Pssbp1-dapE-Cm*) deficient in *htrA* gene, containing an *hlyA* gene under the control of *PsseA* and a *dapE* gene under the control of a strictly hypoxia inducible promoter *Pssbp1.*

1.7 Preparation of a *PdapE-dapE-loxp-KnaR-loxp* PCR product using the forward primer CAATATTTTGCCAGCCAGTCCATGCTTATTTCCTCTTACCGGAACGCTCAATTTGTCCTACTCAG (SEQ ID NO:15) and the reverse primer TTGGTTTCAGTGAATCCCGTTATCAGCAGTTTTTTGATGA GGTGTAGTCTCGAAGAAAGGCCCACCCG (SEQ ID NO:16) at an annealing temperature of 60°C. The *PdapE-dapE-loxp-KnaR-loxp* PCR product (SEQ ID NO: 4) was transferred into DB-ZW1 (pSim6) competent cells by electroporation (E=18KV/cm) using λ-red homologous recombination to replace the *Pssbp1-dapE* fragment to obtain the DB-ZW2 strain. It was a SL7207 strain deficient in the *htrA* gene, containing the *hlyA* gene under the control of the promoter *PsseA* and the *dapE* gene under the control of its natural promoter *PdapE.*

1.8 Preparation of a loxp-KnaR-loxp PCR product using the forward primer ATGAAAAAAATAATGCTAGTTTTTATTACACTTATATTAGTTAGTCTACCGCCGATCATATTCAATA ACCC (SEQ ID NO: 17) and the reverse primer TTATTCGATTGGATTATCTACTTTATTACTATATTTCG GATAAAGCGTGGCTAGTGAACCTCTTCGAGGG (SEQ ID NO: 18) at an annealing temperature of 55°C. The loxp-KnaR-loxp PCR product (SEQ ID NO: 3) was transferred into DB-ZW1 (pSim6) competent cells by electroporation (E=18KV/cm) using λ-red homologous recombination to replace the *hlyA* gene. This resulted in the DB-ZW3 strain, which was a SL7207 strain deficient in the *htrA* gene, containing the *dapE* gene under the control of a strictly hypoxia inducible promoter *Pssbp1* but without the *hlyA* gene.

1.9 Preparation of a *PhtrA-htrA*-loxp-aadA-lox*p* PCR product using the forward primer GATAATGTATTCACAGGTTGCTCCGGGCTTGAAAAAGTTAAATGACATCCATTTGTCCTACTCAG GAGAGC (SEQ ID NO: 19) and the reverse primer AAAAGGAACTTTTTACCTTTTCGCCTTCCCGTT TCGTTCAACTTAGTATACGAAGAAAGGCCCACCC (SEQ ID NO: 20) at an annealing temperature of 55°C. The *PhtrA-htrA-loxp-aadA-loxp* PCR product (SEQ ID NO: 5) was transferred into DB-ZW1 (pSim6) and DB-ZW3 (pSim6) competent cells by electroporation (E=18KV/cm) using λ-red homologous recombination and inserted to the *attB* site, to construct the strains DB-ZW4 and DB-ZW5, respectively. Among them, DB-ZW4 was a SL7207 strain containing the *htrA* gene under the control of the natural promoter, the *hlyA* gene under the control of the promoter *PsseA* and the *dapE* gene under the control of the promoter *Pssbp1;* DB-ZW5 was a SL7207 strain containing the *htrA* gene under the control of the natural promoter, the *dapE* gene under the control of the promoter *Pssbp1*, but without the *hlyA* gene.

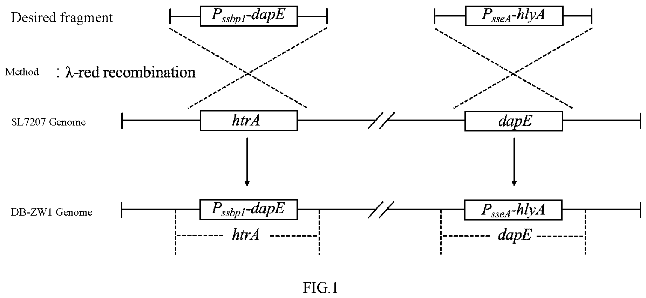

The strain construction scheme was shown in FIG.1. The successful construction of DB-ZW2, DB-ZW3, DB-ZW4 and DB-ZW5 strains was confirmed by PCR electrophoresis.

### Examples 2: Growth characteristics of DB-ZW1 and DB-ZW2 strains in different cells

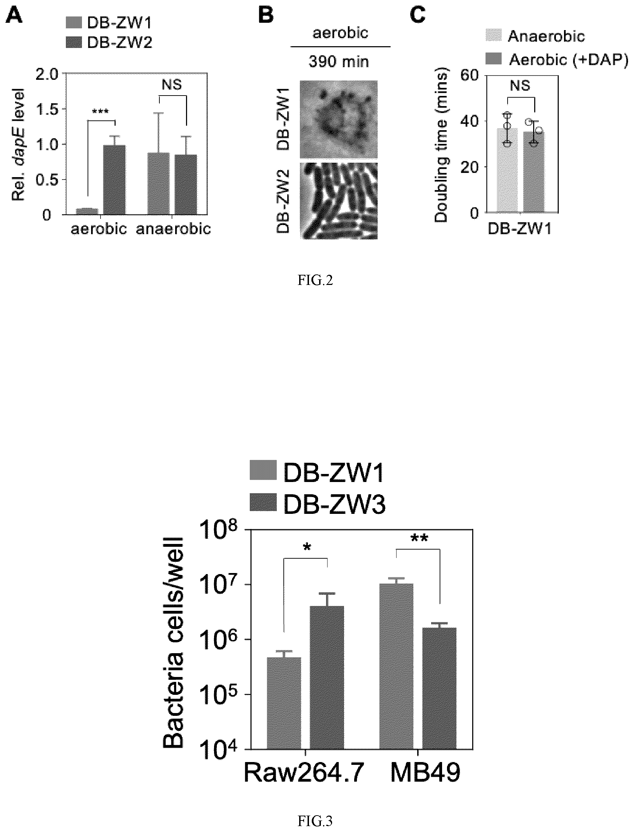

DB-ZW1 and DB-ZW2 were cultured in LB broth at 37°C under anaerobic and aerobic conditions, respectively, and when they were grown to an OD₆₀₀ of 0.2, bacteria were collected by centrifugation at 5000 rpm, washed with PBS. RNA was then extracted using Qiagen RNA Extraction Kit (74106), and after reverse transcription, the expression of *dapE* in each strain was measured using RT-qPCR. As compared to the strain DB-ZW2 carrying a natural promoter, the strain DB-ZW1 could transcribe and express the *dapE* gene more under anaerobic conditions, whereas transcription and expression of *dapE* gene in the strain DB-ZW1 were significantly reduced under aerobic conditions (FIG.2A). Meanwhile, the growth of the DB-ZW1 strain haboring an anaerobic gene circuit and the DB-ZW2 strain lacking the anaerobic gene circuit was observed under aerobic conditions. DB-ZW1 and DB-ZW2 were cultured in LB broth under aerobic conditions at 37°C, respectively, where 50 µg/mL of DAP was added to the DB-ZW1 medium. When cultured to an OD₆₀₀ of 0.2, both bacteria were collected by centrifugation at 5000 rpm, washed with PBS and then an appropriate amount of the bacterial solution was taken and added to a 2 cm glass-bottomed petri dish, and pressed with an agarose gel pad for observing under a microscope. It was found that after about 6 hours of incubation, all bacterial cells of the DB-ZW1 strain lysed and the bacteria died, whereas the DB-ZW2 strain lacking the anaerobic gene circuit, could survive and grow under aerobic conditions (FIG.2B). In addition, the doubling time of the DB-ZW1 strain was further measured to be approximately 38 minutes in an aerobic environment with the addition of DAP, and in an anaerobic environment (FIG.2C). The above results indicated that the anaerobic gene circuit was able to control the growth of strains under different oxygen conditions.

In addition, 2.5×10⁶ Raw 264.7 mouse macrophages (ATCC Number: TIB-71) or MB49 mouse bladder cancer cells (Merck SCC148) were added to each well of 6-well plates for cell culture, which were cultured in a complete medium (DMEM basal medium + 10% fetal bovine serum + double antibody) in a 5% CO₂ incubator at 37°C for cell adherence. Cells from two wells were counted, and the DB-ZW1 and DB-ZW3 strains (after counting, bacteria were resuspended in DMEM base media containing 50 µg/mL DAP) were added according to the number of cells, respectively, with an infestation ratio of 50:1, and co-cultured for 15 minutes or 1 hour to allow bacteria to enter into the cells. Afterward, the cells were washed with PBS and cultured with DMEM basic medium containing 100 µg/mL of gentamicin and 50 µg/mL of DAP for 1 hour. The supernatant was removed, and the cells were washed with PBS. After collection of cells, the membrane thereof was then ruptured, and the number of live bacteria inside the cells was determined by plating. It was verified by using macrophages and tumor cells that the DB-ZW1 strain harboring the pH-regulated gene circuit *PsseA-hlyA* was verified to be more quickly cleared by the mouse macrophage cell line Raw 264.7, while it showed good growth in the mouse bladder cancer cell line MB49 (FIG.3).

### Example 3: In vivo distribution of different variant strains

3.1 C57BL/6 mice (purchased from Beijing Vital River Laboratory Animal Technology Co., Ltd., weighing about 18 g, kept in a Specified Pathogen Free (SPF) environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

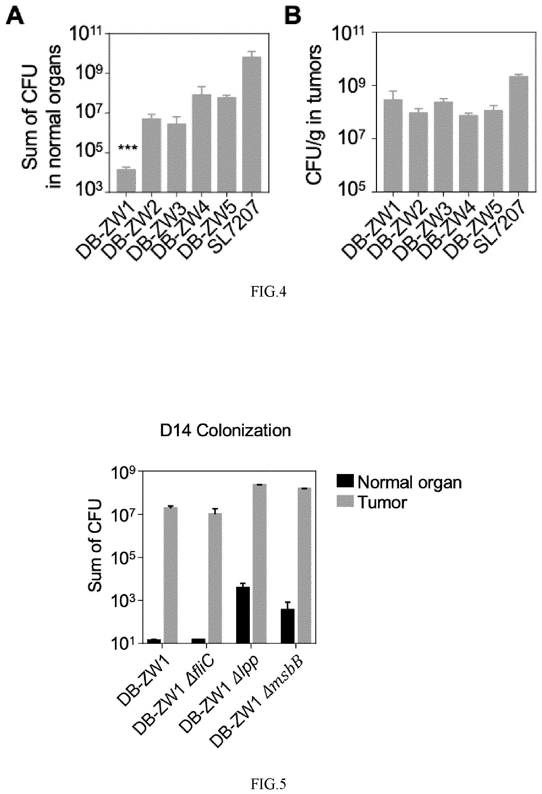

3.2 The tumor-bearing mice were divided into seven groups, each consisting of three mice: PBS group, DB-ZW1 group, DB-ZW2 group, DB-ZW3 group, DB-ZW4 group, DB-ZW5 group, and SL7207 group. The latter 6 groups of tumor-bearing mice were injected with 1×10⁷ CFU of bacteria in a volume of 125 µL via tail vein. The quantitative distribution of the above strains in normal tissues and organs of tumor-bearing mice was examined, respectively. It was found that 14 days after injection, only the DB-ZW1 strain could be rapidly cleared in normal tissues (FIG.4A) while aggregating rapidly in tumors (FIG.4B), compared with other groups of strains. Loss of the anaerobic regulatory gene circuit *Pssbp1-dapE* or the pH regulatory gene circuit *PsseA-hlyA* or containing the *htrA* gene would slow down the clearance of the DB-ZW1 strain in normal tissues.

### Example 4: Construction of DB-ZW1 variant strains

4.1 Desired fragments 3, 4 and 5 were obtained by PCR amplification by using the pBSK plasmid (Addgene: #67504) as a template, specifically as follows: *msbB*-HR-loxp-KnaR-loxp (SEQ ID NO: 6, the forward primer: AGGTAGTACAGGGTTTGTCAGCATAAAGCCTCTCTTACGAGAGGCTTTATGCCG ATCATATTCAATAACCC (SEQ ID NO:21); the reverse primer: TCGCTACACTATTCACAATTCCTTTT CGCGTCAGCAGACCCTGGAAAAGCCTAGTGAACCTCTTCGAGGG (SEQ ID NO:22); the annealing temperature: 55 °C); lpp-HR-loxp-KnaR-loxp (SEQ ID NO:7, the forward primer: CGGACAAAAAAATG GCGCACGATGTGCGCCATTTTATATTGTGCGTCAAAGCCGATCATATTCAATAACCC (SEQ ID NO:23); the reverse primer: AATACTTGTAACGCTACATGGAGATTAACTCAATCTAGAGGGTATTAA TACTAGTGAACCTCTTCGAGGG (SEQ ID NO:24); the annealing temperature: 55 °C) ; and fliC-HR-loxp-KnaR-loxp (SEQ ID NO:8, the forward primer: GCTTTCGCTGCCTTGATTGTGTACCAC GTGTCGGTGAATCAATCGCCGGAGCCGATCATATTCAATAACCC (SEQ ID NO:25); the reverse primer: ACGGTGAGAAACCGTGGGCAACAGCCCAATAACATCAAGTTGTAATTGATCTAGTGAAC CTCTTCGAGGG (SEQ ID NO:26); the annealing temperature: 55 °C.

4.2 To prepare DB-ZW1 strain electrocompetent cells, the pSim6 plasmid was transferred into the DB-ZW1 strain by electroporation.

4.3 To prepare DB-ZW1 (pSim6) electrocompetent cells, a DB-ZW1 (pSim6) bacteria solution cultured to log phase was washed twice with pre-cooled deionized water, then washed once with an equal volume of 10% glycerol, and finally resuspended in 10% glycerol, and 100 µL of electrocompetent cells were prepared from each 5 mL of log phase bacteria solution.

4.4 Desired fragments 3, 4 and 5 were introduced into DB-ZW1 (pSim6) electrocompetent cells, respectively. The *msbB* gene (encoding lipid acyltransferase, Gene ID: 1253410), the *lpp* gene (encoding lipoprotein, Gene ID: 1252895) and the *fliC* gene (encoding flagellin, Gene ID: 1253480) in the DB-ZW1 genome were replaced with Desired fragments 3, 4 and 5, respectively, by λ-red homologous recombination technology, to obtain the strains DB-ZW1 (*ΔmsbB*) *,* DB-ZW1 (*Δlpp*) and DB-ZW1 (*ΔfliC*) .

The strains DB-ZW1 (*ΔmsbB*) , DB-ZW1 (*Δlpp*) and DB-ZW1 (Δ*fliC*) were successfully constructed as confirmed by PCR electrophoresis.

### Example 5: Anti-cancer effect of DB-ZW1 variant strains

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

The mice were divided into 6 groups, each consisting of 5 mice: PBS group , DB-ZW1 group, DB-ZW1 (*ΔmsbB*) group, DB-ZW1 *(Δlpp)* group, DB-ZW1 (Δ*fliC*) group, and SL7207 group, and the latter 5 groups of tumor-bearing mice were inoculated with 125 µL of 1×10⁷ CFU of bacteria by tail vein injection. The distribution of the above strains in normal tissues and tumors of the tumor-bearing mice, the changes in tumor volume, and the weight change and survival rate of mice were detected.

5.1 Bacterial distribution in tumor-bearing mice: 14 days after injection of the strains DB-ZW1 , DB-ZW1 *(ΔmsbB),* DB-ZW1 (*Δlpp*), DB-ZW1 (Δ*fliC*), all bacteria in normal tissues were essentially completely cleared, whereas the number of bacteria in tumors reached 1×10⁸ CFU/g (FIG.5).

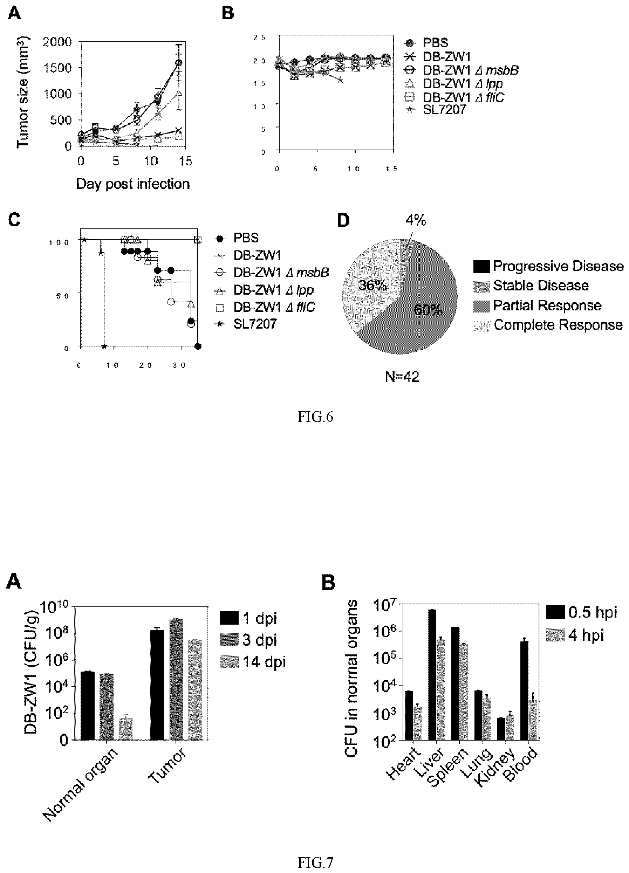

5.2 Changes in tumor volume: compared with the control group treated with PBS injection, the DB-ZW1 strain significantly reduced the tumor volume and significantly increased the survival rate of mice, but the therapeutic effect of *DB-ZW1-ΔmsbB* and *DB-ZW1-Δlpp* was significantly weaker than that of DB-ZW1, whereas the therapeutic effect of flagellin-deficient DB-ZW1-Δ*fliC* was comparable to that of DB-ZW1 (FIG.6A), and the SL7207 group showed a significant reduction in tumor volume before death.

5.3 Changes in body weight of mice: the body weight changes in DB-ZW1 group and DB-ZW1-ΔfliC group mice showed a similar trend of transient decrease, followed by stabilization and rebound. The *DB-ZW1-ΔmsbB* and *DB-ZW1-Δlpp* groups lost less weight than the DB-ZW1 and DB-ZW1-Δ*fliC* groups (FIG.6B), and the SL7207 group lost weight significantly and continuously.

5.4 Mouse survival: The SL7207 group died successively within 7 days after injection. Mice in the PBS, *DB-ZW1-ΔmsbB,* and *DB-ZW1-Δlpp* groups died successively 12 days after injection (Note: animals in the three groups were put to death for ethical reasons due to excessive tumor size). No mice died in the DB-ZW1 group and DB-ZW1-Δ*fliC* group during the experimental cycle (FIG.6C).

5.5 Statistically, DB-ZW1 achieved a partial response rate of 60% (n=42) and a complete response rate of 36% for the treatment of MB49 bladder cancer in mice, indicating that DB-ZW1 had a good therapeutic effect on MB49 (FIG.6D).

The above results showed that DB-ZW1 and DB-ZW1-Δ*fliC* had the efficacy of treating bladder cancer in mice and improving their survival rate, suggesting that lipopolysaccharide (LPS) in DB-ZW1 plays an important role in the treatment of tumors. In the whole observation cycle (35 days), only the DB-ZW1 and *DB-ZW1-ΔfliC* groups achieved 100% survival rate of mice after treatment, indicating that the strains DB-ZW1 and DB-ZW1-Δ*fliC* had a good safety.

### Example 6: Anti-tumor effects on multiple tumor models by DB-ZW1

### 6.1 Construction of tumor model

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells/ B16 melanoma cells (ATCC: CRL-6475) to establish a subcutaneous tumor model of mouse bladder cancer/melanoma. Experiments were carried out 14-18 days after inoculation when the tumor volume was about 100 mm³. *In situ* colon cancer model was induced by DSS/AOM: after a single intraperitoneal injection of 10 mg/kg AOM, water containing 2.5% DSS was fed for 7 days after 1 week, and then normal drinking water for 14 days, which was considered as one cycle of DSS treatment. The DSS treatment was repeated for another 3 cycles to obtain a mouse model with colorectal cancer.

### 6.2 Changes in the distribution of DB-ZW1 in tumors and different organs

125 µL of 1×10⁷ CFU of DB-ZW1 was injected via tail vein into MB49 bladder tumor-bearing mice, with 3 mice in each group. Approximately 90% of DB-ZW1 in mice was observed to reside in tumors 1 day after injection (1 dpi). The density of DB-ZW1 in tumors was further elevated 100-fold (to 10⁸ CFU/g) within the following 2 days and maintained at a high level for the following two weeks. In contrast, the density of DB-ZW1 in normal organs steadily decreased to less than 10² CFU/g within two weeks (FIG.7A).

To further determine whether DB-ZW1 preferentially proliferated in tumors, the number of bacteria distributed in different organs was determined 24 hours after injection of 125 µL of 1×10⁷ CFU of DB-ZW1 bacteria into MB49 bladder tumor-bearing mice via tail vein, with 3 mice in each group (FIG.7B). The results showed that DB-ZW1 spread rapidly in mice within 30 minutes after injection, and most of DB-ZW1 cells (about 99.9%) were distributed in the liver, spleen and blood. And the number of DB-ZW1 cells was significantly reduced within 4 hours after injection, and the total number of DB-ZW1 cells was reduced by about 90%. Subsequently, DB-ZW1 in tumors proliferated exponentially at a growth rate of about 0.7 h⁻¹, reaching saturation within 3 days. In contrast, in normal organs, the number of DB-ZW1 cells were all steadily reduced. This specific tumor growth profile of DB-ZW1 was key to ensuring the validity of the following findings.

### 6.3 Anti-tumor effects of DB-ZW1 on bladder cancer, melanoma and colon

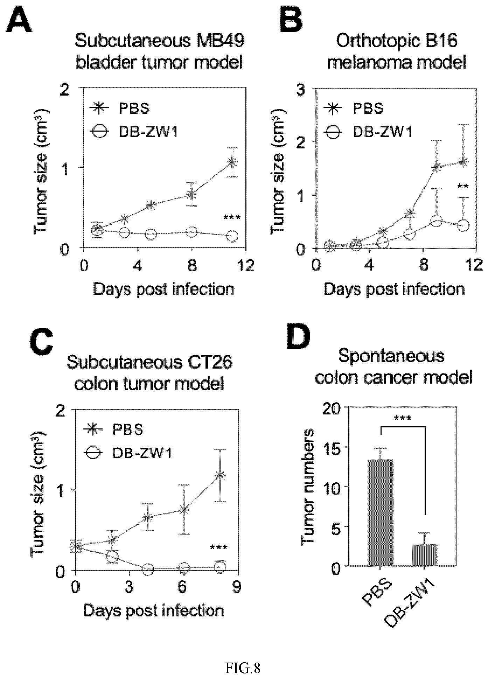

DB-ZW1 was used to treat subcutaneous bladder cancer, *in situ* melanoma and subcutaneous and *in situ* colon cancer in mice. 125 µL of 1×10⁷ CFU of DB-ZW1 was injected into tumor-bearing mice via tail vein, with 5 mice in each group. As shown in FIGs. 8A-C, DB-ZW1 significantly reduced the tumor size. In addition, treatment of mice with *in situ* colon cancer using DB-ZW1 also significantly reduced the number of tumors in the colon of mice (FIG.8D).

### Example 7: Rapid DB-ZW1 proliferation in tumors correlates with its anaerobic circuit

The flagellum-free DB-ZW1-Δ*fliC* variant constructed in Example 2 was used to verify whether tumor-targeting colonization of bacteria is related to their motility, using DB-ZW1 as a control.

### 7.1 Construction of tumor model

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

### 7.2 Tumor-targeting colonization of DB-ZW1 and variant DB-ZW1-ΔfliC

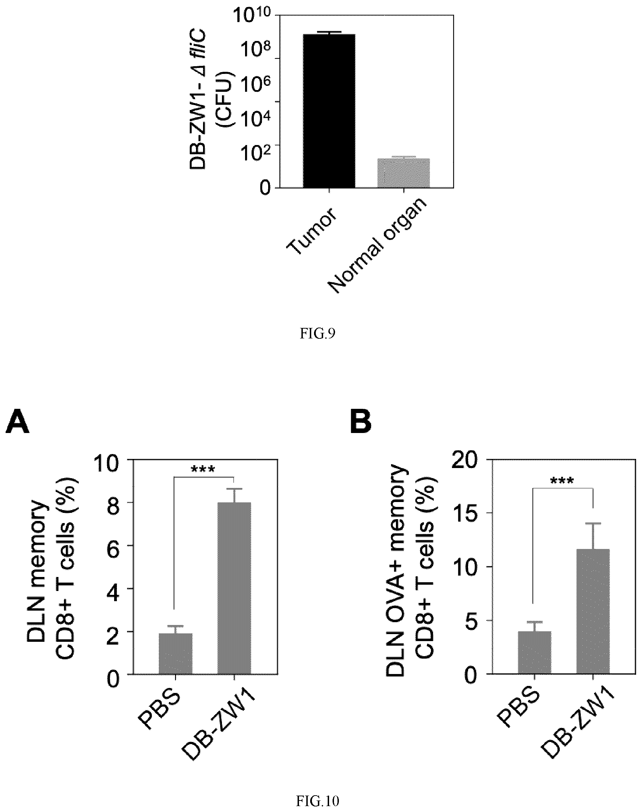

10⁷ CFU of DB-ZW1 and variant DB-ZW1-Δ*fliC* were injected into MB49 tumor-bearing mice via tail vein, respectively, with 3 mice in each group. 14 days after injection, the individual tissue organs (heart, liver, spleen, kidneys, blood, tumors) were taken out, weighed, ground, diluted with PBS and applied to LB plates. After being placed in a 37°C incubator for 16-18 hours, the number of clones on each plate was counted. It was demonstrated that DB-ZW1-Δ*fliC*, which has very weak motility, was also able to colonize in tumors (FIG.9). This result showed that the tumor-targeting effect of DB-ZW1 was not due to bacterial motility, but is closely related to bacterial colonization and rapid proliferation in tumors.

### Example 8: The capacity of DB-ZW1 to specifically activate memory CD8⁺T cells

### 8.1 Construction of tumor model

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

### 8.2 Activation of memory CD8⁺T cells by DB-ZW1

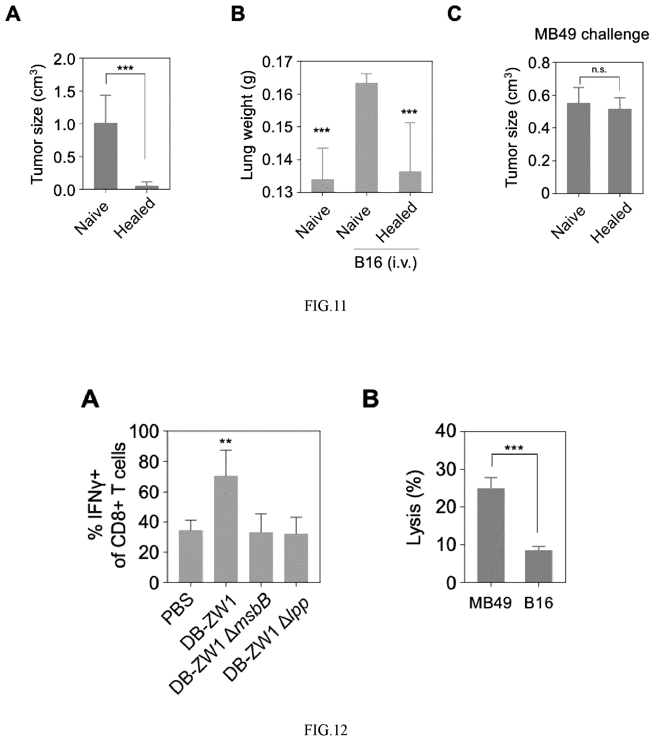

Mice were dissected and tumor draining lymph nodes were taken out. After grinding, a single cell suspension was prepared by filtration through a 40 µm mesh filter. The amount of memory CD8⁺T cells in tumor-draining lymph nodes was then detected by antibody staining and flow analysis. It was found that the level of memory CD8⁺T cells was significantly increased in the draining lymph nodes of mice treated with DB-ZW1, as compared with mice in PBS control group (FIG.10A). Increased memory CD8⁺T cells in the draining lymph nodes of mice inoculated with 1×10⁶ B16-OVA melanoma cells were found to be tumor antigen specific (FIG. 10B). After another subcutaneous inoculation of 1×10⁶ B16-OVA melanoma cells, no tumor recurrence was observed in melanoma mice cured by DB-ZW1 (FIG.11A). Melanoma mice that were previously cured by DB-ZW1 and subsequently subcutaneously inoculated with 1×10⁶ MB49 bladder cancer cells developed tumors just like the mice that received the initial subcutaneous injection of tumor cells (FIG.11C). The above results showed that the anti-tumor effect of DB-ZW1 was characterized by tumor memory.

### 8.3 Inhibition of metastatic tumors by DB-ZW1

125 µL of a cell suspension containing 1×10⁶ B16 cells was used to inoculate DB-ZW1-curedmelanoma mice through tail vein injection, and the formation of tumor metastases in the lung of mice was detected after 18 days. It was found that the lung of mice cured by DB-ZW1 has a significantly reduced body weight as compared to mice with first-induced melanoma (FIG.11B).

### Example 9: Activation of the anti-tumor effect of memory CD8⁺T cells by DB-ZW1

### 9.1 Construction of tumor model

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

### 9.2 Activation of IFNγ⁺CD8⁺T cells by DB-ZW1

Mice subcutaneously inoculated with MB49 bladder cancer cells were treated with DB-ZW1 and variants DB-ZW1-*ΔmsbB* and DB-ZW1-*Δlpp*, respectively, with 3 mice in each group. The content of IFNγ⁺CD8⁺T cells in the tumor microenvironment of each group was detected, and the number of IFNγ⁺CD8⁺T cells infiltrated in the tumor microenvironment of mice in the DB-ZW1 treatment group was significantly higher than that in the PBS control group and the variant DB-ZW1*-ΔmsbB* and DB-ZW1-*Δlpp* treatment groups (FIG.12A).

*In vitro* co-culture experiments of CD8⁺T cells with bladder cancer cell line MB49, and melanoma cell line B16 were performed. CD8⁺T cells were isolated from tumors of tumor-bearing mice treated with DB-ZW1 and then co-cultured with MB49 or B16 tumor cells at a ratio of 5:1 for 5 hours in a CO₂ incubator at 37°C. The killing effect on target cells was determined by measuring lactate dehydrogenase release (LDH) in the supernatant collected from each co-culture well. The results showed that isolated CD8⁺T cells were significantly more effective in killing MB49 cells than killing B16 cells (FIG.12B). It was seen that intratumoral CD8⁺T cells after injection of DB-ZW1 had a stronger killing effect on specifically treated tumor cells. This result suggested that DB-ZW1 exerted anti-tumor effects by specific activation of intratumoral IFNγ⁺CD8⁺T cells.

### Example 10: Activation of tissue-resident memory T cells (TRM cells) in tumors by DB-ZW1

### 10.1 Construction of tumor model

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

### 10.2 Activation of TRM cells by DB-ZW1

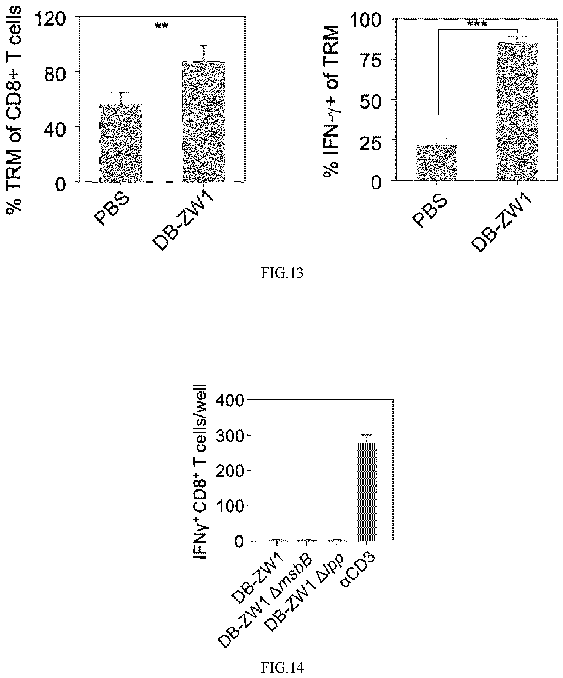

The content of CD8⁺ T cells of CD62L-CD69⁺, CD62L-CD69⁺CD103⁺ or CD62L-CD69⁺CD103⁻, respectively, in the tumor microenvironment before and after treatment with DB-ZW1 was detected, wherein CD62L-CD69⁺CD8⁺T cells were TRM cells. It was found that the proportion of TRM cells in CD8⁺T cells as well as the proportion of IFNγ⁺ cells in the TRM cells, in the tumor-draining lymph nodes and tumors of mice treated with DB-ZW1 were significantly higher than the control group treated with PBS (FIG.13).

The result suggested that DB-ZW1 could exert the anti-tumor effect by specifically activating TRM cells in tumors.

### Example 11: Activation of CD8⁺T cells through stimulating IL-10 production in macrophages by DB-ZW1 11.1 Construction of tumor model

C57BL/6 mice (purchased from Beijing Vital River Laboratory Animal Technology Co., Ltd., weighing about 18 g, kept in a SPF environment) were subcutaneously inoculated with 1×10⁶ MB49 mouse bladder cancer cells to establish a subcutaneous tumor model of mouse bladder cancer. Experiments were carried out 14-18 days after inoculation when the tumor volume was about 100 mm³.

### 11.2 Activation of the anti-tumor effect of CD8⁺T cells by IL-10

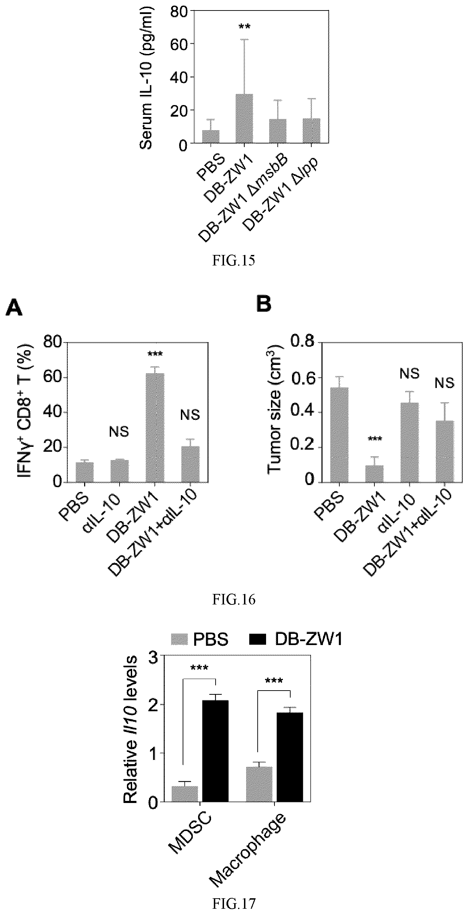

*In vitro* stimulation of intratumoral isolated CD8⁺T cells using anti-CD3 antibody and DB-ZW1, DB-ZW1-*ΔmsbB,* DB-ZW1-*Δlpp* respectively revealed that all of DB-ZW1, DB-ZW1-*ΔmsbB,* and DB-ZW1-*Δlpp* were unable to efficiently stimulate activation of CD8⁺T cells (Fig.14). Subcutaneous bladder cancer in mice was further treated with DB-ZW1, DB-ZW1-*ΔmsbB,* and DB-ZW1-*Δlpp*, respectively, and the levels of various inflammatory factors in serum were detected. It was found that the content of IL-10 in the DB-ZW1 treatment group was significantly higher than that in the other groups (FIG.15), suggesting that IL-10 may play an important role in the treatment of tumors by DB-ZW1.

To demonstrate the role of cytokine IL-10 in the treatment of tumors by DB-ZW1, anti-IL-10 (BioXCell: #BE0049), a neutralizing antibody of IL-10, and DB-ZW1 were used to treat MB49-induced bladder cancer mice. After 7 days of treatment, the anti-IL-10 antibody significantly inhibited the increase in the percentage of DB-ZW1-induced IFNγ⁺CD8⁺T cells at tumor sites compared with the PBS control group (FIG.16A). Meanwhile, measurement of tumor volume showed that DB-ZW1 significantly reduced tumor volume compared with the PBS control group, while the anti-IL-10 antibody significantly inhibited tumor volume reduction induced by DB-ZW1 (FIG.16B).

### 11.3 IL-10 production in primary macrophages induced by DB-ZW1

Primary macrophages were obtained by flow sorting of mouse bone marrow cells, and the primary macrophages cultured *in vitro* were stimulated by DB-ZW1. After stimulation, all RNA was extracted by TRIzol reagent. After obtaining cDNA by reverse transcription, the transcript level of IL-10 was detected by qPCR, and the relative expression of the gene was calculated using the standard curve method and normalized to the expression level of Actb. It was found that DB-ZW1 significantly induced IL-10 expression in primary macrophages (FIG.17).

These results indicated that DB-ZW1 stimulated macrophages to produce IL-10, and then activated CD8+T cells to exert anti-tumor effects.

### Example 12: Remodeling of the intratumoral immune environment by DB-ZW1

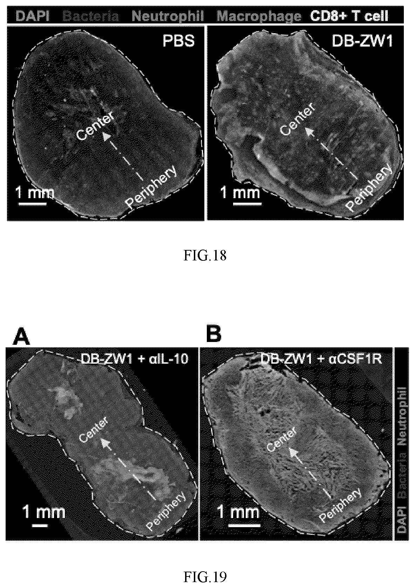

Tumors from MB49 bladder cancer mice treated with DB-ZW1 injection were sampled and subjected to frozen section and immunofluorescence staining. After the tumors were taken out, they were immersed in 4% paraformaldehyde tissue fixing solution for fixing for 5 hours and then transferred to 30% sucrose for dehydration for 12 hours. Then the tumors were embedded in OCT embedding agent and frozen in a refrigerator at -20°C. The frozen samples were sectioned continuously at a thickness of 10 mm using a freezing microtome and stored at -80°C. After fixation in acetone for 10 minutes, the sections were air-dried, allowed to return to room temperature before staining with the corresponding antibodies, and finally covered with a drop of DAPI-containing anti-fluorescence mounting medium before covering a coverslip and sealing with nail polish. The sections were observed and photographed under a fluorescence microscope. It was found that most of DB-ZW1 were localized in the center of the tumor sections and that neutrophils aggregated and formed a ring around DB-ZW1 (FIG.18). It was also found that T cells and macrophages in tumors were also transformed from an intratumoral disordered distribution to an orderly regular outward-to-inward ordering after treatment with DB-ZW1 (FIG.18). It was further found that the formation of neutrophil ring structure was closely related to the level of IL-10 in TME, the obvious ring structure of neutrophils completely disappeared when using the corresponding anti-IL-10 antibody (FIGs. 19A, 19B), and the spatial distribution of neutrophils and residual DB-ZW1 cells was uniform and overlapped throughout the tumor sections, with a significant decrease in DB-ZW1 density.

An anti-CSF1R antibody (50 mg/kg, BioXCell, BE0213) was injected via tail vein 3 days before bacterial treatment and every 2 to 3 days until the endpoint of the experiment to deplete tumor-infiltrating macrophages, and it was found that the ring structure of neutrophils disappeared (FIG.19C), and the density of DB-ZW1 in tumors was also decreased. This suggested that macrophages were able to protect bacteria from neutrophil phagocytosis. These results suggest that DB-ZW1 is able to remodel the intratumoral immune environment, wherein bacteria present in the center of the tumor attract neutrophils while the bacteria defend against neutrophils by stimulating IL-10 prodcution in macrophages. Once IL-10 is removed, neutrophils will invade the tumor center to destroy the bacteria.

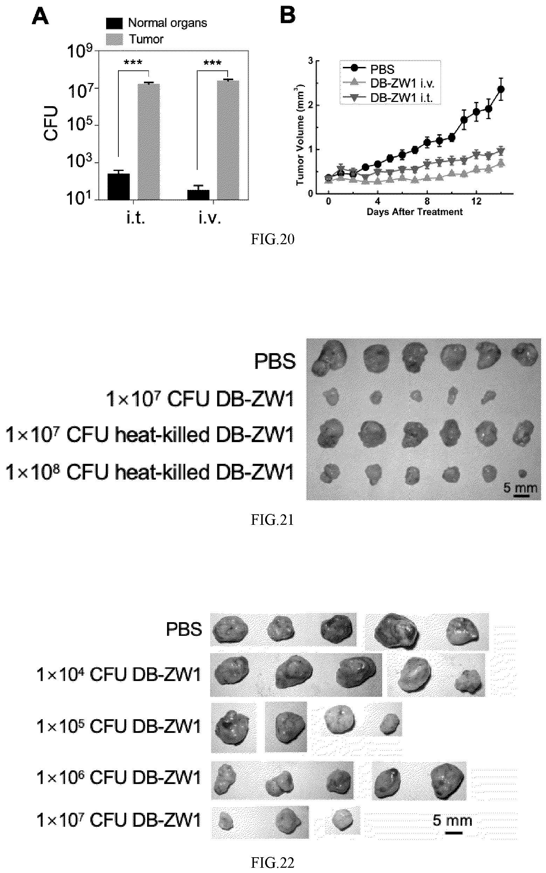

Example 13: Anti-tumor effect of DB-ZW1 with different administration manner and administration doses Currently, common routes of administration in clinical practice include subcutaneous injection, intravenous injection, oral administration, and topical application. The route of drug administration is closely related to the therapeutic effect for various clinical conditions, and the same drug may have significantly different effects depending on the route of administration. Given the specificity of DB-ZW1 in targeting tumors, the effects of both intratumoral and intravenous injections on the therapeutic efficacy were explored. First, the route of administration and dose of DB-ZW1 were optimized. As shown in FIG.20A, on day 14 after intratumoral injection (i.t.) or intravenous injection (i.v.) administration of 1×10⁷ CFU of DB-ZW1, the distribution of DB-ZW1 in tumors was all significantly higher than that in other normal tissues. The anti-tumor effect of the intravenous injection administration group was superior to that of the intratumoral injection administration group (FIG.20B).

Tumor treatment with inactivated DB-ZW1 was also attempted (FIG.21), and the results of tumor volume on the 14th day after injection showed that inactivated DB-ZW1 exhibited a certain anti-tumor effect, which improved as the injection dosage increases.

The effects of different administration doses of DB-ZW1 on the therapeutic efficacy in subcutaneous MB49 bladder tumor-bearing mice were further explored (FIG.22). On the 14th day after intravenous injection of DB-ZW1, the results showed that there was no therapeutic effect when the quantity of DB-ZW1 was less than 1×10⁴ CFU, while a therapeutic effect began to manifest when the quantity of DB-ZW1 was greater than or equal to 1×10⁵ CFU, with a more pronounced effect observed upon intravenous injection of 1×10⁶ CFU of DB-ZW1.

The above results indicate that there is a very close relationship between the administration route and dose of DB-ZW1 and the therapeutic effect. This result provides technical guidance for the future practical application of DB-ZW1 as a drug.

### Sequence Listing

SEQ ID NO: 1: PsseA-hlyA-loxp-KnaR-loxp 
TTCACAGAAAAGTGTTGCCCCCTTCCATGGCGGAAGGGGGACAAAGGTGAATTTGTCCTACTCAGGAGAGCGTTC
ACCGACAAACAACAGATAAAACGAAAGGCCCAGTCTTTCGACTGAGCCTTTCGTTTTATTTGATGCACTAGTGCC
GATCATATTCAATAACCCTTAATATAACTTCGTATAATGTATGCTATACGAAGTTATTAGGTCTGAAGAGGAGTT
TACGTCCCATATGGAGTCCCGCTCAGAAGAACTCGTCAAGAAGGCGATAGAAGGCGATGCGCTGCGAATCGGGAG
CGGCGATACCGTAAAGCACGAGGAAGCGGTCAGCCCATTCGCCGCCAAGCTCTTCAGCAATATCACGGGTAGCCA
ACGCTATGTCCTGATAGCGGTCCGCCACACCCAGCCGGCCACAGTCGATGAATCCAGAAAAGCGGCCATTTTCCA
CCATGATATTCGGCAAGCAGGCATCGCCATGGGTCACGACGAGATCCTCGCCGTCGGGCATGCGCGCCTTGAGCC
TGGCGAACAGTTCGGCCGGCGCGAGCCCCTGATGCTCTTCGTCCAGATCATCCTGATCGACAAGACCGGCTTCCA
TCCGAGTACGTGCTCGCTCGATGCGATGTTTCGCTTGGTGGTCGAATGGGCAGGTAGCCGGATCAAGCGTATGCA
GCCGCCGCATTGCATCAGCCATGATGGATACTTTCTCGGCAGGAGCAAGGTGAGATGACAGGAGATCCTGCCCCG
GCACTTCGCCCAATAGCAGCCAGTCCCTTCCCGCTTCAGTGACAACGTCGAGCACAGCTGCGCAAGGAACGCCCG
TCGTGGCCAGCCACGATAGCCGCGCTGCCTCGTCCTGCAGTTCATTCAGGGCACCGGACAGGTCGGTCTTGACAA
AAAGAACCGGGCGCCCCTGCGCTGACAGCCGGAACACGGCGGCATCAGAGCAGCCGATTGTCTGTTGTGCCCAGT
CATAGCCGAATAGCCTCTCCACCCAAGCGGCCGGAGAACCTGCGTGCAATCCATCTTGTTCAATCATGCGAAACG
ATCCTCATCCTGTCTCTTGATCAGATCCTGATCCCCTGCGCCATCAGATCCTTGGCGGCAAGAAAGCCATCCAGT
TTACTTTGCAGGGCTTCCCAACCTTACCAGAGGGCGCCCCAGCTGGCAATTCCGACGTCATTGCGTTGCTAGCCA
AGCTTAGGATCCGGAACCCTTAATATAACTTCGTATAATGTATGCTATACGAAGTTATTAGGTCCCTCGAAGAGG
TTCACTAGCTATCTAGAGCTTGGACTCCTGTTGATAGATCCAGTAATGACCTCAGAACTCCATCTGGATTTGTTC
AGAACGCTCGGTTGCCGCCGGGCGTTTTTTATTGGTGAGAATCCAAGCCCGGGAAACGGGATCCTGATCTTGATC
AGGCTGTGGGTCAGGCCACTCTTGGCATCGACACCAATGTGGGCCTTCATGCCAAAGTGCCACTGATTGCCTTTC
TTGGTCTGATGCATCTCCGGATCGCGTTGCTGCTCTTTGTTCTTGGTCGAGCTGGGTGCCTCAATGATGGTGGCA
TCGACCAAGGTGCCTTGAGTCATCATGACGCCTGCTTCGGCCAGCCAGCGATTGATGGTCTTGAACAATTGGCGG
GCCAGTTGATGCTGCTCCAGCAGGTGGCGGAAATTCATGATGGTGGTGCGGTCCGGCAAGGCGCTATCCAGGGAT
AACCGGGCAAACAGACGCATGGAGGCGATTTCGTACAGAGCATCTTCCATCGCGCCATCGCTCAGGTTGTACCAA
TGCTGCATGCAGTGAATGCGTAGCATGGTTTCCAGCGGATAAGGTCGCCGGCCATTACCAGCCTTGGGGTAAAAC
GGCTCGATGACTTCCACCATGTTTTGCCATGGCAGAATCTGCTCCATGCGGGACAAGAAAATCTCTTTTCTGGTC
TGACGGCGCTTACTGCTGAATTCACTGTCGGCGAAGGTAAGTTGATGACTCATGATGAACCCTGTTCTATGGCTC
CAGATGACAAACATGATCTCATATCAGGGACTTGTTCGCACCTTCCTTAGCACGTTAATTATCTATCGTGTATAT
GGAGGGGATCTAGAATGAAAAAAATAATGCTAGTTTTTATTACACTTATATTAGTTAGTCTACCAATTGCGCAAC
AAACTGAAGCAAAGGATGCATCTGCATTCAATAAAGAAAATTCAATTTCATCCATGGCACCACCAGCATCTCCGC
CTGCAAGTCCTAAGACGCCAATCGAAAAGAAACACGCGGATGAAATCGATAAGTATATACAAGGATTGGACTACA
ATAAAAACAATGTATTAGTATACCACGGAGATGCAGTGACAAATGTGCCGCCAAGAAAAGGTTACAAAGATGGAA
ATGAATATATTGTTGTGGAGAAAAAGAAGAAATCCATCAATCAAAATAATGCAGACATTCAAGTTGTGAATGCAA
TTTCGAGCCTAACCTATCCAGGTGCTCTCGTAAAAGCGAATTCGGAATTAGTAGAAAATCAACCAGATGTTCTCC
CTGTAAAACGTGATTCATTAACACTCAGCATTGATTTGCCAGGTATGACTAATCAAGACAATAAAATCGTTGTAA
AAAATGCCACTAAATCAAACGTTAACAACGCAGTAAATACATTAGTGGAAAGATGGAATGAAAAATATGCTCAAG
CTTATCCAAATGTAAGTGCAAAAATTGATTATGATGACGAAATGGCTTACAGTGAATCACAATTAATTGCGAAAT
TTGGTACAGCATTTAAAGCTGTAAATAATAGCTTGAATGTAAACTTCGGCGCAATCAGTGAAGGAAAAATGCAAG
AAGAAGTCATTAGTTTTAAACAAATTTACTATAACGTGAATGTTAATGAACCTACAAGACCTTCCAGATTTTTCG
GCAAAGCTGTTACTAAAGAGCAGTTGCAAGCGCTTGGAGTGAATGCAGAAAATCCTCCTGCATATATCTCAAGTG
TGGCGTATGGCCGTCAAGTTTATTTGAAATTATCAACTAATTCCCATAGTACTAAAGTAAAAGCTGCTTTTGATG
CTGCCGTAAGCGGAAAATCTGTCTCAGGTGATGTAGAACTAACAAATATCATCAAAAATTCTTCCTTCAAAGCCG
TAATTTACGGAGGTTCCGCAAAAGATGAAGTTCAAATCATCGACGGCAACCTCGGAGACTTACGAGATATTTTGA
AAAAAGGCGCTACTTTTAATCGAGAAACACCAGGAGTTCCCATTGCTTATACAACAAACTTCCTAAAAGACAATG
AATTAGCTGTTATTAAAAACAACTCAGAATATATTGAAACAACTTCAAAAGCTTATACAGATGGAAAAATTAACA
TCGATCACTCTGGAGGATACGTTGCTCAATTCAACATTTCTTGGGATGAAGTAAATTATGATCCTGAAGGTAACG
AAATTGTTCAACATAAAAACTGGAGCGAAAACAATAAAAGTAAGCTAGCTCATTTCACATCGTCCATCTATTTGC
CAGGTAACGCGAGAAATATTAATGTTTACGCTAAAGAATGCACTGGTTTAGCTTGGGAATGGTGGAGAACGGTAA
TTGATGACCGGAACTTACCACTTGTGAAAAATAGAAATATCTCCATCTGGGGCACCACGCTTTATCCGAAATATA
GTAATAAAGTAGATAATCCAATCGAATAAGTCGACCTCGAGGACTAGTGAACCTCTTCGAGGGACCTAATAACTT
 
CGTATAGCATACATTATACGAAGTTATATTAAGGGTTATTGAATATGATCGGCTCGAGATTACTTCGCATTGCAA
TCAGCTACGGACTCAAGCTTACGTCAGGTCATACGATGAAGGCGATAAGTCGCGTAAGTTGCTAGCCCACGCGGC
TCACCTTCACGGGTGGGCCTTTCTTCGGTGTTTCAATCTCGATTAACAGGTAACGCAAAATTGCTGTGTACGTCA
GA
SEQ ID NO:2: Pssbp1-dapE-Cm 
TTGGTTTCAGTGAATCCCGTTATCAGCAGTTTTTTGATGAGGTGTAGTCTATTTGTCCTACTCAGGAGAGCGTTC
ACCGACAAACAACAGATAAAACGAAAGGCCCAGTCTTTCGACTGAGCCTTTCGTTTTATTTGATGCACTAGTGCC
GATCATATTCAATAACCCTTAATATAACTTCGTATAATGTATGCTATACGAAGTTATTAGGTCTGAAGAGGAGTT
TACGTCCCATATGGAGTCCCGCTTACGCCCCGCCCTGCCACTCATCGCAGTACTGTTGTAATTCATTAAGCATTC
TGCCGACATGGAAGCCATCACAGACGGCATGATGAACCTGAATCGCCAGCGGCATCAGCACCTTGTCGCCTTGCG
TATAATATTTGCCCATGGTGAAAACGGGGGCGAAGAAGTTGTCCATATTGGCCACGTTTAAATCAAAACTGGTGA
AACTCACCCAGGGATTGGCTGAGACGAAAAACATATTCTCAATAAACCCTTTAGGGAAATAGGCCAGGTTTTCAC
CGTAACACGCCACATCTTGCGAATATATGTGTAGAAACTGCCGGAAATCGTCGTGGTATTCACTCCAGAGCGATG
AAAACGTTTCAGTTTGCTCATGGAAAACGGTGTAACAAGGGTGAACACTATCCCATATCACCAGCTCACCGTCTT
TCATTGCCATACGAAATTCCGGATGAGCATTCATCAGGCGGGCAAGAATGTGAATAAAGGCCGGATAAAACTTGT
GCTTATTTTTCTTTACGGTCTTTAAAAAGGCCGTAATATCCAGCTGAACGGTCTGGTTATAGGTACATTGAGCAA
CTGACTGAAATGCCTCAAAATGTTCTTTACGATGCCATTGGGATATATCAACGGTGGTATATCCAGTGATTTTTT
TCTCCATGCGAAACGATCCTCATCCTGTCTCTTGATCAGATCCTGATCCCCTGCGCCATCAGATCCTTGGCGGCA
AGAAAGCCATCCAGTTTACTTTGCAGGGCTTCCCAACCTTACCAGAGGGCGCCCCAGCTGGCAATTCCGACGTCA
TTGCGTTGCTAGCCAAGCTTAGGATCCGGAACCCTTAATATAACTTCGTATAATGTATGCTATACGAAGTTATTA
GGTCCCTCGAAGAGGTTCACTAGCTATCTAGAGCTTGGACTCCTGTTGATAGATCCAGTAATGACCTCAGAACTC
CATCTGGATTTGTTCAGAACGCTCGGTTGCCGCCGGGCGTTTTTTATTGGTGAGAATCCAAGCCCGGGAAACGGG
ATCCTGATCTTGATCAACCGAGGTCACAACATAGTAAAAGCGCTATTGGTAATGGTACAATCGCGCGTTTACACT
TATTCAGAACGATTTTTTTCAGGAGGGCCTACTTGTCACACAGGACGGTAATGTCGTGCCCGGTTATTGAGCTGA
CACAGCAACTTATTCGTCGCCCCTCCCTGAGTCCGGATGACGCAGGCTGTCAGGCATTAATGATTGAACGCCTGC
GTAAAATCGGTTTTACCATTGAGCATATGGATTTTGGCGACACGCAAAATTTTTGGGCATGGCGTGGGCGTGGCG
AAACGCTGGCGTTCGCCGGGCATACTGACGTCGTACCGGCTGGCGATGTCGATCGCTGGATCAATCCGCCGTTTG
AGCCTACGATTCGCGACGGAATGTTGTTCGGTCGCGGCGCGGCGGATATGAAAGGTTCACTGGCGGCCATGGTGG
TGGCTGCTGAACGTTTTGTTGCGCAGCATCCTCATCACCGGGGCCGCCTCGCATTTTTGATTACCTCTGATGAGG
AAGCCAGCGCAAAAAATGGCACCGTAAAGGTGGTTGAAGCGCTGATGGCCCGTAACGAGCGGCTGGATTACTGTC
TGGTCGGCGAACCTTCCAGTACTGAAATTGTGGGCGATGTCGTCAAAAACGGGCGTCGCGGTTCTCTCACCTGTA
ACCTCACTATCCATGGCGTTCAGGGGCACGTCGCCTATCCGCATCTGGCGGATAACCCTGTTCATCGCGCCGCGC
CTTTTCTTAATGAACTGGTCGCTATCGAGTGGGATCGGGGCAATGACTTTTTCCCGGCCACCAGTATGCAGGTAG
CCAATATCCAGGCAGGCACCGGCAGCAATAATGTCATTCCCGGCGAACTGTTCGTGCAGTTTAACTTCCGTTTCA
GCACGGAACTGACCGATGAGATGATCAAAGAGCGGGTACATGCGCTGCTTGAAAAGCATCAGCTACGCTATACCG
TAGACTGGTGGCTTTCCGGGCAGCCGTTCCTGACCGCACGCGGCAAGCTGGTGGACGCGGTGGTAAACGCCATTG
AGCACTATAATGAAATTAAACCGCAATTACTGACGACGGGCGGTACGTCCGACGGACGTTTTATCGCCCGTATGG
GAGCGCAGGTGGTAGAACTGGGGCCGGTGAACGCCACCATTCATAAAATTAATGAATGTGTGAATGCCGCCGATC
TCCAGCTACTGGCCCGTATGTATCAACGCATTATGGAACAGCTCGTCGCCCATCACCATCACCATCACTAAGATT
ACTTCGCATTGCAATCAGCTACGGACTCAAGCTTACGTCAGGTCATACGATGAAGGCGATAAGTCGCGTAAGTTG
CTAGCCCACGCGGCTCACCTTCACGGGTGGGCCTTTCTTCGTGAGCGTTCCGGTAAGAGGAAATAAGCATGGACT
GGCTGGCAAAATATTG
SEQ ID NO:3: loxp-knar-loxp 
TTATTCGATTGGATTATCTACTTTATTACTATATTTCGGATAAAGCGTGGCTAGTGAACCTCTTCGAGGGACCTA
ATAACTTCGTATAGCATACATTATACGAAGTTATATTAAGGGTTCCGGATCCTAAGCTTGGCTAGCAACGCAATG
ACGTCGGAATTGCCAGCTGGGGCGCCCTCTGGTAAGGTTGGGAAGCCCTGCAAAGTAAACTGGATGGCTTTCTTG
CCGCCAAGGATCTGATGGCGCAGGGGATCAGGATCTGATCAAGAGACAGGATGAGGATCGTTTCGCATGATTGAA
CAAGATGGATTGCACGCAGGTTCTCCGGCCGCTTGGGTGGAGAGGCTATTCGGCTATGACTGGGCACAACAGACA
 
ATCGGCTGCTCTGATGCCGCCGTGTTCCGGCTGTCAGCGCAGGGGCGCCCGGTTCTTTTTGTCAAGACCGACCTG
TCCGGTGCCCTGAATGAACTGCAGGACGAGGCAGCGCGGCTATCGTGGCTGGCCACGACGGGCGTTCCTTGCGCA
GCTGTGCTCGACGTTGTCACTGAAGCGGGAAGGGACTGGCTGCTATTGGGCGAAGTGCCGGGGCAGGATCTCCTG
TCATCTCACCTTGCTCCTGCCGAGAAAGTATCCATCATGGCTGATGCAATGCGGCGGCTGCATACGCTTGATCCG
GCTACCTGCCCATTCGACCACCAAGCGAAACATCGCATCGAGCGAGCACGTACTCGGATGGAAGCCGGTCTTGTC
GATCAGGATGATCTGGACGAAGAGCATCAGGGGCTCGCGCCGGCCGAACTGTTCGCCAGGCTCAAGGCGCGCATG
CCCGACGGCGAGGATCTCGTCGTGACCCATGGCGATGCCTGCTTGCCGAATATCATGGTGGAAAATGGCCGCTTT
TCTGGATTCATCGACTGTGGCCGGCTGGGTGTGGCGGACCGCTATCAGGACATAGCGTTGGCTACCCGTGATATT
GCTGAAGAGCTTGGCGGCGAATGGGCTGACCGCTTCCTCGTGCTTTACGGTATCGCCGCTCCCGATTCGCAGCGC
ATCGCCTTCTATCGCCTTCTTGACGAGTTCTTCTGAGCGGGACTCCATATGGGACGTAAACTCCTCTTCAGACCT
AATAACTTCGTATAGCATACATTATACGAAGTTATATTAAGGGTTATTGAATATGATCGGCGGTAGACTAACTAA
TATAAGTGTAATAAAAACTAGCATTATTTTTTTCAT
SEQ ID NO:4: PdapE-dapE-loxp-KnaR-loxp 
CAATATTTTGCCAGCCAGTCCATGCTTATTTCCTCTTACCGGAACGCTCAATTTGTCCTACTCAGGAGAGCGTTC
ACCGACAAACAACAGATAAAACGAAAGGCCCAGTCTTTCGACTGAGCCTTTCGTTTTATTTGATGCACTAGTGCC
GATCATATTCAATAACCCTTAATATAACTTCGTATAATGTATGCTATACGAAGTTATTAGGTCTGAAGAGGAGTT
TACGTCCCATATGGAGTCCCGCTCAGAAGAACTCGTCAAGAAGGCGATAGAAGGCGATGCGCTGCGAATCGGGAG
CGGCGATACCGTAAAGCACGAGGAAGCGGTCAGCCCATTCGCCGCCAAGCTCTTCAGCAATATCACGGGTAGCCA
ACGCTATGTCCTGATAGCGGTCCGCCACACCCAGCCGGCCACAGTCGATGAATCCAGAAAAGCGGCCATTTTCCA
CCATGATATTCGGCAAGCAGGCATCGCCATGGGTCACGACGAGATCCTCGCCGTCGGGCATGCGCGCCTTGAGCC
TGGCGAACAGTTCGGCCGGCGCGAGCCCCTGATGCTCTTCGTCCAGATCATCCTGATCGACAAGACCGGCTTCCA
TCCGAGTACGTGCTCGCTCGATGCGATGTTTCGCTTGGTGGTCGAATGGGCAGGTAGCCGGATCAAGCGTATGCA
GCCGCCGCATTGCATCAGCCATGATGGATACTTTCTCGGCAGGAGCAAGGTGAGATGACAGGAGATCCTGCCCCG
GCACTTCGCCCAATAGCAGCCAGTCCCTTCCCGCTTCAGTGACAACGTCGAGCACAGCTGCGCAAGGAACGCCCG
TCGTGGCCAGCCACGATAGCCGCGCTGCCTCGTCCTGCAGTTCATTCAGGGCACCGGACAGGTCGGTCTTGACAA
AAAGAACCGGGCGCCCCTGCGCTGACAGCCGGAACACGGCGGCATCAGAGCAGCCGATTGTCTGTTGTGCCCAGT
CATAGCCGAATAGCCTCTCCACCCAAGCGGCCGGAGAACCTGCGTGCAATCCATCTTGTTCAATCATGCGAAACG
ATCCTCATCCTGTCTCTTGATCAGATCCTGATCCCCTGCGCCATCAGATCCTTGGCGGCAAGAAAGCCATCCAGT
TTACTTTGCAGGGCTTCCCAACCTTACCAGAGGGCGCCCCAGCTGGCAATTCCGACGTCATTGCGTTGCTAGCCA
AGCTTAGGATCCGGAACCCTTAATATAACTTCGTATAATGTATGCTATACGAAGTTATTAGGTCCCTCGAAGAGG
TTCACTAGCTATCTAGAGCTTGGACTCCTGTTGATAGATCCAGTAATGACCTCAGAACTCCATCTGGATTTGTTC
AGAACGCTCGGTTGCCGCCGGGCGTTTTTTATTGGTGAGAATCCAAGCCCGGGAAACGGGATCCTGATCTTGATC
AGGAACATGGCATTGATTATCGTTTTCACGATTACCGTGTGGATGGCATAGACCTCCCTCTTCTTAATACCTTTA
TTGCCGAACTCGGCTGGCAGCCTTTACTGAATACGCGCGGCACAACATGGCGCAAACTGGATGAAGGCCACCGCA
GCGGGATTACCGATGCCGACTCCGCCGCTGCTCTCATGGTTGAACAGCCGGCAATCATCAAACGCCCATTGCTCT
GCGCGCCCGGGAAGCCTATGCTGCTTGGTTTCAGTGAATCCCGTTATCAGCAGTTTTTTGATGAGGTGTAGTCTG
GCCTACTTGTCACACAGGACGGTAATGTCGTGCCCGGTTATTGAGCTGACACAGCAACTTATTCGTCGCCCCTCC
CTGAGTCCGGATGACGCAGGCTGTCAGGCATTAATGATTGAACGCCTGCGTAAAATCGGTTTTACCATTGAGCAT
ATGGATTTTGGCGACACGCAAAATTTTTGGGCATGGCGTGGGCGTGGCGAAACGCTGGCGTTCGCCGGGCATACT
GACGTCGTACCGGCTGGCGATGTCGATCGCTGGATCAATCCGCCGTTTGAGCCTACGATTCGCGACGGAATGTTG
TTCGGTCGCGGCGCGGCGGATATGAAAGGTTCACTGGCGGCCATGGTGGTGGCTGCTGAACGTTTTGTTGCGCAG
CATCCTCATCACCGGGGCCGCCTCGCATTTTTGATTACCTCTGATGAGGAAGCCAGCGCAAAAAATGGCACCGTA
AAGGTGGTTGAAGCGCTGATGGCCCGTAACGAGCGGCTGGATTACTGTCTGGTCGGCGAACCTTCCAGTACTGAA
ATTGTGGGCGATGTCGTCAAAAACGGGCGTCGCGGTTCTCTCACCTGTAACCTCACTATCCATGGCGTTCAGGGG
CACGTCGCCTATCCGCATCTGGCGGATAACCCTGTTCATCGCGCCGCGCCTTTTCTTAATGAACTGGTCGCTATC
GAGTGGGATCGGGGCAATGACTTTTTCCCGGCCACCAGTATGCAGGTAGCCAATATCCAGGCAGGCACCGGCAGC
AATAATGTCATTCCCGGCGAACTGTTCGTGCAGTTTAACTTCCGTTTCAGCACGGAACTGACCGATGAGATGATC
AAAGAGCGGGTACATGCGCTGCTTGAAAAGCATCAGCTACGCTATACCGTAGACTGGTGGCTTTCCGGGCAGCCG
TTCCTGACCGCACGCGGCAAGCTGGTGGACGCGGTGGTAAACGCCATTGAGCACTATAATGAAATTAAACCGCAA
 
TTACTGACGACGGGCGGTACGTCCGACGGACGTTTTATCGCCCGTATGGGAGCGCAGGTGGTAGAACTGGGGCCG
GTGAACGCCACCATTCATAAAATTAATGAATGTGTGAATGCCGCCGATCTCCAGCTACTGGCCCGTATGTATCAA
CGCATTATGGAACAGCTCGTCGCCCATCACCATCACCATCACTAAGATTACTTCGCATTGCAATCAGCTACGGAC
TCAAGCTTACGTCAGGTCATACGATGAAGGCGATAAGTCGCGTAAGTTGCTAGCCCACGCGGCTCACCTTCACGG
GTGGGCCTTTCTTCGAGACTACACCTCATCAAAAAACTGCTGATAACGGGATTCACTGAAACCAA
SEQ ID NO:5: PhtrA-htrA-loxp-aadA-loxp 
GATAATGTATTCACAGGTTGCTCCGGGCTTGAAAAAGTTAAATGACATCCATTTGTCCTACTCAGGAGAGCGTTC
ACCGACAAACAACAGATAAAACGAAAGGCCCAGTCTTTCGACTGAGCCTTTCGTTTTATTTGATGCACTAGTGCC
GATCATATTCAATAACCCTTAATATAACTTCGTATAATGTATGCTATACGAAGTTATTAGGTCTGAAGAGGAGTT
TACGTCCCATATGGAGTCCCGCCTTATTTGCCGACTACCTTGGTGATCTCGCCTTTCACGTAGTGGACAAATTCT
TCCAACTGATCTGCGCGCGAGGCCAAGCGATCTTCTTCTTGTCCAAGATAAGCCTGTCTAGCTTCAAGTATGACG
GGCTGATACTGGGCCGGCAGGCGCTCCATTGCCCAGTCGGCAGCGACATCCTTCGGCGCGATTTTGCCGGTTACT
GCGCTGTACCAAATGCGGGACAACGTAAGCACTACATTTCGCTCATCGCCAGCCCAGTCGGGCGGCGAGTTCCAT
AGCGTTAAGGTTTCATTTAGCGCCTCAAATAGATCCTGTTCAGGAACCGGATCAAAGAGTTCCTCCGCCGCTGGA
CCTACCAAGGCAACGCTATGTTCTCTTGCTTTTGTCAGCAAGATAGCCAGATCAATGTCGATCGTGGCTGGCTCG
AAGATACCTGCAAGAATGTCATTGCGCTGCCATTCTCCAAATTGCAGTTCGCGCTTAGCTGGATAACGCCACGGA
ATGATGTCGTCGTGCACAACAATGGTGACTTCTACAGCGCGGAGAATCTCGCTCTCTCCAGGGGAAGCCGAAGTT
TCCAAAAGGTCGTTGATCAAAGCTCGCCGCGTTGTTTCATCAAGCCTTACGGTCACCGTAACCAGCAAATCAATA
TCACTGTGTGGCTTCAGGCCGCCATCCACTGCGGAGCCGTACAAATGTACGGCCAGCAACGTCGGTTCGAGATGG
CGCTCGATGACGCCAACTACCTCTGATAGTTGAGTCGATACTTCGGCGATCACCGCTTCCCTCATGACATTGCAC
TCCACCGCTGATGACATCAGTCGATCATAGCACGATCAACGGCACTGTTGCAAATAGTCGGTGGTGATAAACTTA
TCATCCCCTTTTGCTGATGGAGCTGCACATGAACTCGAGACGTCATTGCGTTGCTAGCCAAGCTTAGGATCCGGA
ACCCTTAATATAACTTCGTATAATGTATGCTATACGAAGTTATTAGGTCCCTCGAAGAGGTTCACTAGCTATCTA
GAGCTTGGACTCCTGTTGATAGATCCAGTAATGACCTCAGAACTCCATCTGGATTTGTTCAGAACGCTCGGTTGC
CGCCGGGCGTTTTTTATTGGTGAGAATCCAAGCCTTATCGCTGTCGGTTGATTCAGGATTATATCAGCGGGATGA
CTGACCTTTACGCATGGGATGAATATCGGCGTTTGATGGCGGTCGAACAGTAAATGGACTTTTGTAAAGATGGAC
AATAAATTTTTACTTTTTCCAGAAACTTTATTCCGGAACTTCGCGTTATAAAATGAATCTGACGTACACAGCAAT
TTTGCGTTACCTGTTAATCGAGATTGAAACACATGAAAAAAACCACATTAGCAATGAGTGCACTGGCTCTGAGTT
TAGGTTTGGCATTGTCGCCTCTGTCTGCCACGGCGGCTGAAACGTCCTCTTCAGCAATGACTGCCCAGCAGATGC
CAAGCCTGGCACCGATGCTCGAAAAAGTGATGCCATCGGTGGTCAGTATTAATGTTGAAGGTAGCACCACGGTGA
ATACGCCGCGTATGCCGCGTAATTTCCAGCAGTTCTTTGGCGATGACTCCCCGTTCTGCCAGGACGGTTCTCCGT
TCCAGAATTCTCCGTTCTGCCAGGGCGGCGGTAACGGCGGCAACGGCGGTCAACAACAGAAATTCATGGCGCTGG
GCTCCGGCGTAATTATTGACGCCGCGAAGGGCTACGTCGTCACCAACAACCACGTGGTTGATAACGCCAGCGTGA
TTAAAGTACAGCTTAGCGATGGGCGTAAATTCGATGCTAAAGTGGTGGGCAAAGATCCGCGTTCTGATATCGCGC
TGATTCAAATTCAGAATCCGAAGAACCTGACGGCGATTAAGCTGGCGGACTCCGACGCGCTGCGCGTGGGGGATT
ATACCGTCGCTATTGGTAACCCGTTTGGTCTGGGCGAAACGGTGACGTCAGGTATCGTTTCGGCGCTGGGGCGTA
GCGGCCTGAACGTAGAAAATTACGAGAACTTTATTCAGACCGACGCCGCGATTAACCGTGGTAACTCCGGCGGCG
CGCTGGTGAACCTGAACGGTGAGCTGATCGGTATTAACACCGCGATTCTGGCGCCGGACGGCGGCAACATCGGTA
TCGGCTTCGCTATCCCCAGTAACATGGTGAAAAACCTGACGTCGCAGATGGTGGAATACGGCCAGGTGAAACGCG
GCGAACTGGGGATCATGGGGACTGAGCTGAATTCCGAATTGGCGAAAGCGATGAAAGTCGACGCCCAGCGAGGCG
CGTTCGTCAGCCAGGTGATGCCGAATTCGTCCGCGGCGAAAGCGGGTATCAAAGCCGGGGATGTCATTACCTCGC
TGAACGGTAAACCGATCAGCAGCTTTGCGGCGCTGCGCGCTCAGGTCGGCACTATGCCGGTCGGCAGCAAAATCA
GCCTCGGTCTGCTGCGTGAAGGTAAAGCGATTACGGTGAATCTGGAACTGCAGCAGAGCAGCCAGAGTCAGGTTG
ATTCCAGCACCATCTTCAGCGGGATTGAAGGCGCTGAAATGAGCAATAAAGGCCAGGATAAAGGCGTTGTGGTGA
GCAGCGTGAAAGCGAACTCACCCGCCGCGCAAATTGGCCTCAAAAAAGGCGATGTGATTATCGGCGCTAACCAGC
AGCCGGTGAAAAATATCGCCGAGCTGCGTAAGATTCTCGACAGCAAGCCGTCGGTTCTGGCGCTGAATATTCAGC
GTGGTGATAGTTCTATTTATTTGCTGATGCAGTAAGAAGTCGCGTAAGTTGCTAGCCCACGCGGCTCACCTTCAC
GGGTGGGCCTTTCTTCGTATACTAAGTTGAACGAAACGGGAAGGCGAAAAGGTAAAAAGTTCCTTTT
SEQ ID NO:6: Msbb-HR-loxp-KnaR-loxp 
AGGTAGTACAGGGTTTGTCAGCATAAAGCCTCTCTTACGAGAGGCTTTATGCCGATCATATTCAATAACCCTTAA
TATAACTTCGTATAATGTATGCTATACGAAGTTATTAGGTCTGAAGAGGAGTTTACGTCCCATATGGAGTCCCGC
TCAGAAGAACTCGTCAAGAAGGCGATAGAAGGCGATGCGCTGCGAATCGGGAGCGGCGATACCGTAAAGCACGAG
GAAGCGGTCAGCCCATTCGCCGCCAAGCTCTTCAGCAATATCACGGGTAGCCAACGCTATGTCCTGATAGCGGTC
CGCCACACCCAGCCGGCCACAGTCGATGAATCCAGAAAAGCGGCCATTTTCCACCATGATATTCGGCAAGCAGGC
ATCGCCATGGGTCACGACGAGATCCTCGCCGTCGGGCATGCGCGCCTTGAGCCTGGCGAACAGTTCGGCCGGCGC
GAGCCCCTGATGCTCTTCGTCCAGATCATCCTGATCGACAAGACCGGCTTCCATCCGAGTACGTGCTCGCTCGAT
GCGATGTTTCGCTTGGTGGTCGAATGGGCAGGTAGCCGGATCAAGCGTATGCAGCCGCCGCATTGCATCAGCCAT
GATGGATACTTTCTCGGCAGGAGCAAGGTGAGATGACAGGAGATCCTGCCCCGGCACTTCGCCCAATAGCAGCCA
GTCCCTTCCCGCTTCAGTGACAACGTCGAGCACAGCTGCGCAAGGAACGCCCGTCGTGGCCAGCCACGATAGCCG
CGCTGCCTCGTCCTGCAGTTCATTCAGGGCACCGGACAGGTCGGTCTTGACAAAAAGAACCGGGCGCCCCTGCGC
TGACAGCCGGAACACGGCGGCATCAGAGCAGCCGATTGTCTGTTGTGCCCAGTCATAGCCGAATAGCCTCTCCAC
CCAAGCGGCCGGAGAACCTGCGTGCAATCCATCTTGTTCAATCATGCGAAACGATCCTCATCCTGTCTCTTGATC
AGATCCTGATCCCCTGCGCCATCAGATCCTTGGCGGCAAGAAAGCCATCCAGTTTACTTTGCAGGGCTTCCCAAC
CTTACCAGAGGGCGCCCCAGCTGGCAATTCCGACGTCATTGCGTTGCTAGCCAAGCTTAGGATCCGGAACCCTTA
ATATAACTTCGTATAATGTATGCTATACGAAGTTATTAGGTCCCTCGAAGAGGTTCACTAGGCTTTTCCAGGGTC
TGCTGACGCGAAAAGGAATTGTGAATAGTGTAGCGA
SEQ ID NO:7: Lpp-HR-loxp-Knar-loxp 
CGGACAAAAAAATGGCGCACGATGTGCGCCATTTTATATTGTGCGTCAAAGCCGATCATATTCAATAACCCTTAA
TATAACTTCGTATAATGTATGCTATACGAAGTTATTAGGTCTGAAGAGGAGTTTACGTCCCATATGGAGTCCCGC
TCAGAAGAACTCGTCAAGAAGGCGATAGAAGGCGATGCGCTGCGAATCGGGAGCGGCGATACCGTAAAGCACGAG
GAAGCGGTCAGCCCATTCGCCGCCAAGCTCTTCAGCAATATCACGGGTAGCCAACGCTATGTCCTGATAGCGGTC
CGCCACACCCAGCCGGCCACAGTCGATGAATCCAGAAAAGCGGCCATTTTCCACCATGATATTCGGCAAGCAGGC
ATCGCCATGGGTCACGACGAGATCCTCGCCGTCGGGCATGCGCGCCTTGAGCCTGGCGAACAGTTCGGCCGGCGC
GAGCCCCTGATGCTCTTCGTCCAGATCATCCTGATCGACAAGACCGGCTTCCATCCGAGTACGTGCTCGCTCGAT
GCGATGTTTCGCTTGGTGGTCGAATGGGCAGGTAGCCGGATCAAGCGTATGCAGCCGCCGCATTGCATCAGCCAT
GATGGATACTTTCTCGGCAGGAGCAAGGTGAGATGACAGGAGATCCTGCCCCGGCACTTCGCCCAATAGCAGCCA
GTCCCTTCCCGCTTCAGTGACAACGTCGAGCACAGCTGCGCAAGGAACGCCCGTCGTGGCCAGCCACGATAGCCG
CGCTGCCTCGTCCTGCAGTTCATTCAGGGCACCGGACAGGTCGGTCTTGACAAAAAGAACCGGGCGCCCCTGCGC
TGACAGCCGGAACACGGCGGCATCAGAGCAGCCGATTGTCTGTTGTGCCCAGTCATAGCCGAATAGCCTCTCCAC
CCAAGCGGCCGGAGAACCTGCGTGCAATCCATCTTGTTCAATCATGCGAAACGATCCTCATCCTGTCTCTTGATC
AGATCCTGATCCCCTGCGCCATCAGATCCTTGGCGGCAAGAAAGCCATCCAGTTTACTTTGCAGGGCTTCCCAAC
CTTACCAGAGGGCGCCCCAGCTGGCAATTCCGACGTCATTGCGTTGCTAGCCAAGCTTAGGATCCGGAACCCTTA
ATATAACTTCGTATAATGTATGCTATACGAAGTTATTAGGTCCCTCGAAGAGGTTCACTAGTATTAATACCCTCT
AGATTGAGTTAATCTCCATGTAGCGTTACAAGTATT
SEQ ID NO:8: fliC-HR-loxp-KnaR-loxp 
GCTTTCGCTGCCTTGATTGTGTACCACGTGTCGGTGAATCAATCGCCGGAGCCGATCATATTCAATAACCCTTAA
TATAACTTCGTATAATGTATGCTATACGAAGTTATTAGGTCTGAAGAGGAGTTTACGTCCCATATGGAGTCCCGC
TCAGAAGAACTCGTCAAGAAGGCGATAGAAGGCGATGCGCTGCGAATCGGGAGCGGCGATACCGTAAAGCACGAG
GAAGCGGTCAGCCCATTCGCCGCCAAGCTCTTCAGCAATATCACGGGTAGCCAACGCTATGTCCTGATAGCGGTC
CGCCACACCCAGCCGGCCACAGTCGATGAATCCAGAAAAGCGGCCATTTTCCACCATGATATTCGGCAAGCAGGC
ATCGCCATGGGTCACGACGAGATCCTCGCCGTCGGGCATGCGCGCCTTGAGCCTGGCGAACAGTTCGGCCGGCGC
GAGCCCCTGATGCTCTTCGTCCAGATCATCCTGATCGACAAGACCGGCTTCCATCCGAGTACGTGCTCGCTCGAT
GCGATGTTTCGCTTGGTGGTCGAATGGGCAGGTAGCCGGATCAAGCGTATGCAGCCGCCGCATTGCATCAGCCAT
GATGGATACTTTCTCGGCAGGAGCAAGGTGAGATGACAGGAGATCCTGCCCCGGCACTTCGCCCAATAGCAGCCA
GTCCCTTCCCGCTTCAGTGACAACGTCGAGCACAGCTGCGCAAGGAACGCCCGTCGTGGCCAGCCACGATAGCCG
CGCTGCCTCGTCCTGCAGTTCATTCAGGGCACCGGACAGGTCGGTCTTGACAAAAAGAACCGGGCGCCCCTGCGC
 
TGACAGCCGGAACACGGCGGCATCAGAGCAGCCGATTGTCTGTTGTGCCCAGTCATAGCCGAATAGCCTCTCCAC
CCAAGCGGCCGGAGAACCTGCGTGCAATCCATCTTGTTCAATCATGCGAAACGATCCTCATCCTGTCTCTTGATC
AGATCCTGATCCCCTGCGCCATCAGATCCTTGGCGGCAAGAAAGCCATCCAGTTTACTTTGCAGGGCTTCCCAAC
CTTACCAGAGGGCGCCCCAGCTGGCAATTCCGACGTCATTGCGTTGCTAGCCAAGCTTAGGATCCGGAACCCTTA
ATATAACTTCGTATAATGTATGCTATACGAAGTTATTAGGTCCCTCGAAGAGGTTCACTAGATCAATTACAACTT
GATGTTATTGGGCTGTTGCCCACGGTTTCTCACCGT
SEQ ID NO:9: PsseA-hlyA-loxp-KnaR-loxp plasmid 
CATGGTCATAGCTGTTTCCTTTAATGCCCATGGCGTTTACCAATGCTTAATCAGTGAGGCACCTATCTCAGCGAT
CTGTCTATTTCGTTCATCCATAGTTGCCTGACTCCCCGTCGTGTAGATAACTACGATACGGGAGGGCTTACCATC
TGGCCCCAGTGCTGCAATGATACCGCGACTTCCACGCTCACCGGCTCCAGATTTATCAGCAATAAACCAGCCAGC
CGGAAGGGCCGAGCGCAGAAGTGGTCCTGCAACTTTATCCGCCTCCATCCAGTCTATTAATTGTTGCCGGGAAGC
TAGAGTAAGTAGTTCGCCAGTTAATAGTTTGCGCAACGTTGTTGCCATTGCTACAGGCATCGTGGTGTCACGCTC
GTCGTTTGGTATGGCTTCATTCAGCTCCGGTTCCCAACGATCAAGGCGAGTTACATGATCCCCCATGTTGTGCAA
AAAAGCGGTTAGCTCCTTCGGTCCTCCGATCGTTGTCAGAAGTAAGTTGGCCGCAGTGTTATCACTCATGGTTAT
GGCAGCACTGCATAATTCTCTTACTGTCATGCCATCCGTAAGATGCTTTTCTGTGACTGGTGAGTACTCAACCAA
GTCATTCTGAGAATAGTGTATGCGGCGACCGAGTTGCTCTTGCCCGGCGTCAATACGGGATAATACCGCGCCACA
TAGCAGAACTTTAAAAGTGCTCATCATTGGAAAACGTTCTTCGGGGCGAAAACTCTCAAGGATCTTACCGCTGTT
GAGATCCAGTTCGATGTAACCCACTCGTGCACCCAACTGATCTTCAGCATCTTTTACTTTCACCAGCGTTTCTGG
GTGAGCAAAAACAGGAAGGCAAAATGCCGCAAAAAAGGGAATAAGGGCGACACGGAAATGTTGAATACTCATACT
CTTCCTTTTTCAATATTATTGAAGCATTTATCAGGGTTATTGTCTCATGAGCGGATACATATTTGAATGTATTTA
GAAAAATAAACAAATAGGGGTTCCGCGCACATTTCCCCGAAAAGTGCCACCTGACGTCTAAGAAACCATTATTAT
CATGACATTAACCTATAAAAATAGGCGTATCACGAGGCCCTTAGGCCAAATAGGCCGTTCAGATCCTTCCGTATT
TAGCCAGTATGTTCTCTAGTGTGGTTCGTTGTTTTTGCGTGAGCCATGAGAACGAACCATTGAGATCATGCTTAC
TTTGCATGTCACTCAAAAATTTTGCCTCAAAACTGGTGAGCTGAATTTTTGCAGTTAAAGCATCGTGTAGTGTTT
TTCTTAGTCCGTTACGTAGGTAGGAATCTGATGTAATGGTTGTTGGTATTTTGTCACCATTCATTTTTATCTGGT
TGTTCTCAAGTTCGGTTACGAGATCCATTTGTCTATCTAGTTCAACTTGGAAAATCAACGTATCAGTCGGGCGGC
CTCGCTTATCAACCACCAATTTCATATTGCTGTAAGTGTTTAAATCTTTACTTATTGGTTTCAAAACCCATTGGT
TAAGCCTTTTAAACTCATGGTAGTTATTTTCAAGCATTAACATGAACTTAAATTCATCAAGGCTAATCTCTATAT
TTGCCTTGTGAGTTTTCTTTTGTGTTAGTTCTTTTAATAACCACTCATAAATCCTCATAGAGTATTTGTTTTCAA
AAGACTTAACATGTTCCAGATTATATTTTATGAATTTTTTTAACTGGAAAAGATAAGGCAATATCTCTTCACTAA
AAACTAATTCTAATTTTTCGCTTGAGAACTTGGCATAGTTTGTCCACTGGAAAATCTCAAAGCCTTTAACCAAAG
GATTCCTGATTTCCACAGTTCTCGTCATCAGCTCTCTGGTTGCTTTAGCTAATACACCATAAGCATTTTCCCTAC
TGATGTTCATCATCTGAGCGTATTGGTTATAAGTGAACGATACCGTCCGTTCTTTCCTTGTAGGGTTTTCAATCG
TGGGGTTGAGTAGTGCCACACAGCATAAAATTAGCTTGGTTTCATGCTCCGTTAAGTCATAGCGACTAATCGCTA
GTTCATTTGCTTTGAAAACAACTAATTCAGACATACATCTCAATTGGTCTAGGTGATTTTAATCACTATACCAAT
TGAGATGGGCTAGTCAATGATAATTACTAGTCCTTTTCCTTTGAGTTGTGGGTATCTGTAAATTCTGCTAGACCT
TTGCTGGAAAACTTGTAAATTCTGCTAGACCCTCTGTAAATTCCGCTAGACCTTTGTGTGTTTTTTTTGTTTATA
TTCAAGTGGTTATAATTTATAGAATAAAGAAAGAATAAAAAAAGATAAAAAGAATAGATCCCAGCCCTGTGTATA
ACTCACTACTTTAGTCAGTTCCGCAGTATTACAAAAGGATGTCGCAAACGCTGTTTGCTCCTCTACAAAACAGAC
CTTAAAACCCTAAAGGCTTAAGTAGCACCCTCGCAAGCTCGGGCAAATCGCTGAATATTCCTTTTGTCTCCGACC
ATCAGGCACCTGAGTCGCTGTCTTTTTCGTGACATTCAGTTCGCTGCGCTCACGGCTCTGGCAGTGAATGGGGGT
AAATGGCACTACAGGCGCCTTTTATGGATTCATGCAAGGAAACTACCCATAATACAAGAAAAGCCCGTCACGGGC
TTCTCAGGGCGTTTTATGGCGGGTCTGCTATGTGGTGCTATCTGACTTTTTGCTGTTCAGCAGTTCCTGCCCTCT
GATTTTCCAGTCTGACCACTTCGGATTATCCCGTGACAGGTCATTCAGACTGGCTAATGCACCCAGTAAGGCAGC
GGTATCATCAACAGGCTTACCCGTCTTACTGTCAAGAGGACATCCGGTCAGATAACCGTCATCTTCGGCTACTGT
CTAGGGCGGCGGATTTGTCCTACTCAGGAGAGCGTTCACCGACAAACAACAGATAAAACGAAAGGCCCAGTCTTT
CGACTGAGCCTTTCGTTTTATTTGATGCACTAGTGCCGATCATATTCAATAACCCTTAATATAACTTCGTATAAT
GTATGCTATACGAAGTTATTAGGTCTGAAGAGGAGTTTACGTCCCATATGGAGTCCCGCTCAGAAGAACTCGTCA
AGAAGGCGATAGAAGGCGATGCGCTGCGAATCGGGAGCGGCGATACCGTAAAGCACGAGGAAGCGGTCAGCCCAT
 
TCGCCGCCAAGCTCTTCAGCAATATCACGGGTAGCCAACGCTATGTCCTGATAGCGGTCCGCCACACCCAGCCGG
CCACAGTCGATGAATCCAGAAAAGCGGCCATTTTCCACCATGATATTCGGCAAGCAGGCATCGCCATGGGTCACG
ACGAGATCCTCGCCGTCGGGCATGCGCGCCTTGAGCCTGGCGAACAGTTCGGCCGGCGCGAGCCCCTGATGCTCT
TCGTCCAGATCATCCTGATCGACAAGACCGGCTTCCATCCGAGTACGTGCTCGCTCGATGCGATGTTTCGCTTGG
TGGTCGAATGGGCAGGTAGCCGGATCAAGCGTATGCAGCCGCCGCATTGCATCAGCCATGATGGATACTTTCTCG
GCAGGAGCAAGGTGAGATGACAGGAGATCCTGCCCCGGCACTTCGCCCAATAGCAGCCAGTCCCTTCCCGCTTCA
GTGACAACGTCGAGCACAGCTGCGCAAGGAACGCCCGTCGTGGCCAGCCACGATAGCCGCGCTGCCTCGTCCTGC
AGTTCATTCAGGGCACCGGACAGGTCGGTCTTGACAAAAAGAACCGGGCGCCCCTGCGCTGACAGCCGGAACACG
GCGGCATCAGAGCAGCCGATTGTCTGTTGTGCCCAGTCATAGCCGAATAGCCTCTCCACCCAAGCGGCCGGAGAA
CCTGCGTGCAATCCATCTTGTTCAATCATGCGAAACGATCCTCATCCTGTCTCTTGATCAGATCCTGATCCCCTG
CGCCATCAGATCCTTGGCGGCAAGAAAGCCATCCAGTTTACTTTGCAGGGCTTCCCAACCTTACCAGAGGGCGCC
CCAGCTGGCAATTCCGACGTCATTGCGTTGCTAGCCAAGCTTAGGATCCGGAACCCTTAATATAACTTCGTATAA
TGTATGCTATACGAAGTTATTAGGTCCCTCGAAGAGGTTCACTAGCTATCTAGAGCTTGGACTCCTGTTGATAGA
TCCAGTAATGACCTCAGAACTCCATCTGGATTTGTTCAGAACGCTCGGTTGCCGCCGGGCGTTTTTTATTGGTGA
GAATCCAAGCCCGGGAAACGGGATCCTGATCTTGATCAGGCTGTGGGTCAGGCCACTCTTGGCATCGACACCAAT
GTGGGCCTTCATGCCAAAGTGCCACTGATTGCCTTTCTTGGTCTGATGCATCTCCGGATCGCGTTGCTGCTCTTT
GTTCTTGGTCGAGCTGGGTGCCTCAATGATGGTGGCATCGACCAAGGTGCCTTGAGTCATCATGACGCCTGCTTC
GGCCAGCCAGCGATTGATGGTCTTGAACAATTGGCGGGCCAGTTGATGCTGCTCCAGCAGGTGGCGGAAATTCAT
GATGGTGGTGCGGTCCGGCAAGGCGCTATCCAGGGATAACCGGGCAAACAGACGCATGGAGGCGATTTCGTACAG
AGCATCTTCCATCGCGCCATCGCTCAGGTTGTACCAATGCTGCATGCAGTGAATGCGTAGCATGGTTTCCAGCGG
ATAAGGTCGCCGGCCATTACCAGCCTTGGGGTAAAACGGCTCGATGACTTCCACCATGTTTTGCCATGGCAGAAT
CTGCTCCATGCGGGACAAGAAAATCTCTTTTCTGGTCTGACGGCGCTTACTGCTGAATTCACTGTCGGCGAAGGT
AAGTTGATGACTCATGATGAACCCTGTTCTATGGCTCCAGATGACAAACATGATCTCATATCAGGGACTTGTTCG
CACCTTCCTTAGCACGTTAATTATCTATCGTGTATATGGAGGGGATCTAGAATGAAAAAAATAATGCTAGTTTTT
ATTACACTTATATTAGTTAGTCTACCAATTGCGCAACAAACTGAAGCAAAGGATGCATCTGCATTCAATAAAGAA
AATTCAATTTCATCCATGGCACCACCAGCATCTCCGCCTGCAAGTCCTAAGACGCCAATCGAAAAGAAACACGCG
GATGAAATCGATAAGTATATACAAGGATTGGACTACAATAAAAACAATGTATTAGTATACCACGGAGATGCAGTG
ACAAATGTGCCGCCAAGAAAAGGTTACAAAGATGGAAATGAATATATTGTTGTGGAGAAAAAGAAGAAATCCATC
AATCAAAATAATGCAGACATTCAAGTTGTGAATGCAATTTCGAGCCTAACCTATCCAGGTGCTCTCGTAAAAGCG
AATTCGGAATTAGTAGAAAATCAACCAGATGTTCTCCCTGTAAAACGTGATTCATTAACACTCAGCATTGATTTG
CCAGGTATGACTAATCAAGACAATAAAATCGTTGTAAAAAATGCCACTAAATCAAACGTTAACAACGCAGTAAAT
ACATTAGTGGAAAGATGGAATGAAAAATATGCTCAAGCTTATCCAAATGTAAGTGCAAAAATTGATTATGATGAC
GAAATGGCTTACAGTGAATCACAATTAATTGCGAAATTTGGTACAGCATTTAAAGCTGTAAATAATAGCTTGAAT
GTAAACTTCGGCGCAATCAGTGAAGGGAAAATGCAAGAAGAAGTCATTAGTTTTAAACAAATTTACTATAACGTG
AATGTTAATGAACCTACAAGACCTTCCAGATTTTTCGGCAAAGCTGTTACTAAAGAGCAGTTGCAAGCGCTTGGA
GTGAATGCAGAAAATCCTCCTGCATATATCTCAAGTGTGGCGTATGGCCGTCAAGTTTATTTGAAATTATCAACT
AATTCCCATAGTACTAAAGTAAAAGCTGCTTTTGATGCTGCCGTAAGCGGAAAATCTGTCTCAGGTGATGTAGAA
CTAACAAATATCATCAAAAATTCTTCCTTCAAAGCCGTAATTTACGGAGGTTCCGCAAAAGATGAAGTTCAAATC
ATCGACGGCAACCTCGGAGACTTACGAGATATTTTGAAAAAAGGCGCTACTTTTAATCGAGAAACACCAGGAGTT
CCCATTGCTTATACAACAAACTTCCTAAAAGACAATGAATTAGCTGTTATTAAAAACAACTCAGAATATATTGAA
ACAACTTCAAAAGCTTATACAGATGGAAAAATTAACATCGATCACTCTGGAGGATACGTTGCTCAATTCAACATT
TCTTGGGATGAAGTAAATTATGATCCTGAAGGTAACGAAATTGTTCAACATAAAAACTGGAGCGAAAACAATAAA
AGTAAGCTAGCTCATTTCACATCGTCCATCTATTTGCCAGGTAACGCGAGAAATATTAATGTTTACGCTAAAGAA
TGCACTGGTTTAGCTTGGGAATGGTGGAGAACGGTAATTGATGACCGGAACTTACCACTTGTGAAAAATAGAAAT
ATCTCCATCTGGGGCACCACGCTTTATCCGAAATATAGTAATAAAGTAGATAATCCAATCGAATAAGTCGACCTC
GAGGACTAGTGAACCTCTTCGAGGGACCTAATAACTTCGTATAGCATACATTATACGAAGTTATATTAAGGGTTA
TTGAATATGATCGGCTCGAGATTACTTCGCATTGCAATCAGCTACGGACTCAAGCTTACGTCAGGTCATACGATG
AAGGCGATAAGTCGCGTAAGTTGCTAGCCCACGCGGCTCACCTTCACGGGTGGGCCTTTCTTCGCGATCCACATG
SEQ ID NO:10: Pssbp1-dapE-Cm plasmid 
CATGGTCATAGCTGTTTCCTTTAATGCCCATGGCGTTTACCAATGCTTAATCAGTGAGGCACCTATCTCAGCGAT
CTGTCTATTTCGTTCATCCATAGTTGCCTGACTCCCCGTCGTGTAGATAACTACGATACGGGAGGGCTTACCATC
TGGCCCCAGTGCTGCAATGATACCGCGACTTCCACGCTCACCGGCTCCAGATTTATCAGCAATAAACCAGCCAGC
CGGAAGGGCCGAGCGCAGAAGTGGTCCTGCAACTTTATCCGCCTCCATCCAGTCTATTAATTGTTGCCGGGAAGC
TAGAGTAAGTAGTTCGCCAGTTAATAGTTTGCGCAACGTTGTTGCCATTGCTACAGGCATCGTGGTGTCACGCTC
GTCGTTTGGTATGGCTTCATTCAGCTCCGGTTCCCAACGATCAAGGCGAGTTACATGATCCCCCATGTTGTGCAA
AAAAGCGGTTAGCTCCTTCGGTCCTCCGATCGTTGTCAGAAGTAAGTTGGCCGCAGTGTTATCACTCATGGTTAT
GGCAGCACTGCATAATTCTCTTACTGTCATGCCATCCGTAAGATGCTTTTCTGTGACTGGTGAGTACTCAACCAA
GTCATTCTGAGAATAGTGTATGCGGCGACCGAGTTGCTCTTGCCCGGCGTCAATACGGGATAATACCGCGCCACA
TAGCAGAACTTTAAAAGTGCTCATCATTGGAAAACGTTCTTCGGGGCGAAAACTCTCAAGGATCTTACCGCTGTT
GAGATCCAGTTCGATGTAACCCACTCGTGCACCCAACTGATCTTCAGCATCTTTTACTTTCACCAGCGTTTCTGG
GTGAGCAAAAACAGGAAGGCAAAATGCCGCAAAAAAGGGAATAAGGGCGACACGGAAATGTTGAATACTCATACT
CTTCCTTTTTCAATATTATTGAAGCATTTATCAGGGTTATTGTCTCATGAGCGGATACATATTTGAATGTATTTA
GAAAAATAAACAAATAGGGGTTCCGCGCACATTTCCCCGAAAAGTGCCACCTGACGTCTAAGAAACCATTATTAT
CATGACATTAACCTATAAAAATAGGCGTATCACGAGGCCCTTAGGCCAAATAGGCCGTTCAGATCCTTCCGTATT
TAGCCAGTATGTTCTCTAGTGTGGTTCGTTGTTTTTGCGTGAGCCATGAGAACGAACCATTGAGATCATGCTTAC
TTTGCATGTCACTCAAAAATTTTGCCTCAAAACTGGTGAGCTGAATTTTTGCAGTTAAAGCATCGTGTAGTGTTT
TTCTTAGTCCGTTACGTAGGTAGGAATCTGATGTAATGGTTGTTGGTATTTTGTCACCATTCATTTTTATCTGGT
TGTTCTCAAGTTCGGTTACGAGATCCATTTGTCTATCTAGTTCAACTTGGAAAATCAACGTATCAGTCGGGCGGC
CTCGCTTATCAACCACCAATTTCATATTGCTGTAAGTGTTTAAATCTTTACTTATTGGTTTCAAAACCCATTGGT
TAAGCCTTTTAAACTCATGGTAGTTATTTTCAAGCATTAACATGAACTTAAATTCATCAAGGCTAATCTCTATAT
TTGCCTTGTGAGTTTTCTTTTGTGTTAGTTCTTTTAATAACCACTCATAAATCCTCATAGAGTATTTGTTTTCAA
AAGACTTAACATGTTCCAGATTATATTTTATGAATTTTTTTAACTGGAAAAGATAAGGCAATATCTCTTCACTAA
AAACTAATTCTAATTTTTCGCTTGAGAACTTGGCATAGTTTGTCCACTGGAAAATCTCAAAGCCTTTAACCAAAG
GATTCCTGATTTCCACAGTTCTCGTCATCAGCTCTCTGGTTGCTTTAGCTAATACACCATAAGCATTTTCCCTAC
TGATGTTCATCATCTGAGCGTATTGGTTATAAGTGAACGATACCGTCCGTTCTTTCCTTGTAGGGTTTTCAATCG
TGGGGTTGAGTAGTGCCACACAGCATAAAATTAGCTTGGTTTCATGCTCCGTTAAGTCATAGCGACTAATCGCTA
GTTCATTTGCTTTGAAAACAACTAATTCAGACATACATCTCAATTGGTCTAGGTGATTTTAATCACTATACCAAT
TGAGATGGGCTAGTCAATGATAATTACTAGTCCTTTTCCTTTGAGTTGTGGGTATCTGTAAATTCTGCTAGACCT
TTGCTGGAAAACTTGTAAATTCTGCTAGACCCTCTGTAAATTCCGCTAGACCTTTGTGTGTTTTTTTTGTTTATA
TTCAAGTGGTTATAATTTATAGAATAAAGAAAGAATAAAAAAAGATAAAAAGAATAGATCCCAGCCCTGTGTATA
ACTCACTACTTTAGTCAGTTCCGCAGTATTACAAAAGGATGTCGCAAACGCTGTTTGCTCCTCTACAAAACAGAC
CTTAAAACCCTAAAGGCTTAAGTAGCACCCTCGCAAGCTCGGGCAAATCGCTGAATATTCCTTTTGTCTCCGACC
ATCAGGCACCTGAGTCGCTGTCTTTTTCGTGACATTCAGTTCGCTGCGCTCACGGCTCTGGCAGTGAATGGGGGT
AAATGGCACTACAGGCGCCTTTTATGGATTCATGCAAGGAAACTACCCATAATACAAGAAAAGCCCGTCACGGGC
TTCTCAGGGCGTTTTATGGCGGGTCTGCTATGTGGTGCTATCTGACTTTTTGCTGTTCAGCAGTTCCTGCCCTCT
GATTTTCCAGTCTGACCACTTCGGATTATCCCGTGACAGGTCATTCAGACTGGCTAATGCACCCAGTAAGGCAGC
GGTATCATCAACAGGCTTACCCGTCTTACTGTCAAGAGGACATCCGGTCAGATAACCGTCATCTTCGGCTACTGT
CTAGGGCGGCGGATTTGTCCTACTCAGGAGAGCGTTCACCGACAAACAACAGATAAAACGAAAGGCCCAGTCTTT
CGACTGAGCCTTTCGTTTTATTTGATGCACTAGTGCCGATCATATTCAATAACCCTTAATATAACTTCGTATAAT
GTATGCTATACGAAGTTATTAGGTCTGAAGAGGAGTTTACGTCCCATATGGAGTCCCGCTTACGCCCCGCCCTGC
CACTCATCGCAGTACTGTTGTAATTCATTAAGCATTCTGCCGACATGGAAGCCATCACAGACGGCATGATGAACC
TGAATCGCCAGCGGCATCAGCACCTTGTCGCCTTGCGTATAATATTTGCCCATGGTGAAAACGGGGGCGAAGAAG
TTGTCCATATTGGCCACGTTTAAATCAAAACTGGTGAAACTCACCCAGGGATTGGCTGAGACGAAAAACATATTC
TCAATAAACCCTTTAGGGAAATAGGCCAGGTTTTCACCGTAACACGCCACATCTTGCGAATATATGTGTAGAAAC
TGCCGGAAATCGTCGTGGTATTCACTCCAGAGCGATGAAAACGTTTCAGTTTGCTCATGGAAAACGGTGTAACAA
GGGTGAACACTATCCCATATCACCAGCTCACCGTCTTTCATTGCCATACGAAATTCCGGATGAGCATTCATCAGG
CGGGCAAGAATGTGAATAAAGGCCGGATAAAACTTGTGCTTATTTTTCTTTACGGTCTTTAAAAAGGCCGTAATA
TCCAGCTGAACGGTCTGGTTATAGGTACATTGAGCAACTGACTGAAATGCCTCAAAATGTTCTTTACGATGCCAT
TGGGATATATCAACGGTGGTATATCCAGTGATTTTTTTCTCCATTTTAGCTTCCTTAGCTCCTGAAAATCTCGAT
 
AACTCAAAAAATACGCCCGGTAGTGATCTTATTTCATTATGGTGAAAGTTGGAACCTCTTACGTGCCGATCAGAC
GTCATTGCGTTGCTAGCCAAGCTTAGGATCCGGAACCCTTAATATAACTTCGTATAATGTATGCTATACGAAGTT
ATTAGGTCCCTCGAAGAGGTTCACTAGCTATCTAGAGCTTGGACTCCTGTTGATAGATCCAGTAATGACCTCAGA
ACTCCATCTGGATTTGTTCAGAACGCTCGGTTGCCGCCGGGCGTTTTTTATTGGTGAGAATCCAAGCCCGGGAAA
CGGGATCCTGATCTTGATCAACCGAGGTCACAACATAGTAAAAGCGCTATTGGTAATGGTACAATCGCGCGTTTA
CACTTATTCAGAACGATTTTTTTCAGGAGGGCCTACTTGTCACACAGGACGGTAATGTCGTGCCCGGTTATTGAG
CTGACACAGCAACTTATTCGTCGCCCCTCCCTGAGTCCGGATGACGCAGGCTGTCAGGCATTAATGATTGAACGC
CTGCGTAAAATCGGTTTTACCATTGAGCATATGGATTTTGGCGACACGCAAAATTTTTGGGCATGGCGTGGGCGT
GGCGAAACGCTGGCGTTCGCCGGGCATACTGACGTCGTACCGGCTGGCGATGTCGATCGCTGGATCAATCCGCCG
TTTGAGCCTACGATTCGCGACGGAATGTTGTTCGGTCGCGGCGCGGCGGATATGAAAGGTTCACTGGCGGCCATG
GTGGTGGCTGCTGAACGTTTTGTTGCGCAGCATCCTCATCACCGGGGCCGCCTCGCATTTTTGATTACCTCTGAT
GAGGAAGCCAGCGCAAAAAATGGCACCGTAAAGGTGGTTGAAGCGCTGATGGCCCGTAACGAGCGGCTGGATTAC
TGTCTGGTCGGCGAACCTTCCAGTACTGAAATTGTGGGCGATGTCGTCAAAAACGGGCGTCGCGGTTCTCTCACC
TGTAACCTCACTATCCATGGCGTTCAGGGGCACGTCGCCTATCCGCATCTGGCGGATAACCCTGTTCATCGCGCC
GCGCCTTTTCTTAATGAACTGGTCGCTATCGAGTGGGATCGGGGCAATGACTTTTTCCCGGCCACCAGTATGCAG
GTAGCCAATATCCAGGCAGGCACCGGCAGCAATAATGTCATTCCCGGCGAACTGTTCGTGCAGTTTAACTTCCGT
TTCAGCACGGAACTGACCGATGAGATGATCAAAGAGCGGGTACATGCGCTGCTTGAAAAGCATCAGCTACGCTAT
ACCGTAGACTGGTGGCTTTCCGGGCAGCCGTTCCTGACCGCACGCGGCAAGCTGGTGGACGCGGTGGTAAACGCC
ATTGAGCACTATAATGAAATTAAACCGCAATTACTGACGACGGGCGGTACGTCCGACGGACGTTTTATCGCCCGT
ATGGGAGCGCAGGTGGTAGAACTGGGGCCGGTGAACGCCACCATTCATAAAATTAATGAATGTGTGAATGCCGCC
GATCTCCAGCTACTGGCCCGTATGTATCAACGCATTATGGAACAGCTCGTCGCCCATCACCATCACCATCACTAA
GATTACTTCGCATTGCAATCAGCTACGGACTCAAGCTTACGTCAGGTCATACGATGAAGGCGATAAGTCGCGTAA
GTTGCTAGCCCACGCGGCTCACCTTCACGGGTGGGCCTTTCTTCGCGATCCACATG
SEQ ID NO:11: PsseA-hlyA-loxp-KnaR-loxp forward primer 
TTCACAGAAAAGTGTTGCCCCCTTCCATGGCGGAAGGGGGACAAAGGTGAATTTGTCCTACTCAGGAGAGCGTTC
A
SEQ ID NO:12: PsseA-hlyA-loxp-KnaR-loxp reverse primer
   TCTGACGTACACAGCAATTTTGCGTTACCTGTTAATCGAGATTGAAACACCGAAGAAAGGCCCACCCG
SEQ ID NO:13: Pssbp1-dapE-Cm forward primer TTGGTTTCAGTGAATCCCGTTATCAGCAGTTTTTTGATGAGGTGTAGTCTATTTGTCCTACTCAGGAGAGCG
SEQ ID NO:14: Pssbp1-dapE-Cm reverse primer
   CAATATTTTGCCAGCCAGTCCATGCTTATTTCCTCTTACCGGAACGCTCACGAAGAAAGGCCCACCCG
SEQ ID NO:15: PdapE-dapE-loxp-KnaR-loxp forward primer
   CAATATTTTGCCAGCCAGTCCATGCTTATTTCCTCTTACCGGAACGCTCAATTTGTCCTACTCAG
SEQ ID NO:16: PdapE-dapE-loxp-KnaR-loxp reverse primer
   TTGGTTTCAGTGAATCCCGTTATCAGCAGTTTTTTGATGAGGTGTAGTCTCGAAGAAAGGCCCACCCG
SEQ ID NO:17: loxp-KnaR-loxp forward primer
   ATGAAAAAAATAATGCTAGTTTTTATTACACTTATATTAGTTAGTCTACCGCCGATCATATTCAATAACCC
SEQ ID NO:18: loxp-KnaR-loxp reverse primer
   TTATTCGATTGGATTATCTACTTTATTACTATATTTCGGATAAAGCGTGGCTAGTGAACCTCTTCGAGGG
SEQ ID NO:19: PhtrA-htrA-loxp-aadA-loxp forward primer
   GATAATGTATTCACAGGTTGCTCCGGGCTTGAAAAAGTTAAATGACATCCATTTGTCCTACTCAGGAGAGC
SEQ ID NO:20: PhtrA-htrA-loxp-aadA-loxp reverse primer
   AAAAGGAACTTTTTACCTTTTCGCCTTCCCGTTTCGTTCAACTTAGTATACGAAGAAAGGCCCACCC
SEQ ID NO:21: msbB-HR-loxp-KnaR-loxp forward primer
   AGGTAGTACAGGGTTTGTCAGCATAAAGCCTCTCTTACGAGAGGCTTTATGCCGATCATATTCAATAACCC
SEQ ID NO:22: msbB-HR-loxp-KnaR-loxp reverse primer
   TCGCTACACTATTCACAATTCCTTTTCGCGTCAGCAGACCCTGGAAAAGCCTAGTGAACCTCTTCGAGGG
SEQ ID NO:23: lpp-HR-loxp-KnaR-loxp forward primer CGGACAAAAAAATGGCGCACGATGTGCGCCATTTTATATTGTGCGTCAAAGCCGATCATATTCAATAACCC
SEQ ID NO:24: lpp-HR-loxp-KnaR-loxp reverse primer
   AATACTTGTAACGCTACATGGAGATTAACTCAATCTAGAGGGTATTAATACTAGTGAACCTCTTCGAGGG
SEQ ID NO:25: fliC-HR-loxp-KnaR-loxp forward primer
   GCTTTCGCTGCCTTGATTGTGTACCACGTGTCGGTGAATCAATCGCCGGAGCCGATCATATTCAATAACCC
SEQ ID NO:26: fliC-HR-loxp-KnaR-loxp reverse primer
   ACGGTGAGAAACCGTGGGCAACAGCCCAATAACATCAAGTTGTAATTGATCTAGTGAACCTCTTCGAGGG
SEQ ID NO: 27: dapE (encoding succinyl-l,l-diaminopimelic acid desuccinylase, Gene ID: 1254005) 
ATGTCGTGCCCGGTTATTGAGCTGACACAGCAACTTATTCGTCGCCCCTCCCTGAGTCCGGATGACGCAGGCTGT
CAGGCATTAATGATTGAACGCCTGCGTAAAATCGGTTTTACCATTGAGCATATGGATTTTGGCGACACGCAAAAT
TTTTGGGCATGGCGTGGGCGTGGCGAAACGCTGGCGTTCGCCGGGCATACTGACGTCGTACCGGCTGGCGATGTC
GATCGCTGGATCAATCCGCCGTTTGAGCCTACGATTCGCGACGGAATGTTGTTCGGTCGCGGCGCGGCGGATATG
AAAGGTTCACTGGCGGCCATGGTGGTGGCTGCTGAACGTTTTGTTGCGCAGCATCCTCATCACCGGGGCCGCCTC
GCATTTTTGATTACCTCTGATGAGGAAGCCAGCGCAAAAAATGGCACCGTAAAGGTGGTTGAAGCGCTGATGGCC
CGTAACGAGCGGCTGGATTACTGTCTGGTCGGCGAACCTTCCAGTACTGAAATTGTGGGCGATGTCGTCAAAAAC
GGGCGTCGCGGTTCTCTCACCTGTAACCTCACTATCCATGGCGTTCAGGGGCACGTCGCCTATCCGCATCTGGCG
GATAACCCTGTTCATCGCGCCGCGCCTTTTCTTAATGAACTGGTCGCTATCGAGTGGGATCGGGGCAATGACTTT
TTCCCGGCCACCAGTATGCAGGTAGCCAATATCCAGGCAGGCACCGGCAGCAATAATGTCATTCCCGGCGAACTG
TTCGTGCAGTTTAACTTCCGTTTCAGCACGGAACTGACCGATGAGATGATCAAAGAGCGGGTACATGCGCTGCTT
GAAAAGCATCAGCTACGCTATACCGTAGACTGGTGGCTTTCCGGGCAGCCGTTCCTGACCGCACGCGGCAAGCTG
GTGGACGCGGTGGTAAACGCCATTGAGCACTATAATGAAATTAAACCGCAATTACTGACGACGGGCGGTACGTCC
GACGGACGTTTTATCGCCCGTATGGGAGCGCAGGTGGTAGAACTGGGGCCGGTGAACGCCACCATTCATAAAATT
AATGAATGTGTGAATGCCGCCGATCTCCAGCTACTGGCCCGTATGTATCAACGCATTATGGAACAGCTCGTCGCC
TGA
SEQ ID NO:28: htrA(encoding serine protease, Gene ID: 1251727) 
ATGAAAAAAACCACATTAGCAATGAGTGCACTGGCTCTGAGTTTAGGTTTGGCATTGTCGCCTCTGTCTGCCACG
GCGGCTGAAACGTCCTCTTCAGCAATGACTGCCCAGCAGATGCCAAGCCTGGCACCGATGCTCGAAAAAGTGATG
CCATCGGTGGTCAGTATTAATGTTGAAGGTAGCACCACGGTGAATACGCCGCGTATGCCGCGTAATTTCCAGCAG
TTCTTTGGCGATGACTCCCCGTTCTGCCAGGACGGTTCTCCGTTCCAGAATTCTCCGTTCTGCCAGGGCGGCGGT
AACGGCGGCAACGGCGGTCAACAACAGAAATTCATGGCGCTGGGCTCCGGCGTAATTATTGACGCCGCGAAGGGC
 
TACGTCGTCACCAACAACCACGTGGTTGATAACGCCAGCGTGATTAAAGTACAGCTTAGCGATGGGCGTAAATTC
GATGCTAAAGTGGTGGGCAAAGATCCGCGTTCTGATATCGCGCTGATTCAAATTCAGAATCCGAAGAACCTGACG
GCGATTAAGCTGGCGGACTCCGACGCGCTGCGCGTGGGGGATTATACCGTCGCTATTGGTAACCCGTTTGGTCTG
GGCGAAACGGTGACGTCAGGTATCGTTTCGGCGCTGGGGCGTAGCGGCCTGAACGTAGAAAATTACGAGAACTTT
ATTCAGACCGACGCCGCGATTAACCGTGGTAACTCCGGCGGCGCGCTGGTGAACCTGAACGGTGAGCTGATCGGT
ATTAACACCGCGATTCTGGCGCCGGACGGCGGCAACATCGGTATCGGCTTCGCTATCCCCAGTAACATGGTGAAA
AACCTGACGTCGCAGATGGTGGAATACGGCCAGGTGAAACGCGGCGAACTGGGGATCATGGGGACTGAGCTGAAT
TCCGAATTGGCGAAAGCGATGAAAGTCGACGCCCAGCGAGGCGCGTTCGTCAGCCAGGTGATGCCGAATTCGTCC
GCGGCGAAAGCGGGTATCAAAGCCGGGGATGTCATTACCTCGCTGAACGGTAAACCGATCAGCAGCTTTGCGGCG
CTGCGCGCTCAGGTCGGCACTATGCCGGTCGGCAGCAAAATCAGCCTCGGTCTGCTGCGTGAAGGTAAAGCGATT
ACGGTGAATCTGGAACTGCAGCAGAGCAGCCAGAGTCAGGTTGATTCCAGCACCATCTTCAGCGGGATTGAAGGC
GCTGAAATGAGCAATAAAGGCCAGGATAAAGGCGTTGTGGTGAGCAGCGTGAAAGCGAACTCACCCGCCGCGCAA
ATTGGCCTCAAAAAAGGCGATGTGATTATCGGCGCTAACCAGCAGCCGGTGAAAAATATCGCCGAGCTGCGTAAG
ATTCTCGACAGCAAGCCGTCGGTTCTGGCGCTGAATATTCAGCGTGGTGATAGTTCTATTTATTTGCTGATGCAG
TAA
SEQ ID NO:29: hlyA(encoding Listeria hemolysin, Gene ID:987033) 
ATGAAAAAAATAATGCTAGTTTTTATTACACTTATATTAGTTAGTCTACCAATTGCGCAACAAACTGAAGCAAAG
GATGCATCTGCATTCAATAAAGAAAATTCAATTTCATCCATGGCACCACCAGCATCTCCGCCTGCAAGTCCTAAG
ACGCCAATCGAAAAGAAACACGCGGATGAAATCGATAAGTATATACAAGGATTGGACTACAATAAAAACAATGTA
TTAGTATACCACGGAGATGCAGTGACAAATGTGCCGCCAAGAAAAGGTTACAAAGATGGAAATGAATATATTGTT
GTGGAGAAAAAGAAGAAATCCATCAATCAAAATAATGCAGACATTCAAGTTGTGAATGCAATTTCGAGCCTAACC
TATCCAGGTGCTCTCGTAAAAGCGAATTCGGAATTAGTAGAAAATCAACCAGATGTTCTCCCTGTAAAACGTGAT
TCATTAACACTCAGCATTGATTTGCCAGGTATGACTAATCAAGACAATAAAATCGTTGTAAAAAATGCCACTAAA
TCAAACGTTAACAACGCAGTAAATACATTAGTGGAAAGATGGAATGAAAAATATGCTCAAGCTTATCCAAATGTA
AGTGCAAAAATTGATTATGATGACGAAATGGCTTACAGTGAATCACAATTAATTGCGAAATTTGGTACAGCATTT
AAAGCTGTAAATAATAGCTTGAATGTAAACTTCGGCGCAATCAGTGAAGGGAAAATGCAAGAAGAAGTCATTAGT
TTTAAACAAATTTACTATAACGTGAATGTTAATGAACCTACAAGACCTTCCAGATTTTTCGGCAAAGCTGTTACT
AAAGAGCAGTTGCAAGCGCTTGGAGTGAATGCAGAAAATCCTCCTGCATATATCTCAAGTGTGGCGTATGGCCGT
CAAGTTTATTTGAAATTATCAACTAATTCCCATAGTACTAAAGTAAAAGCTGCTTTTGATGCTGCCGTAAGCGGA
AAATCTGTCTCAGGTGATGTAGAACTAACAAATATCATCAAAAATTCTTCCTTCAAAGCCGTAATTTACGGAGGT
TCCGCAAAAGATGAAGTTCAAATCATCGACGGCAACCTCGGAGACTTACGAGATATTTTGAAAAAAGGCGCTACT
TTTAATCGAGAAACACCAGGAGTTCCCATTGCTTATACAACAAACTTCCTAAAAGACAATGAATTAGCTGTTATT
AAAAACAACTCAGAATATATTGAAACAACTTCAAAAGCTTATACAGATGGAAAAATTAACATCGATCACTCTGGA
GGATACGTTGCTCAATTCAACATTTCTTGGGATGAAGTAAATTATGATCCTGAAGGTAACGAAATTGTTCAACAT
AAAAACTGGAGCGAAAACAATAAAAGTAAGCTAGCTCATTTCACATCGTCCATCTATTTGCCAGGTAACGCGAGA
AATATTAATGTTTACGCTAAAGAATGCACTGGTTTAGCTTGGGAATGGTGGAGAACGGTAATTGATGACCGGAAC
TTACCACTTGTGAAAAATAGAAATATCTCCATCTGGGGCACCACGCTTTATCCGAAATATAGTAATAAAGTAGAT
AATCCAATCGAATAA
SEQ ID NO:30: promoter pepTp 
GCAGGGGTAAAAGTGACCTGACGCAATATTTGTCTTTTCTTGCTTCTTAATAATGTTGTCACAAAAAGTGAGGGT
GACTACATGG
SEQ ID NO:31: promoter fnrSp 
CGCCGCAAAGTTTGAGCGAAGTCAATAAACTCTCTACCCATTCAGGGCAATATCTCTCTTGCAGGTGAATGCAAC
GTCAAGCGAT
SEQ ID NO:32: promoter ysgAp TCAGAAGAAGCAAAAAGACACTTTACCGAAGGGTTTAACATTTTTTCGTGATACTCATCACCATGACGCAAATGC GTTGCATAAA
SEQ ID NO:33: promoter ssbp1 
AACCGAGGTCACAACATAGTAAAAGCGCTATTGGTAATGGTACAATCGCGCGTTTACACTTATTCAGAACGATTT
TTTTCAGGAG
SEQ ID NO:34: promoter Hip1
   GGATAAAAGTGACCTGACGCAATATTTGTCTTTTCTTGCTTAATAATGTTGTCA
SEQ ID NO:35: promoter BBa_I14018
   T GTAAGT T TATACATAGGC GAGTACT CT GT TAT GG
SEQ ID NO:36: promoter BBa_R1074
   TTAAATTTCCTCTCGTCAGGCCGGAATAACTCCCTATAATGCGCCACCACACTGATAGTGCTAGTGTAGATCAC
SEQ ID NO:37: promoter Ptet-Fnr
   AAAATTGATCTGAATCAATATTTTGACAAAAATTGATCTGAATCAATATTTACTGAGC
SEQ ID NO:38: promoter Ptet-arcA
   GT TAATAAAAT GT TAT T GACAGT TAATAAAAT GTTATACTGAGC
SEQ ID NO:39: promoter PsseA
   TTAGCACGTTAATTATCTATCGTGTATATG

## Claims

1. A modified bacterium, wherein the bacterium comprise a hypoxia regulated essential gene expression cassette comprising an essential gene of the bacterium under the control of a strictly hypoxia inducible promoter, and the bacterium is deficient in at least one gene involved in or regulating the endogenous anti-oxidative stress response pathway or a functional expression product thereof, as compared to an unmodified starting strain.

2. The modified bacterium of claim 1, wherein the strictly hypoxia inducible promoter is selected from the group consisting of pepTp, fnrSp, ysgAp, ssbp1, Hip1, BBa_114018, BBa_R1074, Ptet-arcA, and Ptet-Fnr.

3. The modified bacterium of claim 1, wherein the strictly hypoxia inducible promoter is ssbp1 promoter.

4. The modified bacterium of any one of the preceding claims, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

5. The modified bacterium of claim 4, wherein the essential gene is selected from *dapA, dapB, dapD, dapE, argD, dapF,* and any combination thereof.

6. The modified bacterium of claim 4, wherein the essential gene is selected from *dapA* and *dapE.*

7. The modified bacterium of any one of the preceding claims, wherein the gene involved in or regulating the endogenous anti-oxidative stress response pathway is an HtrA serine protease family gene.

8. The modified bacterium of any one of the preceding claims, wherein the functional expression product of the gene involved in or regulating the endogenous anti-oxidative stress response pathway is an HtrA serine protease family related protein.

9. The modified bacterium of any one of the preceding claims, wherein the bacterium is deficient in the activity of HtrA serine protease.

10. The modified bacterium of any one of the preceding claims, wherein the gene involved in or regulating the endogenous anti-oxidative stress response pathway is *htrA.*

11. The modified bacterium of any one of the preceding claims, wherein the bacterium is deficient in *htrA.*

12. The modified bacterium of any one of claims 1-11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

13. The modified bacterium of any one of claims 1-11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

14. The modified bacterium of claim 13, wherein the exogenous essential gene expression cassette is integrated into the bacterial chromosome.

15. The modified bacterium of claim 13, wherein the exogenous essential gene expression cassette is outside the bacterial chromosome.

16. The modified bacterium of claim 15, wherein the exogenous essential gene expression cassette is present in the plasmid carried by the bacterium.

17. The modified bacterium of any one of the preceding claims, which further comprises a pH regulated expression cassette, wherein the pH regulated expression cassette comprises a gene encoding a bacteria-derived hemolysin protein under the control of a promoter that is active under acid pH condition.

18. The modified bacterium of claim 17, wherein the bacteria-derived hemolysin protein is a gram-negative bacterial hemolysin protein.

19. The modified bacterium of claim 18, wherein the gene encoding the gram-negative bacterial hemolysin protein is *hlyA* or *hlyE.*

20. The modified bacterium of claim 19, wherein the *hlyA* or *hlyE* is derived from *Listeria monocytogenes, Vibrio cholerae,* or *Escherichia coli.*

21. The modified bacterium of any one of claims 17-20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

22. The modified bacterium of any one of claims 17-21, wherein the promoter that is active under acid pH condition is selected from the group consisting of sseA, ssrA, ssaB, ssaG, ssaM, and ssaR.

23. The modified bacterium of any one of claims 17-21, wherein the promoter that is active under acid pH condition is sseA.

24. The modified bacterium of any one of the preceding claims, wherein the unmodified starting strain is a facultative anaerobic bacterium.

25. The modified bacterium of any one of the preceding claims, wherein the bacterium is a bacteirum of *Enterobacteriaceae.*

26. The modified bacterium of any one of the preceding claims, wherein the bacterium is a bacteirum of *Escherichia sp., Salmonella* sp., *Shigella* sp., *Klebsiella* sp., *Yersinia sp., Citrobacter sp., Enterobacter* sp., *Serratia sp., Proteus sp., Morganella sp., Providencia sp., Hajnia sp.,* and *Pantoea sp.*

27. The modified bacterium of any one of the preceding claims, wherein the bacterium is selected from the group consisting of *Escherichia coli, Enterobacter blattae* (*E.blattae*), *Enterobacter fergusonii* (*E.fergusonii*), *Enterobacter hermannii* (*E.hermannii*), *Enterobacter vulneris* (*E.vulneris*), *Salmonella enterica* (*S.enterica*), *Salmonella bongori* (*S.bongori*), *Salmonella typhi* (*S.typhi*), *Salmonella choleraesuis* (*S.choleraesuis*), *Salmonella typhimurium* (*S.typhimurium*), *Shigella dysenteriae* (*S.dysenteriae*), *Shigella flexneri* (*S.flexneri*), *Shigella boydii* (*S.boydii*), *Shigella sonnei* (*S.sonnei*)*, Klebsiella pneumoniae* (*K.peumoniae*), *Klebsiella oxytoca* (*K.oxytoca*), *Yersinia pestis* (*Y.pestos*), *Yersinia enterocolitica* (*Y.enterocolitica*), *Yersinia pseudotuberculosis* (*Y.pseudotuberculosis*), *Yersinia aldouae* (*Y.aldouae*), *Yersinia bercovieri* (*Y.bercovieri*)*, Yersinia frederiksenii* (*Y.frederiksenii*), *Yersinia intermedia* (*Y.intermedia*), *Yersinia kristersenii* (*Y.kristersenii*), *Yersinia mollaretti* (*Y.mollaretti*), *Yersinia rohdei* (*Y.rohdei*), *Yersinia ruckeri* (*Y.ruckeri*)*, Citrobacter freundii* (*C.freundii*), *Citrobacter koseri* (*C.kaseri*), *Citrobacter braakii* (*C.braakii*), *Enterobacter aerogenes* (*E.aerogenes*), *Enterobacter cloacae* (*E.cloacae*), *Enterobacter gergoviae* (*E.gergoviac*)*, Enterobacter sakazakii* (*E.sakazakii*)*, Enterobacter taylorae* (*E.tavlorae*)*, Enterobacter amnigenus* (*E.aminigenus*), *Enterobacter intermedius* (*E.intermedius*), *Enterobacter asburiae* (*E.asburiac*), *Enterobacter cancerogenus* (*E.cancerogenus*), *Enterobacter dissolvens* (*E.dissolvens*), *Enterobacter nimipressuralis* (*E.nimipressuralis*), *Serratia marcescens* (*S.marcescens*), *Serratia entomophila* (*S.entomophila*), *Serratia ficaria* (*S.ficaria*), *Serratia fonticola* (*S.fonticola*), *Serratia grimesii* (*S.grimesii*), *Serratia liquefaciens* (*S.liquefaciens*), *Serratia odorifera* (*S.odorifera*), *Serratia plymuthica* (*S.plymuthica*), *Serratia proteamaculans* (*S.proteamaculans*), *Serratia rubidaea* (*S.rubidaea*), *Serratia ureilytica* (*S.ureilytica*), *Proteus mirabilis* (*P.mirabilis*), *Proteus vulgaris* (*P.vulgaris*), *Proteus myxofaciens* (*P.myxofαciens*), *Proteus penneri* (*P.penneri*), *Proteus hauseri* (*P.hauseri*))*, Morganella morganii* (*M.morganii*), *Providencia alcalifaciens* (*P.alcalifaciens*), *Providencia rustigianii* (*P.rustigianii*), *Providencia stuartii* (*P.stuartii*), *Providencia rettgeri* (*P.rettgeri*), *Providencia heimbachae* (*P.heimbochae*), *Hafnia alvei* (*H.alvei*), and *Pantoea agglomerans* (*P.agglomerans*).

28. The modified bacterium of any one of the preceding claims, wherein the bacterium is *Salmonella typhimurium.*

29. The modified bacterium of claim 28, wherein the starting strain is *Salmonella typhimurium* SL7207.

30. The modified bacterium of any one of the preceding claims, wherein the bacterium expresses a wild-type lipopolysaccharide (LPS).

31. The modified bacterium of any one of the preceding claims, wherein, when administrated to a subject with a tumor, the bacterium is capable of surviving and proliferating in tumor tissue and is rapidly cleared in normal tissue.
